(19)

Europäisches Patentamt
European Patent Office
Office européen des brevets

(11) **EP 4 288 559 B1**

(12) **EUROPEAN PATENT SPECIFICATION**

(45) Date of publication and mention
of the grant of the patent:
**26.11.2025   Bulletin 2025/48**

(21) Application number: **22749386.3**

(22) Date of filing: **03.02.2022**

(51) International Patent Classification (IPC):
**G16B 5/00** (2019.01)      **C12Q 1/68** (2018.01)
**A61K 35/37** (2015.01)      **A61K 35/66** (2015.01)
**A61K 35/74** (2015.01)      **G16H 20/60** (2018.01)
**A61K 35/741** (2015.01)      **A61K 35/00** (2006.01)
**A23L 33/00** (2016.01)

(52) Cooperative Patent Classification (CPC):
**G16H 20/60; A61K 35/741; G16B 5/00;**
A23L 33/00; A61K 2035/115; Y02A 90/10

(86) International application number:
**PCT/IN2022/050094**

(87) International publication number:
**WO 2022/168120 (11.08.2022 Gazette 2022/32)**

(54) **METHOD AND SYSTEM FOR DESIGNING PERSONALIZED THERAPEUTICS AND DIET BASED ON FUNCTIONS OF MICROBIOME**

VERFAHREN UND SYSTEM ZUM ENTWURF PERSONALISIERTER THERAPEUTIKA UND DIÄT AUF BASIS VON MIKROBIOMFUNKTIONEN

PROCÉDÉ ET SYSTÈME POUR CONCEVOIR DES AGENTS THÉRAPEUTIQUES PERSONNALISÉS ET UN RÉGIME SUR LA BASE DE FONCTIONS DU MICROBIOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **03.02.2021   IN 202121004712**

(43) Date of publication of application:
**13.12.2023   Bulletin 2023/50**

(73) Proprietor: **Tata Consultancy Services Limited Maharashtra (IN)**

(72) Inventors:
- **ANAND, Swadha**
  **Maharashtra, Pune 411013 (IN)**
- **MANDE, Sharmila Shekhar**
  **Maharashtra, Pune 411013 (IN)**
- **BOSE, Chandrani**
  **Maharashtra, Pune 411013 (IN)**
- **SINGH, Rashmi**
  **Maharashtra, Pune 411013 (IN)**
- **KAUR, Harrisham**
  **Maharashtra, Pune 411013 (IN)**
- **BHUSAN, Kuntal Kumar**
  **Maharashtra, Pune 411013 (IN)**
- **DAS BAKSI, Krishanu**
  **New Delhi 110001 (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**EP-A1- 3 421 616**      **WO-A1-2019/183317**
**US-A1- 2014 179 726**      **US-A1- 2017 329 897**
**US-A1- 2018 318 323**      **US-A1- 2019 192 581**
**US-B2- 10 755 800**

- XOCHITL C MORGAN ET AL: "Dysfunction of the intestinal microbiome in inflammatory bowel disease and treatment", GENOME BIOLOGY, BIOMED CENTRAL LTD, vol. 13, no. 9, 26 September 2012 (2012-09-26), pages R79, XP021123384, ISSN: 1465-6906, DOI: 10.1186/GB-2012-13-9-R79

**(Cont. next page)**

- **GUPTA AKSHITA ET AL: "Therapies to modulate gut microbiota: Past, present and future", vol. 26, no. 8, 15 February 2020 (2020-02-15), CN, pages 777 - 788, XP093224900, ISSN: 1007-9327, Retrieved from the Internet <URL:https://www.wjgnet.com/1007-9327/full/v26/i8/777.htm> DOI: 10.3748/wjg.v26.i8.777**
- **FRANZOSA ERIC A., SIROTA-MADI ALEXANDRA, AVILA-PACHECO JULIAN, FORNELOS NADINE, HAISER HENRY J., REINKER STEFAN, VATANEN TOMMI, HA: "Gut microbiome structure and metabolic activity in inflammatory bowel disease", HHS PUBLIC ACCESS, February 2019 (2019-02-01), pages 293 - 305, XP036679932, Retrieved from the Internet <URL:https://www.ncbi.nlm.nih.gov/PMC/articies/PMC6342642/pdf/nihms-1510763.pdf> [retrieved on 20220415]**

**Description**

CROSS-REFERENCE TO RELATED APPLICATIONS AND PRIORITY

This present application claims priority from International Application No. PCT/IN2022/050094 filed on February 03, 2022, which application claims priority from Indian application no. 202121004712 filed on February 3, 2021.

TECHNICAL FIELD

The disclosure herein generally relates to the field of microbiome-based therapeutics, and, more particularly, to a method and a system for designing personalized therapeutics and diet based on functions of microbiome.

BACKGROUND

Human and other vertebrates are inhabited by a variety of microorganisms termed as the 'microbiota' and the collection of the genetic material of the microbiota is called 'microbiome'. However, the two terms 'microbiota' and 'microbiome' are widely used interchangeably. The gastrointestinal tract or GI tract or gut is the most colonized body site for harboring these microbial communities. The microbes are known to perform several metabolic activities which can lead to interactions between an individual's microbiome and its own genetics termed as human microbiome interactions. The microbiota is known to influence the individual/host's health in multiple ways including modulating immune system, energy harvest, influencing various signaling mechanisms etc. Hence altering the microbial community within the gut of an individual can alleviate the risk or manifestations of certain diseases/ disorders.

The composition of the gut microbiome varies with multiple factors including geography, lifestyle, dietary patterns etc., among different individuals. Further the microbial composition of microbiome might differ even between individuals residing in similar surroundings and even between twins who have an identical genetic makeup. Therefore, different taxonomic groups might be involved in dysbiosis in different populations or different individuals, wherein alterations in these microbial communities or dysbiosis have been associated with a myriad of diseases/ disorders. Thus, the dysbiotic state can be characterized by a loss of a set of microbes contributing to a beneficial function like biosynthesis of a useful metabolite or may also involve an increase in abundance of microbes which might produce a metabolite deleterious to different aspects of human health. Dysbiosis might also involve an increase in abundance of microbes which might produce a metabolite deleterious to different aspects of human health. To add to the complexity, variations in the form of reduction or augmentation of more than one function may be associated to a specific dysbiotic state. In other words, owing to a unique makeup of microbiome as well as genetic background in different individuals, the combination of strains for effective probiotics and prebiotics designed for each individual may be different. This emphasizes the importance of personalized microbiome therapeutics.

In recent times, the costs associated with sequencing and analyses of microbiome have become nominal. It has become possible to obtain an individual's gut microbiome composition using identification of microbe specific DNA or RNA marker sequences. The samples for gut microbiome profiling can be obtained using non-invasive methods like collection of faecal matter. The obtained taxonomic information is often compared to the composition obtained for/known for a population of individuals suffering from a particular disease/disorder or expected to have a disease risk. Indicators for assessment of gut health often include analytics of abundances of microbes or the functions performed by them.

It is known in the art that altering the microbial community within the gut of an individual can alleviate the risk or manifestations of certain diseases/disorders. Such alterations may involve (a) using antibiotics to target pathogenic strains and reduce their abundances (b) administration of live therapeutics like probiotics or dietary components like prebiotics to augment the abundance of organisms beneficial for the gut (c) fecal microbial transplants where organisms inhabiting the gut of a healthy individual are obtained from the fecal matter and transplanted into the gut of a diseased individual to replace the microbial communities.

The use of probiotics in changing the relative abundance of desired microbes in gut microbiome as well as in altering metabolic makeup and modulation of immune system in some cases has been established.

For Example, document EP18180341 discloses a system and method for predicting gut health of an individual using non-invasive techniques. The system is making use of two types of pathways, i.e. one which are beneficial to gut health and the second which are harmful to gut health. These two types of pathways are annotated in the genomes of gut bacteria. Best combinations of subsets of these pathways capable of distinguishing between gut commensals and pathogens are assigned as pathway biomarkers. The identified pathway biomarkers are then used to develop scheme for prediction of gut health status.

Despite that there are studies which show that the efficacy of probiotics is limited or absent in case of certain individuals. One explanation could be that most probiotics involve administration of strains assuming that same organisms might be in low abundance in all individuals or that same functions might be compromised in most individuals. In summary, the gut microbiome dysbiosis in different individuals might be attributed to reduced presence of different beneficial functions or an increased prevalence of different harmful functions or a combination of both these scenarios. Thus, one probiotic or dietary regimen may not have same efficacy in different individuals. The utilization of an individuals' microbiome profile before probiotic/dietary supplement administration as well as analyzing functions which are either compromised or augmented in a dysbiotic state for a particular individual may help in improving the efficacy of the probiotic. Therefore, probiotics which are customized and personalized for an individual on the basis of microbiome functions or taxonomic changes associated with the dysbiosis is the need of the art.

SUMMARY

Embodiments of the present disclosure present technological improvements as solutions to one or more of the above-mentioned technical problems recognized by the inventors in conventional systems. For example, in one embodiment, a method for designing personalized therapeutics and diet based on functions of microbiome is provided. The system includes a memory storing instructions, one or more communication interfaces, and one or more hardware processors coupled to the memory via the one or more communication interfaces, wherein the one or more hardware processors are configured by the instructions to receive a set of input data, wherein the set of input data comprises data associated with a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts, a plurality of diet optimization pre-requisite parameters. The system is further configured to generate a set of knowledge bases using the set of input data, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap. The system is further configured to receive a plurality of gut samples of an individual at two-time stamps, wherein the two-time stamps comprise a time stamp 1 and a time stamp 2 at which the two samples have been collected. The system is further configured to generate a taxa abundance matrix for the plurality of gut samples, via the one or more hardware processors, wherein the taxa abundance matrix comprises of a taxa set 1 and a taxa set 2, where the taxa set 1 and the taxa set 2 is generated using the time stamp 1 and the time stamp 2. The system is further configured to generate a pathway abundance for each of the plurality of gut-samples obtained at the time stamp 1 and the time stamp 2 using the taxa abundance matrix and the FGmap, wherein the pathway abundance is generated using a sample processing technique, wherein the pathway abundance for each time stamp is indicative of presence of the plurality of metabolic pathways of the GMP in the gut microbiome of the individual at the corresponding time stamp. The system is further configured to identify a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein (a) Case A : a decrease in the pathway abundance of the set of beneficial pathways, wherein the decrease is an indicative of a decrease in the set of beneficial microbes, or (b) Case B : an increase in the pathway abundance of the set of harmful pathways wherein the increase is indicative of an increase in the set of harmful microbes , or (c) Case C : a decrease in the pathway abundance of the set of beneficial pathways and increase in the pathway abundance of the set of harmful pathways indicative of an increase in set of harmful microbes and decrease in the set of beneficial microbes. The system is further configured to determine an initial set of personalized therapeutics and diet recommendation, wherein the initial set of personalized therapeutics and diet comprises atleast one of : a personalized therapeutic and a personalized dietary regimen using the ProbMap, wherein the initial set of personalized therapeutics and diet recommendation is a row corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap. The system is further configured to identify a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP. The system is further configured to identify a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW. The system is further configured to recommend a personalized therapeutic intervention for improving gut health of the individual, via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of: (a) For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes, (b) For Case B ( an increase in the pathway abundance of the set of harmful microbes) recommending an optimized diet, wherein the optimized diet promotes growth of the plurality of beneficial microbes and inhibits growth of harmful microbes, and (c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

In another aspect, a method for designing personalized therapeutics and diet based on functions of microbiome is provided. The method includes receiving a set of input data, wherein the set of input data comprises data associated with a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts, a plurality of diet optimization pre-requisite parameters. The method further includes generating a set of knowledge bases using the set of input data, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap. The method further includes receiving a plurality of gut samples of an individual at two-time stamps, wherein the two-time stamps comprise a time stamp 1 and a time stamp 2 at which the two samples have been collected. The method further includes generating a taxa abundance matrix for the plurality of gut samples, via the one or more hardware processors, wherein the taxa abundance matrix comprises of a taxa set 1 and a taxa set 2, where the taxa set 1 and the taxa set 2 is generated using the time stamp 1 and the time stamp 2. The method further includes generating a pathway abundance for each of the plurality of gut-samples obtained at the time stamp 1 and the time stamp 2 using the taxa abundance matrix and the FGmap, wherein the pathway abundance is generated using a sample processing technique, wherein the pathway abundance for each time stamp is indicative of presence of the plurality of metabolic pathways of the GMP in the gut microbiome of the individual at the corresponding time stamp. The method further includes identifying a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein (a) Case A : a decrease in the pathway abundance of the set of beneficial pathways , wherein the decrease is an indicative of a decrease in the set of beneficial microbes, or (b) Case B : an increase in the pathway abundance of the set of harmful pathways wherein the increase is indicative of an increase in the set of harmful microbes , or (c) Case C : a decrease in the pathway abundance of the set of beneficial pathways and increase in the pathway abundance of the set of harmful pathways indicative of an increase in set of harmful microbes and decrease in the set of beneficial microbes. The method further includes determining an initial set of personalized therapeutics and diet recommendation, wherein the initial set of personalized therapeutics and diet comprises atleast one of : a personalized therapeutic and a personalized dietary regimen using the ProbMap, wherein the initial set of personalized therapeutics and diet recommendation is a row corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap. The method further includes identifying a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP. The method further includes identifying a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW. The method further includes recommending a personalized therapeutic intervention for improving gut health of the individual, via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of: (a) For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes, (b) For Case B ( an increase in the pathway abundance of the set of harmful microbes) recommending an optimized diet, wherein the optimized diet promotes growth of the plurality of beneficial microbes and inhibits growth of harmful microbes, and (c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

In yet another aspect, a non-transitory computer readable medium for designing personalized therapeutics and diet based on functions of microbiome is provided. The program includes receiving a set of input data, wherein the set of input data comprises data associated with a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts, a plurality of diet optimization pre-requisite parameters. The program further includes generating a set of knowledge bases using the set of input data, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap. The method further includes receiving a plurality of gut samples of an individual at two-time stamps, wherein the two-time stamps comprise a time stamp 1 and a time stamp 2 at which the two samples have been collected. The program further includes generating a taxa abundance matrix for the plurality of gut samples, via the one or more hardware processors, wherein the taxa abundance matrix comprises of taxa set 1 and a taxa set 2, where the taxa set 1 and the taxa set 2 is generated using the time stamp 1 and the time stamp 2. The program further includes generating a pathway abundance for each of the plurality of gut-samples obtained at the time stamp 1 and the time stamp 2 using the taxa abundance matrix and the FGmap, wherein the pathway abundance is generated using a sample processing technique, wherein the pathway abundance for each time stamp is indicative of presence of the plurality of metabolic pathways of the GMP in the gut microbiome of the individual at the corresponding time stamp. The program further includes identifying a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein (a) Case A : a decrease in the pathway abundance of the set of beneficial pathways , wherein the decrease is an indicative of a decrease in the set of beneficial microbes, or (b) Case B : an increase in the pathway abundance of the set of harmful pathways wherein the increase is indicative of an increase in the set of harmful microbes , or (c) Case C : a decrease in the pathway abundance of the set of beneficial pathways and increase in the pathway abundance of the set of harmful pathways indicative of an increase in set of harmful microbes and decrease in the set of beneficial microbes. The program further includes determining an initial set of personalized therapeutics and diet recommendation, wherein the initial set of personalized therapeutics and diet comprises atleast one of : a personalized therapeutic and a personalized dietary regimen using the ProbMap, wherein the initial set of personalized therapeutics and diet recommendation is a row corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap. The program further includes identifying a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP. The program further includes identifying a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW. The program further includes recommending a personalized therapeutic intervention for improving gut health of the individual, via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of: (a) For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes, (b) For Case B ( an increase in the pathway abundance of the set of harmful microbes) recommending an optimized diet, wherein the optimized diet promotes growth of the plurality of beneficial microbes and inhibits growth of harmful microbes, and (c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

It is to be understood that both the foregoing general description and the following detailed description are exemplary and explanatory only and are not restrictive of the invention, as claimed.

The invention is set out in the appended set of claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG.1 illustrates an exemplary system for designing personalized therapeutics and diet based on functions of microbiome according to some embodiments of the present disclosure.
FIG.2A, FIG.2B, FIG.2C, FIG.2D, FIG.2E, FIG.2F, FIG.2G and FIG.2H is a flow diagram illustrating a method (200) for designing personalized therapeutics and diet based on functions of microbiome according to some embodiments of the present disclosure.
FIG.3A and FIG.3B is a flow diagram illustrating a method (300) for generation of genome metabolite pathway (GMP) map in accordance with some embodiments of the present disclosure.
FIG.4 is a flow diagram illustrating a method (400) for generating the pathway abundance matrices using sample processing technique in accordance with some embodiments of the present disclosure.
FIG.5 is a flow diagram illustrating a method (500) for recommending an optimized diet based on an optimization technique in accordance with some embodiments of the present disclosure.
FIG.6 is a diagram illustrating a set of neighbors for designing personalized therapeutics and diet based on functions of microbiome according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

GLOSSARY

The term "microbiota" in the context of the present disclosure refers to the collection of microorganisms, such as, bacteria, archaea, protists, fungi, and virus, that inhabit a particular ecological niche (e.g. a human body site such as skin, gut, oral cavity, etc.,) or an environmental/geographical site. The term 'gut microbiome' in the context of the present disclosure refers to a plurality of the genetic material of the microbiota (or microbial communities) that reside within gut/ gastrointestinal tract/ intestines of a human or any vertebrate host. Any imbalance or disturbance in the microbiome is termed as dysbiosis. The two terms 'microbiota' and 'microbiome' are widely used interchangeably.
The term 'metabolite' in the context of the present disclosure refers to a chemical entity, a product or a compound which is formed due to metabolic processes within a microbe. The metabolite(s) known to have beneficial effects in humans in terms of nutrition, immune modulation, maintaining organ function, regulation of gene function etc. are termed as beneficial metabolites. The metabolite(s) known to have harmful effects in host like increase in inflammatory responses, damaging gut integrity, aberrations in gene regulation etc. have been termed as deleterious metabolites.
The term 'functions' in the context of the present disclosure refers to presence of genes on a microbial genome which encode for proteins or enzymes that form a pathway for the conversion of a candidate metabolite to a specific candidate product.
The term 'homolog' in the context of the present disclosure refers to two proteins arising from a common evolutionary origin or ancestry. The usage of the term can also be extended to proteins having a protein sequence similarity of greater than 60% arising due to convergent evolution to perform similar functions.
The term 'pathway' in the context of the present disclosure refers to a series of reactions where in one reaction a starting substrate is converted to a product using the genes encoding proteins/ enzymes for this conversion. The product so biosynthesized is taken up by gene(s) encoding proteins/ enzymes for the next reaction in the series. The 'cognate homolog' of a pathway refers to the protein/ enzyme candidate actually functional in a pathway out of all the homologs of this protein/ enzyme encoded on a genome.
The term 'operon' or 'gene cluster' in the context of the present disclosure refers to occurrence of genes involved in a pathway on neighboring locations on a microbial genome.
The term 'protein domain' or 'domain' in the context of the present disclosure refers to the conserved region of a protein which is capable of existing independently by itself performing a similar function. The protein domains constituting a protein can be identified using various methods like sequence homology, hidden markov model etc. in order to understand or annotate functions of the protein.
The term 'hash' in the context of the present disclosure refers to a key-value pair and their associations which can also be termed as associative arrays or a dictionary.

The term 'taxonomic abundance' in the context of the present disclosure refers to the occurrence/ abundance of taxonomic groups within a microbiome sample which can be collected from a body-site. The taxonomic abundance can be obtained as genus, specie or strain taxonomic level in the form of the matrix comprising the abundances with the name of taxa as rows and samples at columns. Certain genes like 16S rRNA in bacteria are used as marker genes for understanding phylogeny of taxonomic groups present in a microbiome sample. In this disclosure, the taxonomic groups comprise of microbes classified according to taxonomic hierarchy, wherein taxonomic hierarchy may comprise one or more. of plurality of phyla, classes, orders, families, genera, species or strains. Reference to any one of these taxonomic groups can be considered as applicable to all the taxonomic groups

The term 'time points' in the context of the present disclosure refers to the samples obtained from same individual at different time data points while the term 'population (s)' would refer to data corresponding to set of individuals which might belong to same/ similar/ comparable/ matched specific analyzed feature(s) like but not limited to health condition, age, geography, pursuing a treatment etc.

The term 'probiotic' in the context of the present disclosure refers to live microorganisms which can provide health benefits when administered to a human or any vertebrate host. The term 'prebiotic' or 'nutrient supplements' refers to food components that can trigger growth of bacteria or their activity towards maintaining host health. The term 'synbiotic' refers to combination or mixture of probiotics (live health-promoting micro-organisms) and prebiotics (nutrient supplements augmenting the growth of beneficial micro-organisms) administered to exert a synergistic beneficial effect. The terms 'metabiotic' or/and 'postbiotic' or/and 'biogenics' refer to the components secreted by live microorganisms either alone or in adjunct with their metabolites/products/by-products that aid in augmentation/depletion of certain host's physiological functions that in-turn regulates/restores the health of the host. The terms 'paraprobiotics' or/and 'ghost probiotics' are modified, immobilized, inactivated and non-viable forms of probiotics that when ingested restore the beneficial functions of gut microbiome.

The term 'Faecal Microbial Transplant' or 'FMT' in the context of the present disclosure refers to administration of components of faecal matter from a donor into the intestinal tract of a recipient individual whose gut microbiome indicates dysbiosis. **In** one embodiment, FMT can also refer to 'a-FMT (autologous FMT)' where an individual's own stool is banked during 'healthy' condition and the components of the same is used when required under dysbiotic condition. FMT helps to change the recipients gut microbiome thereby alleviating the dysbiosis caused in recipient by diseased condition or a therapy administered to a recipient.

The term 'bioengineering' or 'genetic engineering' refers to process of altering/modifying the genetic makeup of one or more microorganisms which in-turn provide/augment the usage and applicability of the said microorganisms for host/ industries/ environment.

The term "perturbed function" or "perturbed pathway" or "probable pathway" refers to an increase in harmful pathways or biosynthesis of harmful metabolites or degradation of beneficial metabolites. It may also refer to decrease in beneficial pathways or beneficial metabolites or decrease in degradation of harmful metabolites. The said metabolites which significantly increase or decrease between two or more gut samples are termed as probable or perturbed metabolites.

The proposed invention enables the identification of microbial functions that need to be augmented or depleted in order to restore microbiome balance. Further, the invention involves enabling a healthcare provider for personalizing the probiotics for an individual based on these identified functions. Further, suitable nutrient supplementation as well as dietary patterns can be predicted for an individual which might prove efficient for a particular individual. Such medicament might prove to be effective for designing personalized and preventive therapeutics for an individual or a group of individuals possessing similar microbiome profile. In an embodiment, these personalized probiotics can be administered alone or in combination with other ongoing treatment regimens for an individual.

As discussed in the background section, the gut microbiome dysbiosis in different individuals might be attributed to reduced presence of different beneficial functions or an increased prevalence of different harmful functions or a combination of both these scenarios. Thus, one probiotic or dietary regimen may not have same efficacy in different individuals. The utilization of an individuals' microbiome profile before probiotic/dietary supplement administration as well as analyzing functions which are either compromised or augmented in a dysbiotic state for a particular individual may help in improving the efficacy of the probiotic. Therefore, there is a requirement for probiotics which are customized and personalized for an individual based on microbiome functions or taxonomic changes associated with the dysbiosis.

The disclosed invention differs from the known in the art methods of designing composition(s) for therapeutic strategies which include dietary regimens, probiotics, prebiotics, metabiotics, synbiotics known in the art as described herein. In an embodiment, the composition may comprise of a probiotic and/or prebiotic and/or dietary suggestion and/or nutritional supplement etc. Any other composition capable of changing the functions of microbiome as described herewith are within scope of the invention.

    1. Identifying the combination of perturbed pathways which may include beneficial metabolic pathways and their constituent metabolites that are reduced and harmful metabolic pathways and their constituent metabolites that are increased in gut microbiome of dysbiotic state of an individual-.

2. Utilization of microbiome profiles and the metabolic pathway information derived from this profile of individuals to identify the perturbed pathways. The information of perturbed pathways and hence the perturbed functions of microbiome can be applied to design the microbiome function based therapeutic strategy. The metabolic pathways identified to be perturbed work as indication of perturbed functions.

3. Utilizing a knowledgebase to derive composition of personalized therapeutic regiments comprising one or more of microbial cocktails, dietary components, nutrient supplements or prebiotics required to correct the effect of said perturbed metabolic pathways and thereby the metabolites associated with dysbiosis.

Utility of the invention

1. The disclosure provides a method of identifying perturbed microbial functions in dysbiotic state of an individual and utilizing the said functions to decide on the personalized therapeutic regimens for improving the health of the individual from dysbiotic to a balance microbiome.

2. The composition is designed such that the compromised beneficial metabolite or taxa responsible for generating that metabolite are replenished in the gut. Similarly, the composition can be designed-to curb harmful metabolite presence or the presence of taxa responsible for biosynthesizing the harmful metabolite.

The personalized therapeutic intervention is designed such that the compromised beneficial metabolite or taxa responsible for generating that metabolite are replenished in the gut. Similarly, the composition can be designed to curb harmful metabolite presence or the presence of taxa responsible for biosynthesizing the harmful metabolite.

Considering the importance & the effects of the microbiomes, use of probiotics in changing the relative abundance of desired microbes in gut microbiome as well as in altering metabolic makeup and modulation of immune system in some cases has been established. However, owing to a unique makeup of microbiome as well as genetic background in different individuals, the combination of strains for effective probiotics and prebiotics designed for everyone should ideally be different/personalized/customized according to his/her gut microbiome to give a maximum benefit to the individual

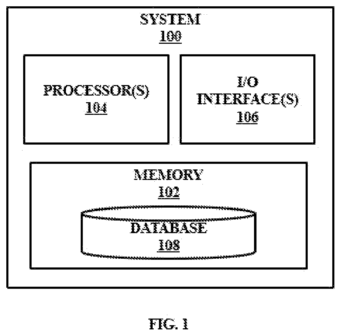

Referring now to the drawings, and more particularly to FIG. 1 through FIG.6, where similar reference characters denote corresponding features consistently throughout the figures, there are shown preferred embodiments and these embodiments are described in the context of the following exemplary system and/or method.

FIG.1 is a functional block diagram of a system 100 for designing personalized therapeutics and diet based on functions of microbiome in accordance with some embodiments of the present disclosure.

In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of the system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The I/O interface(s) 106 can include a variety of software and hardware interfaces, for example, a web interface, a graphical user interface, a touch user interface (TUI) and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices (nodes) of the system 100 to one another or to another server.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 108 configured to include information regarding metabolites including plurality of beneficial microbes and a plurality of harmful microbes along with the pathway-functions of the metabolites. The memory 102 may comprise information pertaining to input(s)/output(s) of each step performed by the processor(s) 104 of the system 100 and methods of the present disclosure. In an embodiment, the database 108 may be external (not shown) to the system 100 and coupled to the system via the I/O interface 106.

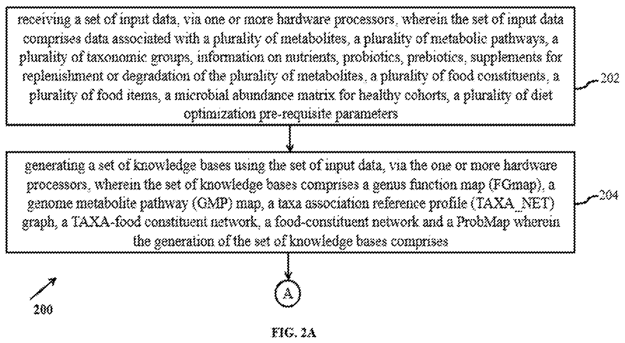

Functions of the components of system 100 are explained in conjunction with flow diagram of FIGS.2A-2H, FIGS.3A-3B, FIG.4 and FIG.5 designing personalized therapeutics and diet based on functions of microbiome.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

The various modules of the system 100 are configured for designing personalized therapeutics and diet based on functions of microbiome are implemented as at least one of a logically self-contained part of a software program, a self-contained hardware component, and/or, a self-contained hardware component with a logically self-contained part of a software program embedded into each of the hardware component that when executed perform the above method described herein.

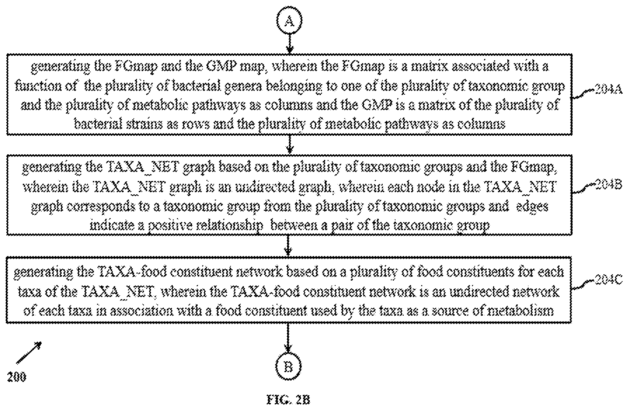

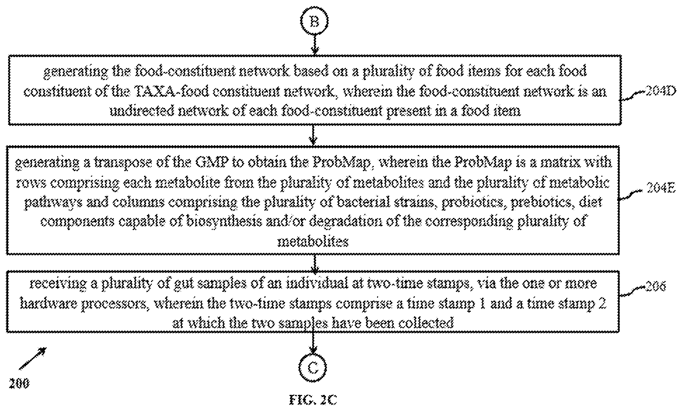

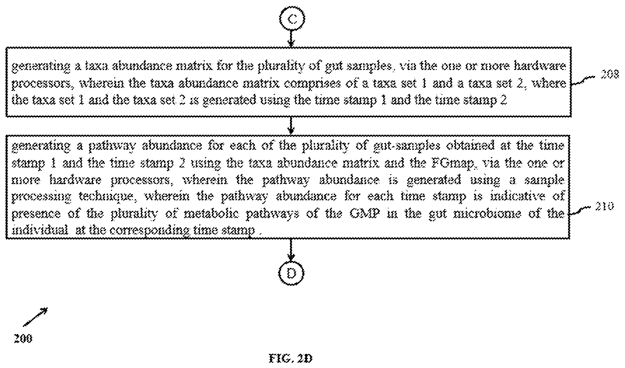

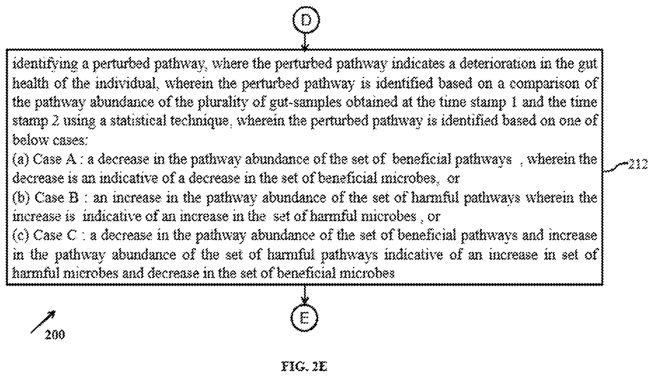

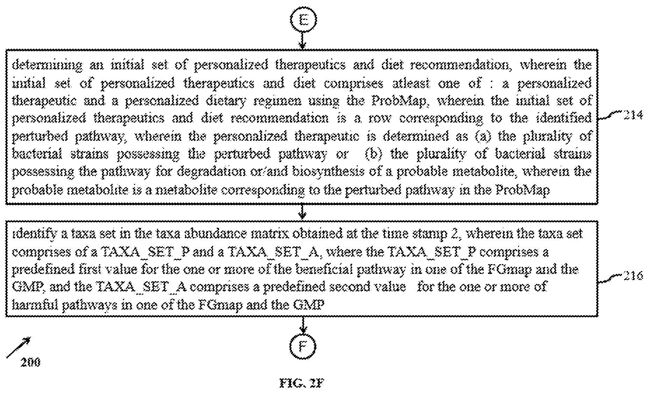

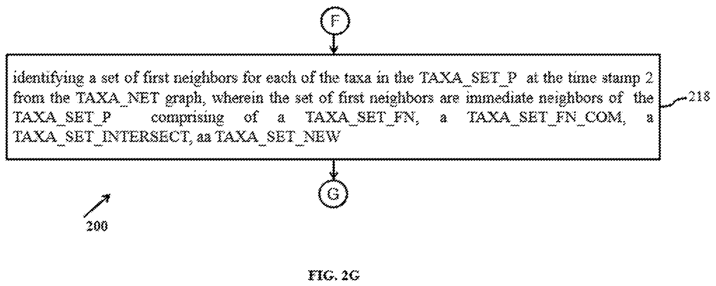

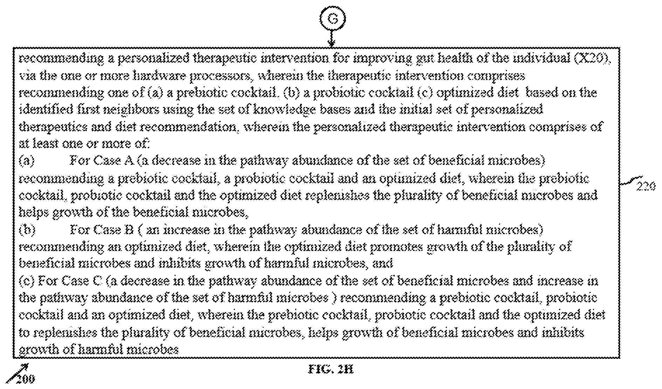

Functions of the functional modules of the system 100 stored in the memory 102 and further explained in conjunction with flow diagram of FIG.2A, FIG.2B, FIG.2C, FIG.2D, FIG.2E, FIG.2F, FIG.2G and FIG.2H. The FIG.2A, FIG.2B, FIG.2C, FIG.2D, FIG.2E, FIG.2F, FIG.2G and FIG.2H with reference to FIG.1, is an exemplary flow diagram illustrating a method 200 for designing personalized therapeutics and diet based on functions of microbiome using the system 100 of FIG. 1 according to an embodiment of the present disclosure.

The steps of the method of the present disclosure will now be explained with reference to the components of the system (100) for designing personalized therapeutics and diet based on functions of microbiome the flow diagrams as depicted in FIG.2A, FIG.2B, FIG.2C, FIG.2D, FIG.2E, FIG.2F, FIG.2G and FIG.2H. Although process steps, method steps, techniques or the like may be described in a sequential order, such processes, methods and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method (200), a set of input data is received at the Inout/Output interface 106.

The set of input data comprises data associated with a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts, a plurality of diet optimization pre-requisite parameters.

The set of input data comprises data associated with the plurality of metabolites comprises a set of beneficial metabolites and a set of harmful metabolites, wherein the set of beneficial metabolites is beneficial for gut health of an individual and the set of harmful metabolites is harmful for the gut health of the individual.

The set of input data further comprises data associated with the plurality of metabolic pathways comprises one of (a) a beneficial pathway (b) harmful pathways.

The set of input data further comprises the beneficial pathways comprise one or more of a set of metabolic pathways for biosynthesis of plurality of beneficial metabolites and a set of metabolic pathways for degradation of the plurality of harmful metabolites.

The set of input data further comprises the harmful pathways comprise one or more of a set of metabolic pathways for degradation of plurality of beneficial metabolites and a set of metabolic pathways for biosynthesis of the plurality harmful metabolites.

The set of input data further comprises the plurality of beneficial microbes harbors the beneficial pathways and the plurality of harmful microbes harbors the harmful pathways.

The set of input data further comprises the plurality of taxonomic groups comprise a plurality of microbes, where the plurality of microbes comprises a plurality of beneficial microbes , a plurality of harmful microbes, where the plurality of microbes are classified according to a taxonomic hierarchy, wherein the taxonomic hierarchy comprises one or more of a plurality of bacterial phyla, a plurality of bacterial classes, a plurality of bacterial orders, a plurality of bacterial families, a plurality of bacterial genera, a plurality of bacterial species and a plurality of bacterial strains, where the plurality of microbes comprises a plurality of genes having distinct genomic location, a set of nucleotide and protein sequences of the bacterial strains.

The set of input data further comprises the plurality of diet optimization pre-requisite parameters comprises a set of food items, a complete set of compounds that are found in the set of food items, a food-constituent matrix, wherein the food-constituent matrix tabulates the content of every compound belonging to the set compounds in every food item belonging to the set foods, the total carbohydrate, protein, fats and caloric content of the each of the food items from the set of food items, a list of bacterial taxa in the human gut, a set of functions to identify a set of beneficial bacteria taxa and a set of harmful bacterial taxa, a taxa-compound utilization matrix, a user defined set of upper bound parameters-lower bound parameters associated with carbohydrate, protein, fat and calorie, and a user selected food items.

A list ML of metabolites which are important for the maintenance of health of gut or those which can be harmful for gut health is catalogued using a literature mining approach. In this disclosure gut health refers to a balance in gut microbiome and thereby their functions which help in maintaining wellness in an individual. In an embodiment, a query string is used as input to search against literature engines like but not limited to Pubmed or Pubtator or in a patent search engine. In an embodiment, the query string might be 'Gut + Health + Metabolites' or 'Metabolite name + Gut'. The metabolites indicated to be beneficial for gut health in literature included Short Chain Fatty Acids like Butyrate, Propionate, Acetate. The pathways leading to ammonia release like Urea metabolism by Urease enzyme, Amino acid metabolism of undigested proteins that reach the gut, metabolism of biogenic amines like putrescine, spermine,cadaverine etc. were found to be harmful for gut health. Similarly, an increase in metabolites like Trimethylamine Oxide and Phenylacetate is found to be implicated in cardiovascular and liver diseases respectively. The list ML also contains information about whether a metabolite is reported in literature to be harmful or beneficial for gut health.

The pathway for formation/degradation/metabolism of each metabolite (MP) comprising ML is obtained by state of art literature mining techniques. Further the genes comprising a metabolic pathway (MP) might have homologs in various other pathways within the same bacterial genome. Therefore, it is important to differentiate the homolog involved in MP, also termed from now on as cognate homolog, from its other homologs. In an embodiment, genes encoding protein or enzymes which function as a pathway and are involved in degradation or formation or metabolism of a compound often occur in juxtaposed positions on the bacterial genome and these genes are together termed as operons or gene clusters. The occurrence of genes encoding metabolic pathways in gene clusters can be utilized to identify the cognate homolog belonging to the pathway from its other homologs on the genome. A query string Q is used as input for metabolite to search against literature search engines which are repositories for scientific literature and may include but are not limited to Pubmed/ Pubtator or pathway repositories like KEGG.

['NameString'] + [Gene cluster OR operon] + [Microbe] where NameString = $(M_i)$ + [Degradation OR Biosynthesis OR Metabolism] or any patent literature search engine.

Usage of any combination of the query string or any substrings from the query string is also within the scope of the invention. The result set obtained from literature search in these databases provides the list of abstracts $(A_{out})$ corresponding to each journal publication as output along with the list of organisms $(ORG_{out})$ in which the pathway is experimentally characterized. Use of any other databases for obtaining pathway information as well results of literature mining in any other format is within scope of the invention. In one embodiment, the formats for results of literature mining can be one or more from full publication, review article, online information, book articles. Further, a manual search of the pathway(s) for metabolite Mi degradation/biosynthesis (which is a series of steps or chemical reactions of degradation/biosynthesis of candidate metabolite encoded by the genes on a bacterial genome) and the genes/ enzymes involved in the process is done by utilizing $A_{out}$ for each input query string Q in the previous step. This process results in a matrix Metabolite Map (Mmap) with each metabolite as a row and its biosynthesis/ degradation pathway and the organisms in which the pathway was characterized forming the corresponding columns. An example scenario of a prototype table for Mmap is shown as Table.1 below:

**Table 1 : Mmap prototype**

| Metabolite | Pathway | Degradation/ Biosynthesis/ Metabolism | List of Organisms obtained from literature |
|---|---|---|---|
| M1 | P1 | Degradation | O1, O2, O3 |
| M2 | P3 | Biosynthesis | O4, O5 |
| M3 | P4 | Degradation | O7, O8, O10 |
| M4 | P7 | Metabolism | O9, O11, O19 |
| ... | ... | .... | .... |

In an embodiment, a metabolic pathway found in beneficial microbes (found in commensal bacteria) which biosynthesize beneficial metabolites are a set of reactions leading to Butyrate production through pyruvate as an initial substrate. Further the commensal or beneficial microbes also possess pathways for degradation of harmful metabolites as well. In an embodiment one such pathway comprises of a set of metabolic reactions leading to Ammonia oxidation. Any other pathway responsible for biosynthesis of metabolites beneficial for health or degradation of metabolites (such as, TMAO) harmful for health are within the scope of being termed as "beneficial pathways" in this invention.

Butyrate production through pyruvate pathway: The substrate pyruvate is converted to Crotonyl CoA through three subsequent steps catalyzed by the three enzymes namely thiolase (Thl, EC 2.3.1.9), hydroxybutyryl dehydrogenase (Hbd, EC 1.1.1.157) and cronotase/enoyl-CoA hydratase (Cro, EC 4.2.1.17). The product from this step Crotonyl CoA is further converted to Butyryl-CoA by butyryl-CoA dehydrogenase (Bcd). Butyryl CoA is metabolized to Butyrate through either of the two routes - (i) with the help of two enzymes namely, phosphate butyryl transferase (Ptb) and butyrate kinase (Buk), (ii) with the help of butyryl-CoA acetate CoA transferase (But). Butyrate production through pyruvate pathway is specifically found in commensal bacteria and this pathway has been referred to as 'beneficial pathway' throughout the description.

Ammonia oxidation pathway: Oxidation of Ammonia releases Nitrate through a three-step process. The enzyme Ammonia monooxygenase (Amo, EC 1.14,99.39) catalyzes the conversion of Ammonia to Hydroxlamine, which is then converted to Nitrite by hydroxylamine dehydrogenase (Hao, EC 1.7.2.6). Nitrite further is metabolized to Nitrate by nitrate reductase (EC 1.7.5.1). In an alternate process, oxidation of Ammonia produces Carbamoyl phosphate by the enzyme carbamoyl-phosphate synthase (EC 6.3.4.16). Ammonia oxidation pathway has been referred to as 'beneficial pathway' throughout the specification.

In an embodiment, the set of genes encoding a metabolic pathway found in microbes which are considered as pathogens which biosynthesize metabolites which are harmful or deleterious to health or degrade metabolites that are beneficial to health are considered as "harmful pathways". In an embodiment the harmful pathways may include ammonia releasing pathways, biogenic amine production pathways as well as trimethylamine oxide producing pathways. Any other pathway responsible for biosynthesis of metabolites harmful for health or degradation of metabolites beneficial for health are within the scope of being termed as "harmful pathways" in this invention.

Ammonia releasing pathways includes:

(a) Urea degradation: The hydrolysis of urea is catalyzed by the enzyme urease (EC 3.5.1.5) which breaks down urea to form ammonia and carbon dioxide. This pathway, being an ammonia-releasing pathway, has been referred to as 'harmful pathway' throughout the specification.

(b) Lysine degradation: Lysine is metabolized to Crotonyl-CoA by five intermediate steps involving five enzymes which are lysine 2,3-aminomutase (KamA), lysine 5,6-aminomutase (Kam D,E), 3,5-diaminohexanoate dehydrogenase (Kdd), 3-keto-5-aminohexanoate cleavage enzymes (Kce) and 3-aminobutyryl-CoA ammonia lyase (Kal). In the 5th step, ammonia is released as a by-product. The product from this step Crotonyl CoA is further converted to Butyryl-CoA by butyryl-CoA dehydrogenase (Bcd, EC 4.3.1.14). Butyryl CoA is metabolized to Butyrate through either of the following two routes - (i) via two reactions involving two enzymes namely, phosphate butyryl transferase (Ptb, EC 2.3.1.19) and butyrate kinase (Buk, EC 2.7.2.7), (ii) via one reaction involving butyryl-CoA acetate CoA transferase (But, EC 2.8.3.8). Lysine degradation pathway, being an ammonia-releasing pathway, has been referred to as 'harmful pathway' throughout the specification.

(c) Glutarate degradation: 2-oxoglutarate is converted to Crotonyl-CoA through four intermediate steps catalyzed by four enzymes which are 2-hydroxyglutarate dehydrogenase (L2Hgdh, EC 1.1.99.2), glutaconate-CoA transferase (Gct, 2.8.3.12), 2-hydroxyglutaryl-CoA dehydrogenase (HgCoAd, EC 4.2.1.-) and Glutaconyl-CoA decarboxylase (Gcd 4.1.1.70). In the 1st step, ammonia is released as a by-product. Crotonyl CoA is further converted to butyryl-CoA by butyryl-CoA dehydrogenase (Bcd, EC 4.3.1.14). Butyryl CoA is metabolized to butyrate through either of the two routes - (i) with the help of two enzymes namely, phosphate butyryl transferase (Ptb, EC 2.3.1.19) and butyrate kinase (Buk, EC 2.7.2.7), (ii) with the help of butyryl-CoA acetate CoA transferase (But, EC 2.8.3.8). Glutarate degradation pathway, being an ammonia-releasing pathway, has been referred to as 'harmful pathway' throughout the specification.

(d) Histidine degradation: Histidine is converted to Glutamate through four intermediate steps. In the first step, Histidine is metabolized to Urocanate by the enzyme histidine ammonia-lyase (Hal, EC 4.3.1.3) and in this step ammonia is released as a by-product. Urocanate is converted to 4-Imidazolone-5-propanoate by urocanase (UroC1, EC 4.2.1.49). The product is further metabolized to N-Formimino-L-glutamate with the help of imidazolonepropionase (AmdhD1, EC 3.5.2.7). The product is then converted to Glutamate through either of the two enzymes - glutamate formimidoyltransferase (FtcD, EC 2.1.2.5) or formimidoylglutamase (HutG, EC 3.5.3.8). Histidine degradation pathway, being an ammonia-releasing pathway, has been referred to as 'harmful pathway' throughout the specification.

(e) Tetrahydrofolate (THF) production: Histidine is converted to Glutamate through four intermediate steps. In the first step, Histidine is metabolized to Urocanate by the enzyme histidine ammonia-lyase (Hal, EC 4.3.1.3) and in this step ammonia is released as a by-product. Urocanate is converted to 4-Imidazolone-5-propanoate by urocanase (UroC1, EC 4.2.1.49). The product is further metabolized to N-Formimino-L-glutamate with the help of imidazolonepropionase (AmdhD1, EC 3.5.2.7). N-Formimino-L-glutamate is converted to N-Formimino-tetrahydrofolate by the enzyme glutamate formimidoyltransferase (FtcD, EC 2.1.2.5) and the coenzyme tetrahydrofolate. The product is then metabolized to 5-10-Methyl-tetrahydrofolate with the help of the enzyme formimidoyltetrahydrofolate cyclodeaminase (FtcD, EC 4.3.1.4). Tetrahydrofolate production pathway, being an ammonia-releasing pathway, has been referred to as 'harmful pathway' throughout the specification.

(f) Glutamate degradation: Glutamate is metabolized to acetate and pyruvate through four intermediate steps. Glutamate is first converted to L-threo-3-methylaspertate involving the enzyme methylaspertate mutase (MutE, EC 5.4.99.1). The product from this step is converted to Mesaconate by methylaspertate ammonia-lyase (Mal, EC 4.3.1.2) and subsequently Ammonia gets released as a by-product. Mesaconate is metabolized to S-citramalate by the enzyme mesaconate hydratase (EC 4.2.1.34). S-citramalate is then converted to acetate and pyruvate by citramalate lyase (EC 4.1.3.22). Glutamate degradation pathway, being an ammonia-releasing pathway, has been referred to as 'harmful pathway' throughout the specification. Trimethylamine TMA Metabolism: TMA metabolism consist of two set of processes Trimethylamine N-oxide (TMAO) degradation and TMAO biosynthesis. (a) TMAO biosynthesis: TMA can be converted to TMAO which a harmful metabolite, by the enzyme trimethylamine monooxygenase (Tmm, EC 1.14.13.148) in one reaction. TMA can also be converted to dimethylamine by the enzyme trimethylamine dehydrogenase (Tmd, EC 1.5.8.2). Dimethylamine is then degraded to methylamine by dimethylamine monooxygenase (DmnABC, EC 1.14.13.238). The enzyme methylamine dehydrogenase (EC 1.4.9.1) further degrades methylamine to release Ammonia and Formaldehyde. These steps in the mentioned pathway lead to formation of harmful metabolites and by-products and thus has been referred to as 'harmful pathway' throughout the specification.

(b) TMAO degradation: TMAO can be converted to TMA involving TMAO reductase (EC 1.7.2.3). Additionally, the enzyme trimethylamine-oxide aldolase (EC 4.1.2.32) can also degrade TMAO to Dimethylamine and Formaldehyde. TMAO is a harmful metabolite and thus its degradation pathway has been referred to as 'beneficial pathway' throughout the specification.

Biogenic amine production pathways includes:

(a) Putrescine production: Putrescine is produced from Arginine via three pathways which are - (i) Arginine is converted to Ornithine by the enzyme arginase (EC 3.5.3.1). Ornithine is then metabolized to Putrescine by ornithine decarboxylase (Odc1, EC 4.1.1.17). (ii) Arginine is converted to Agmatine involving the enzyme arginine decarboxylase (EC 4.1.1.19). Agmatine is then metabolized to Putrescine by agmatinase (EC 3.5.3.11). (iii) Arginine, after getting converted to Agmatine can also be metabolized to N-carbamoyl putrescine involving agmatine deiminase (EC 3.5.3.12). N-carbamoyl putrescine is further converted to Putrescine by N-carbamoylputrescine amidase (EC 3.5.1.53). Putrescine production pathway has been referred to as 'harmful pathway' throughout the specification.

(b) Spermine production: Putrescine can be metabolized to spermine (or also spermidine) with the help of the enzyme spermidine synthase (SpeE, EC: 2.5.1.16). Spermine production pathway has been referred to as 'harmful pathway' throughout the specification.

(c) Cadaverine production: Cadaverine is produced from Lysine by the enzyme lysine decarboxylase (EC 4.1.1.18). Cadaverine production pathway has been referred to as 'harmful pathway' throughout the specification.

In an embodiment, the microbial strains harboring the said harmful pathways are termed as pathogens or harmful microbes while the microbes harboring the said beneficial pathways are termed as beneficial microbes or commensals.

At step 204 of the method (200), a set of knowledge bases is generated using the set of input data. The set of knowledge bases comprises a genus function map (FGmap), a genome metabolite pathway (GMP) map, a taxa association reference profile (TAXA_NET) graph, a TAXA-food constituent network, a food-constituent network and a ProbMap wherein the generation of the set of knowledge bases comprises several steps explained using steps 204A to 204E below:

At step 204A of the method (200), the FGmap and the GMP map is generated.

The FGmap is a matrix associated with a function of the plurality of bacterial genera belonging to one of the plurality of taxonomic group and the plurality of metabolic pathways as columns. The first pre-defined indicator indicates one of :

(a) a presence of the metabolic pathway and
(b) an absence of the metabolic pathway.

In an embodiment, the first pre-defined indicator indicates a presence of the metabolic pathway or an absence of the metabolic pathway, where a value of 1 of the first pre-defined indicator indicates presence of the metabolic pathway and 0 indicates absence of the metabolic pathway in a bacterial genus.

The GMP is a matrix of the plurality of bacterial strains as rows and the plurality of metabolic pathways as columns, wherein a second pre-defined indicator indicates one of (a) a presence of the metabolic pathway and (b) an absence of the metabolic pathway.

In an embodiment, the second pre-defined indicator indicates a presence of the metabolic pathway or an absence of the metabolic pathway, where a value of 1 of the second pre-defined indicator indicates presence of the metabolic pathway and 0 indicates absence of the metabolic pathway in a bacterial strain.

In an embodiment, the first pre-defined threshold criterion is defined based on occurrence of the list of protein domains within a pre-defined window. The second pre-defined threshold criterion is defined based on a threshold ratio of the biosynthesis/ degradation pathways. An example scenario of prototypes for GMP and FGmap are shown in Table 2 and Table 3 respectively below:

**Table 2 : Prototypes for GMP.**

| Genome/ Org/ Strain | P1 | P2 | P3 | P4 | P5 | ..... |
|---|---|---|---|---|---|---|
| Genome1/ Org1/ S1 | 0 | 1 | 0 | 1 | 1 | ... |
| Genome2/ Org2/ S2 | 1 | 0 | 0 | 1 | 1 | ... |
| Genome3/ Org3/ S3 | 0 | 1 | 1 | 1 | 0 | ... |
| Genome4/ Org4/ S4 | 1 | 0 | 0 | 0 | 1 | ... |
| Genome5/ Org5/ S5 | 0 | 1 | 1 | 0 | 0 | ... |
| ..... | ... | ... | ... | ... | ... | ... |

**Table 3 : Prototypes for FGmap.**

| Genus | P1 | P2 | P3 | P4 | P5 | .... |
|---|---|---|---|---|---|---|
| Genus 1 | 1 | 1 | 0 | 1 | 0 | ... |
| Genus 2 | 1 | 0 | 1 | 0 | 1 | ... |
| Genus 3 | 0 | 1 | 0 | 1 | 0 | ... |
| Genus 4 | 1 | 1 | 1 | 0 | 1 | ... |
| Genus 5 | 0 | 1 | 1 | 1 | 0 | ... |
| .... | ... | ... | ... | ... | ... | ... |

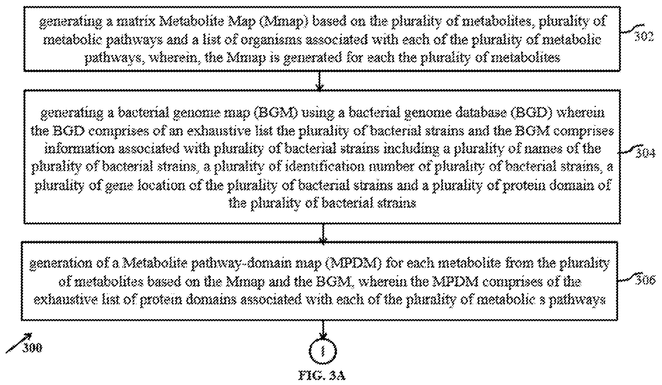

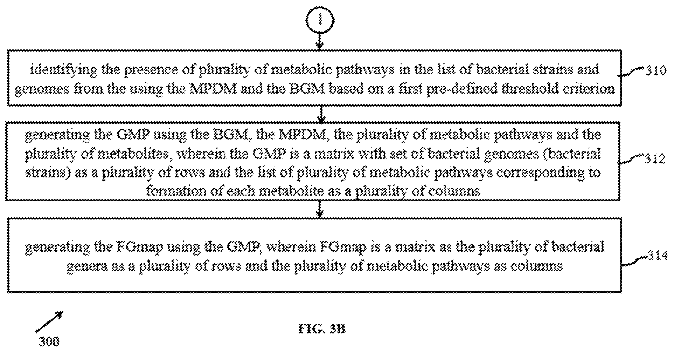

The process for the generation of the GMP is explained in conjunction with flow diagram of FIG.3A and FIG.3B.

At step 302 of the method (300), a matrix Metabolite Map (Mmap) is generated. The Mmap is generated based on the plurality of metabolites, plurality of metabolic pathways and a list of organisms associated with each of the plurality of metabolic pathways, wherein, the Mmap is generated for each the plurality of metabolites. The Mmap is generated for each plurality of metabolites in the list ML, the details of the metabolic pathways involved in the degradation or biosynthesis of each of these plurality of metabolites as has been obtained after the literature mining step, as well as the details of organisms in which the said metabolic pathways have been experimentally characterized in. the said details of metabolic pathway include the genes/enzymes/proteins involved in individual reactions of the pathway.

In an embodiment, post a manual search of the pathway(s) for metabolite Mi degradation/biosynthesis (which is a series of steps or chemical reactions of degradation/biosynthesis of candidate metabolite encoded by the genes on a bacterial genome) and the genes/ enzymes involved in the process is done by utilizing ($A_{out}$)for each input query string Q in the previous step. This process results in a matrix Metabolite Map (Mmap) with each metabolite as a row and its degradation pathway and the organisms in which the pathway was characterized forming the corresponding columns. An example scenario of a prototype table for Mmap is shown as Table.4 below:

**Table 4 : Mmap prototype**

| Metabolite | Pathway | Degradation/ Biosynthesis/ Metabolism | List of Organisms obtained from literature |
|---|---|---|---|
| M1 | P1 | Degradation | O1, O2, O3 |
| M2 | P3 | Biosynthesis | O4, O5 |
| M3 | P4 | Degradation | O7, O8, O10 |
| M4 | P7 | Metabolism | O9, O11, O19 |
| ... | ... | .... | .... |

At step 304 of the method (300), a bacterial genome map (BGM) is generated. The BGD comprises of an exhaustive list the plurality of bacterial strains and the BGM comprises information associated with plurality of bacterial strains including a plurality of names of the plurality of bacterial strains, a plurality of identification number of plurality of bacterial strains, a plurality of gene location of the plurality of bacterial strains and a plurality of protein domain of the plurality of bacterial strains.

The BGD comprises of an exhaustive list of genomic location and corresponding functional protein sequences and the BGM is a functional annotation of gene in form of a constituent functional protein sequences. The BGD comprises of an exhaustive list of the bacteria (strain names) for which the genomes/genomic sequences were used in analysis. Bacterial Genome Map (BGM) comprises of the list of genes in each of the bacterial genomes enlisted in BGD arranged as per the order in terms of the genomic locations and information about functional annotations of each of these genes in the form of constituent protein domains identified in each gene.

In an embodiment, an example scenario of a sample BGM map shown below in Table 5

**Table 5 : A sample BGM map**

| Sr. No. | Genome ID | Organism name | Gene ID | Domains | Gene location |
|---|---|---|---|---|---|
| 1 | Genome1/ Org1/ S1 | Strain XXXX | Gene 1 | D1, D10, D11 | 12--112 |
|  |  |  | Gene 2 | D2, D13 | 114--259 |
|  |  |  | Gene 3 | D3, D14 | 312-555 |
|  |  |  | ...... | ............ | ...... |
| 2 | Genome2/ Org2/ S2 | Strain YYYY | Gene 1 | D1 | 150--306 |
|  |  |  | Gene 2 | D6, D15, D17 | 459--673 |
|  |  |  | Gene 3 | D8, D18 | 682--1245 |
|  |  |  | ...... |  | ...... |
| 3 |  | Strain ZZZZ | Gene5 | D9 | 763--7998 |
|  | Genome3/ Org3/ S3 |  | Gene7 | D4, D19, D20 | 223--569 |
| ..... | ..... | ..... | ...... |  | ...... |

In an embodiment, information is obtained for each bacterial genome pertaining to its translated protein sequences for each gene and its location on the bacterial genomes from the repository National Centre of Biotechnology Information (NCBI). The database of the bacteria (strain names) for which the genomes were used in analysis is termed as Bacterial Genome Database (BGD). The protein sequences are utilized in FASTA format and the feature files in NCBI repository are utilized to ascertain the gene locations, while any other format or source of gene locations and protein sequences for microbial genomes is within scope of invention. These bacterial genomes are functionally annotated to identify protein domains within each gene on the genome using multiple methods which may include but are not limited to gene homology (BLAST etc), Hidden Markov Model based identification (Protein Family or PFAM Database etc.), Position Specific Scoring matrices (PSSM) etc. In one embodiment, the database PfamDB (or protein domain family database) comprising HMMs corresponding to all protein domains can be obtained as taught in PFAM database and the genomes can be searched using publicly available software like HMMER. The use of any other protein domain identification method or functional protein annotation method or database or structure-based protein identification method is also well within the scope of this disclosure. The list of genes in each of the bacterial genomes enlisted in BGD arranged as per the order in terms of the genomic locations as listed in the said NCBI feature files as listed in the said NCBI feature files (starting from the origin of replication) is termed as Bacterial Genome Map (BGM). BGM also contains information about functional annotations of each of these genes in the form of constituent protein domains identified in each gene

At step 306 of the method (300), a Metabolite pathway-domain map (MPDM) is generated. The Metabolite pathway-domain map (MPDM) is generated for each metabolite from the plurality of metabolites based on the Mmap and the BGM, wherein the MPDM comprises of the exhaustive list of protein domains associated with each of the plurality of metabolic s pathways.

In an embodiment, The MPDM comprises of each pathway PiM corresponding to each metabolite Mi in ML and the corresponding protein domains comprising of each of these pathways. An example scenario of a prototype for MPDM is provided in Table 6:

**Table 6 : Prototype for MPDM**

| Sr. No . | Pathway | Metabolite | Domains |
|---|---|---|---|
| 1 | P1 | M1 | D1 |
| | | M3 | D2 |
| | | M9 | D3 |
| | | | .... |
| 2 | P2 | M2 | D2 |
| | | | D5 |
| | | M5 | D9 |
| | | | ...... |

In an embodiment, the Metabolite pathway-domain map (MPDM) comprises all metabolite (in Mmap) degradation and biosynthetic pathways and the domains corresponding to each of these pathways.

In an embodiment, considering each pathway as the key found in the hash MPDM, the value corresponding to the key is a list of corresponding domains in the pathway. The genes belonging to a microbial pathway often occur in proximity on their corresponding genomes and are termed as gene clusters. The distance on the genome in terms of the number of flanking genes within which the set of domains forming a pathway should lie in order to form a functional gene cluster varies and is often defined using manual and literature-based curation. This said distance in this embodiment is defined in terms of the number of genes based on their genomic locations (termed as window size) within which the domains should lie in order to indicate a gene cluster and therefore indicate the pathway presence. Each protein domains (pfams) corresponding to values in hash MPDM for each pathway used as a key is searched in the BGM to find if the protein domains comprising a metabolite biosynthesis/ degradation pathway are present as gene clusters on each genome enlisted in BGM.

At step 308 of the method (300), the presence of plurality of metabolic pathways is identified in the list of bacterial strains and genomes using the MPDM and the BGM based on a first pre-defined threshold criterion.

In an embodiment, the first pre-defined threshold criterion is defined based on occurrence of the list of protein domains within a pre-defined window. Any set of domains are considered to form a gene cluster if they are located within a defined window size on the genome. Window size refers to the number of genes upstream and downstream of a domain known to be a part of a pathway within which all other domains of the pathway must lie in order to accurately annotate the gene cluster. In one embodiment, first pre-defined threshold criterion for the pre-defined window is defined as a window size of 20 genes upstream and downstream of a query protein domain (which refers to any one protein domain within a metabolic pathway) on the genome is utilized. The gene name and pfam database based domain assignment is recorded for presence of other protein domains in a -pathway within the window (20 in this case). Window size can be variable depending on various factors like the candidate pathways and the domains involved. Further, a pathway is considered to be present if the number of domains in the genome contributing to this pathway and occurring within the window size (e.g. if 20 genes is window size then +20 and -20 genes of the query protein domain) crosses a threshold value (variable for each pathway and obtained using literature mining and manual curation) which is the ratio corresponding to minimum number of domains whose presence is necessary to confirm existence of pathway out of the total number of domains assigned in the MPDM corresponding to this pathway

At step 310 of the method (300), the GMP is generated using the BGM, the MPDM, the plurality of metabolic pathways and the plurality of metabolites. The GMP is a matrix with set of bacterial genomes (bacterial strains) as a plurality of rows and the list of plurality of metabolic pathways corresponding to formation of each metabolite as a plurality of columns.

In an embodiment, after identification of the list of biosynthetic-degradation pathway for the list of genomes from the BGM based on the first pre-defined threshold criterion, a multi-dimensional matrix is created with genome names and pathway information for each metabolite. This is called as the genome-metabolite-pathway map (GMP).

The GMP map is given a value of either 0 or 1 for each metabolite and pathway based on a first predefined criterion. The first predefined criterion is for each pathway in a bacterial genome. In case of bacterial genomes where the number of domains corresponding to the pathway in MPDM either do not occur on the genome or the number found within the defined window size (20 in this case) does not cross the threshold value, a value of 0 is assigned corresponding to that genome and the pathway in GMP. In one embodiment, the window size can be 20 genes occurring consecutively on the microbial genome. If the number of pathway protein domains found on a genome are above the threshold value (defined for candidate pathway using literature mining and manual curation) and are present within the defined window size (20 genes in this case) on a microbial genome, a value of 1 is assigned for the corresponding genome and the pathway in matrix GMP.

Window size can be variable depending on the system and the candidate pathway. In an embodiment, the presence of a metabolic pathway in the microbial genome in GMP is indicated by a predefined value of 1 and absence of the pathway in the genome in given a predefined value of 0. In another embodiment wherein the predefined values are values other than 1 and 0 are also within the scope of the invention.

At step 312 of the method (300), the FGmap is generated using the GMP. The FGmap is a matrix as the plurality of bacterial genera as a plurality of rows and the plurality of metabolic pathways as columns.

The FGmap is generated based on the GMP. The FGmap is a matrix comprising of a set of genera as a plurality of rows and the list of biosynthetic-degradation pathways for formation of each metabolite as a plurality of columns. The process of generation of FGmap comprises processing the GMP at a genus level based on a second pre-defined threshold criterion and the BGM. The FGmap is represented as a matrix comprising of a set of genera as rows and the list of biosynthetic-degradation pathways for formation of each metabolite as columns.

In an embodiment, in cases where input taxonomic abundance is obtained at genus level, the GMP can be further processed to identify pathway composition at a genus level. The presence of a pathway in each strain belonging to a genus is analyzed. The pathway is considered to be present in a genus Ge if the ratio of number of strains of Genus Ge possessing a pathway out of the total sequenced strains belonging to a genus Ge in BGD increases the second pre-defined threshold criterion. The second pre-defined threshold criterion value is determined using manual interventions also as the number of strains sequenced for a genus may vary and the threshold needs to be decided accordingly. In an embodiment, the second pre-defined threshold criterion can be taken as 0.7, wherein the pathway can be considered to be present in a genus Ge if 70% of strains of Genus Ge whose sequences are available in repositories for that genus contain the pathway. Different threshold values depending on manual analyses as well as literature mining can be decided and are within the scope of this disclosure. The genus-pathway binary matrix so obtained contains a value of 0 (which is a predefined value) if a pathway is not found to occur in strains belonging to that genus above a threshold and value of 1 (which is a predefined value) if the number of strains in which a pathway is found is more than a threshold value. This matrix is termed as Fgmap. In another embodiment, he predefined values that are values other than 0 and 1 are also within the scope of the invention. The GMP and FGmap together comprise the microbe knowledge base module 204 and are provided with another column mentioning whether a metabolite is known in literature as beneficial or deleterious to gut health as listed in list ML. In an embodiment total number of strains within a genus Ge can be obtained by a genome repository where genomic sequences of bacterial strains are deposited. In one embodiment, the repository might include NCBI or HMP genome repositories.

Referring to FIG.2B, at step 204B of the method (200), the TAXA_NET graph is generated. The TAXA_NET graph based on the plurality of taxonomic groups and the FGmap, wherein the TAXA_NET graph is an undirected graph. Each node in the TAXA_NET graph corresponds to a taxonomic group from the plurality of taxonomic groups and edges indicate a positive relationship between a pair of the taxonomic group wherein each taxa from a plurality of nodes is associated with the function of the plurality of taxonomic groups from the FGmap, wherein the positive relationship comprises one of a mutualism, a syntrophic, a commensalism and a cooperation

In an embodiment, a taxa association reference profile TAXA_NET is constructed using experimentally reported microbial abundance data collated from multiple cohorts in healthy human gut. The TAXA_NET is enriched with functional information and evidence mining, wherein the said functional information can be obtained from the GMP and FGmap. Each taxa in TAXA_NET is associated with a function based on genus-function or genome function mapping described in these matrices. Further function information is added to each taxa on the basis of automated literature mining or by manual curation. The TAXA_NET is an undirected network with nodes as taxon names which are connected by an edge if a positive relationship (including but not limited to mutualism, syntrophic, commensalism or cooperation) is identified between a pair of microbial taxa.

At step 204C of the method (200), the TAXA-food constituent network is generated. The TAXA-food constituent network is generated based on a plurality of food constituents for each taxa of the TAXA_NET. The TAXA-food constituent network is an undirected network of each taxa in association with a food constituent used by the taxa as a source of metabolism.

In an embodiment, an undirected network of taxa association with food constituents that is used by the microbial taxon as a source of metabolism (e.g., carbon and nitrogen source) is constructed and named as TAXA_CONSTITUENT_NET.

At step 204D of the method (200), the food-constituent network is generated. The food-constituent network is generated based on a plurality of food items for each food constituent of the TAXA-food constituent network. The food-constituent network is an undirected network of each food-constituent present in a food item.

In an embodiment, an undirected network of food association with food constituents (e.g., carbon and nitrogen source) is constructed and named as FOOD_CONSTITUENT_NET.

At step 204E of the method (200), the ProbMap is generated. The ProbMap is generating a transpose of the GMP, wherein the ProbMap is a matrix with rows comprising each metabolite from the plurality of metabolites and the plurality of metabolic pathways and columns comprising the plurality of bacterial strains, probiotics, prebiotics, diet components capable of biosynthesis and/or degradation of the corresponding plurality of metabolites.

In an embodiment, the Probmap is generated based on the metabolite biosynthesis/ degradation pathway for metabolites as the rows and genomes as columns along with an additional column providing details of the nutrient supplements, dietary information or prebiotics reported in the literature which augment or reduce a metabolite Mi as reported in literature is provided for each biosynthesis/ degradation pathway for metabolites respectively. The additional column also comprises of details of probiotics comprising of bacterial strains which can replenish the corresponding pathway if it is a beneficial pathway and curb the corresponding pathway if it is harmful. The details of these probiotic strains can be obtained by those mentioned in the columns of ProbMap where the corresponding beneficial or harmful pathway has a value of 1. The value of 1 indicates presence of a pathway in that strain.

At step 206 of the method (200), a plurality of gut samples of an individual is obtained at two-time stamps. The two-time stamps comprise a time stamp 1 and a time stamp 2.

In an embodiment, the plurality of gut samples are obtained from at least one of stool, intestinal swab, intestinal tissue or intestinal fluid of the individual at the time stamp 1 and the time stamp 2.

At step 208 of the method (200), a taxa abundance matrix for the plurality of gut samples is generated. The taxa abundance matrix comprises of a taxa set 1 and a taxa set 2, where the taxa set 1 and the taxa set 2 is generated using the time stamp 1 and the time stamp 2.

At step 210 of the method (200), a pathway abundance is generated for each of the plurality of gut-samples obtained at the time stamp 1 and the time stamp 2 using the taxa abundance matrix and the FGmap. The pathway abundance is generated using a sample processing technique, wherein the pathway abundance for each time stamp is indicative of presence of the plurality of metabolic pathways of the GMP in the gut microbiome of the individual at the corresponding time stamp

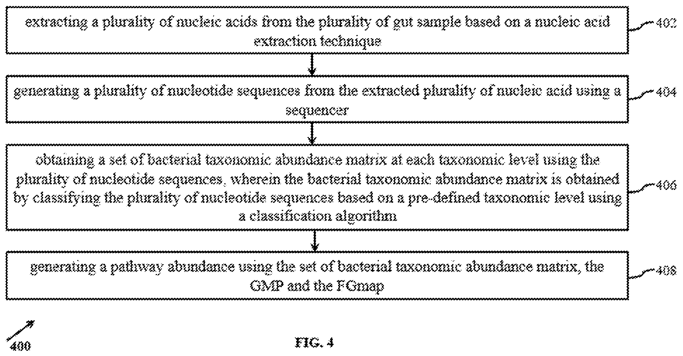

The steps for sample processing technique is explained in conjunction with flow diagram of FIG.4, which is explained below:

At step 402 of the method (400), a nucleic acid is extracted from the obtained gut sample. The extraction of the nucleic acid from the obtained gut sample is based on a nucleic acid extraction technique.

In an embodiment, nucleic acid extraction technique includes extraction of nucleic acid content from the obtained sample using state of art laboratory acceptable methods. In one embodiment, mini-DNA extraction kits provided by companies like Qiagen for extraction of DNA can be utilized.

At step 404 of the method (400), a plurality of nucleotide sequences is generated from the extracted nucleic acid by the sequencer via the nucleotide sequences generation unit 212. The is implemented using a sequencer.

In an embodiment, sequencer is used for sequencing, wherein the sequencing step is performed using high-throughput sequencing techniques. The sequencing results in the generation of a plurality of nucleotide sequences. Nucleic acid content is extracted from the obtained sample using several laboratory acceptable methods. In one embodiment, mini DNA extraction kits provided by companies like Qiagen for extraction of DNA can be utilized. Other extraction kits from Norgen, Purelink, OMNIgene/ Epicenter, etc., can also be utilized. The extracted nucleic acid can be subjected through genotypic assays at genera level for obtaining microbiome composition of the obtained sample. The microbiome composition of the gut sample can be obtained by nucleic acid or protein analysis. Nucleic acid analysis might involve DNA, RNA, mRNA, rRNA and can be performed using several methods like Polymerase Chain Reaction (PCR), qPCR, pyrosequencing, Denaturing gradient gel electrophoresis (DGGE), Restriction Fragment Length Polymorphism, microarrays or next generation sequences. Any other methods capable of detecting nucleic acid sequences or DNA hybridization are within the scope of the invention. Protein analysis might involve different forms of Gel Electrophoresis, Mass spectroscopy, AQUA, iTRAQ etc. Any other method of determining presence of proteins that can be used as indicators of presence of specific bacteria is within the scope of this invention. Any other laboratory acceptable methods for nucleic acid and protein analysis can also be utilized to delineate the composition of gut microbiome in an individual.

At step 406 of the method (400), obtaining a bacterial taxonomic abundance matrix via a plurality of programs and software built into the one or more hardware processors 104.

In an embodiment, the genes considered as markers for bacterial phylogeny such as 16S rRNA can be amplified using methods to classify like Polymerase Chain Reaction (PCR) or qPCR. In one embodiment, the sequenced 16S rRNA can be classified into taxonomic groups and genus or specie level using Naive Bayesian classifier as implemented in Ribosomal Data Project or using sequence-based matches with different 16S rRNA databases as implemented in Ribosomal Database Project or Green genes database. In yet another embodiment an in-house taxonomic database (such as 16S rRNA database) can be created for strains of bacterial genomes which have been sequenced. These databases can be used to classify 16S rRNA sequenced obtained from the gut sample into taxonomic groups at genus or specie level. Any other method of identifying taxonomic composition of the microbiome sample is within the scope of the disclosure.

The taxonomic database stores markers for a plurality of taxa including but not limited to one or more of: genetic sequences (e.g., sequences conserved for a taxonomic group; sequences markers for taxonomic group, sequences invariant for a microorganism group, features like but not limited to microbiome diversity, microbiome functional repertoire including metabolic pathways etc; metabolite markers; natural product or secondary metabolite markers; diagnostic, predictive and molecular biomarkers; or other suitable markers associated with microorganisms (e.g.,taxa)

Genetic sequences stored by the taxonomic database include one or more variable or conserved regions of gene sequences of rRNA corresponding to a plurality of taxonomic groups. These genes may comprise one or more of: 16S, 18S, 30S, 40S, 50S, 60S, 5S, 23S, 5.8S, 28S, 70S, 80S, and/or any other suitable rRNA.

In another embodiment, the taxonomic database can store reference values for relative abundance ranges of different microorganism. These ranges may include the abundances associated with a healthy state for one or more conditions and those associated with an unhealthy state. The said reference values for abundance ranges can be obtained based on a set of values obtained from gut biological samples from a population of individuals.

The reference markers in a gut sample can be based on amplification of a set of primers corresponding to these markers selected on the basis of a characteristic of the marker shared by various taxonomic groups which has variable regions which differ for each taxonomic group. The amplification of primers leads to formation of reads which can be sequenced using a sequencer as described in the disclosure. In an embodiment the reads obtained can be compared to the set of reference markers in the database using the sequence similarity to identify the taxonomic group to which the read belongs.

Further a genus abundance matrix is created which comprises of genera as rows and the corresponding abundance for each input sample. In an embodiment the input sample may be obtained from single or multiple time points from an individual. In another embodiment, the input sample may be obtained from individuals belonging to two distinct groups or populations. In one embodiment the populations or groups may be individuals participating in a case control study. Any other study comprising of subjects belonging to two or more distinct categories are within the scope of invention. Similarly, a specie abundance matrix or a strain abundance matrix is created depending on the taxonomic level at which the 16S rRNA sequences are classified. The matrices are named as M1 and M2 corresponding to samples obtained for two time points for an individual or those from two distinct populations. Further a genus and a species are taxonomic rankings of the biological classification of organisms, wherein the species ranks below the genus in the taxonomic classification hierarchy. Hence abundance tables at each taxonomic level are prepared depending on the taxonomic resolution obtained in a data set using a classification algorithm.

At step 408 of the method (400), obtaining a pathway abundance matrix for the ML at the two-time stamps. Upon obtaining the microbiome composition, the bacterial taxonomic abundance matrix of each sample M1 and M2 are fed into a processor with software instructions to convert them into pathway abundance matrices for each sample. In an embodiment, for the samples obtained for individual at two-time stamps or a population with one matrix representing two phenotypically different populations or groups of subjects. In one embodiment the distinct populations or groups may comprise of one for healthy sub-population and other representing a dysbiotic sub-population. In another embodiment, distinct populations or groups of subjects belong to two stages of a disease during treatment with matched features between populations in terms of geography, age, diet, lifestyle etc. In one embodiment the populations or groups may be individuals participating in a case control study. Any other study comprising of subjects belonging to two or more distinct categories are within the scope of invention. The genus abundance matrices M1 and M2 for said samples can be obtained using methods discussed for obtaining taxonomic abundance matrix. The pathway abundance matrices for two stages can be calculated as follows:

$$\text{Pathway abundance matrix } M1P = FGmap * M1$$

$$\text{Pathway abundance matrix } M2P = FGmap * M2.$$

In another embodiment, the genes which can be used as markers (for example 16S rRNA in bacteria) for phylogeny of a bacteria can be utilized to determine pathway abundances. In one embodiment, this marker may include 16S rRNA in bacteria and the encoded product. The utilization of any other bacterial marker genes such as 23S rRNA, *rpoB, cpn60, gyrB, dnaK, dsrAB, amoA, amoB, mip, horA, hitAM, recA, ica, frc. oxc,* etc., or/and assessment of fungal internal transcribed spacer 1 (ITS1) etc., are within the scope of the invention. The 16S rRNA sequences corresponding to the gut microbiome of an individual can be amplified after DNA/ RNA extraction and sequencing procedure. The sequences so obtained can be matched to the database of 16S rRNA sequences of each of the strains in BGD which stored in memory as SEQB. The match can be performed by processor with software instructions to identify and the closest neighbor. The sequences of the input 16S rRNA corresponding to an individual of population can be matched to sequences in SEQB using sequence alignment software like BLAST, FASTA etc. The pathway composition of the nearest neighbor so obtained can be utilized as the pathways for the input 16S rRNA sequence. The abundance matrix corresponding to each match can be obtained such that the rows comprise of strain names and the columns comprise of the sample names where number of times 16SrRNA sequence corresponding to that strain has been obtained in each sample is recorded. Similar methodology can be used for other said bacterial marker genes in the scope of this disclosure. In an embodiment, for the samples obtained for individual at two-time stamps or a population with one matrix representing two phenotypically different populations or groups of subjects. In one embodiment the distinct populations or groups may comprise of one for healthy sub-population and other representing a dysbiotic sub-population. In another embodiment, distinct populations or groups of subjects belong to two stages of a disease during treatment with matched features between populations in terms of geography, age, diet, lifestyle etc. In one embodiment the populations or groups may be individuals participating in a case control study. Any other study comprising of subjects belonging to two or more distinct categories are within the scope of invention. The strain abundance matrices M1S and M2S for the samples can be obtained where the reads are classified till the strain taxonomic level also.

$$\text{Pathway abundance matrix } M1P = M1S * GMP$$

$$\text{Pathway abundance matrix } M2P = M2S * GMP$$

In another embodiment, the genera (Gi) can be identified to increase or decrease significantly using methods like statistical tests like parametric, non-parametric tests, Machine learning based algorithms including decision trees. Neural networks, random forest etc. for M1 and M2. The abundance matrix corresponding to these differentially abundant genera Gi comprising selected genera as rows and samples as columns can be obtained from M1 and M2 and these matrices can be termed as SM1 and SM2. These genera can be queried against the knowledgebase FGmap using a processor with software instructions which can provide information about metabolites each of these genera is capable of producing.

$$\text{Pathway abundance for SM1 called } SM1G = SM1 * FGmap$$

$$\text{Pathway abundance for SM2 called } SM2G = SM2 * FGmap$$

In an embodiment, after obtaining the pathway abundances for each sample, compare pathway abundances (M1P and M2P). These comparisons can be made by the processor using software instructions for performing statistical tests like parametric, non-parametric tests, Machine learning based algorithms including decision trees. Neural networks, random forest etc. The software programs for analyzing these changes might also involve assessing the fold change within each bacterium between two time points or populations. In another embodiment, only one sample can be collected from an individual, and the abundances of genera in the sample can be compared to the predetermined reference values or a range of values for the corresponding genera in a healthy population. Values can also be compared to reference values or a range of values for abundance of specific genera in a dysbiotic gut microbiome. The genera with abundance values significantly different from the reference values can be used for further analysis. In an embodiment, the processor is configured to analyze and identify metabolites that might significantly increase or decrease between these samples (same individual at two time points or two datasets for populations) on the basis of comparison of pathway matrices and utilization of MPDM where these pathways are mapped to corresponding metabolites. The processor is also configured to analyze if these metabolite changes are statistically significant using statistical techniques mentioned above. Thus, the processor is configured to analyze the taxonomic composition of the microbiome samples and identify metabolites that might significantly increase or decrease between these samples. In an embodiment the input sample may be obtained from single or multiple time points from an individual. In another embodiment, the input sample may be obtained from individuals belonging to two distinct groups or populations. In one embodiment the distinct populations or groups may comprise of one for healthy sub-population and other representing a dysbiotic sub-population. In another embodiment, distinct populations or groups of subjects belong to two stages of a disease during treatment with matched features between populations in terms of geography, age, diet, lifestyle etc. In one embodiment the populations or groups may be individuals participating in a case control study. Any other study comprising of subjects belonging to two or more distinct categories are within the scope of invention. Further the parsing of knowledgebase as well as list ML using software programs within the processor can help to identify whether the metabolites are beneficial or deleterious to human health. In one embodiment, metabolites like Short Chain Fatty Acids including butyrate are well known in literature to be beneficial to human health. Similarly, metabolites like ammonia or bioamines like trimethylamine have been associated to various inflammatory or diseased conditions in humans. The comparison of pathway abundance matrices using the said statistical techniques yield pathways as well as metabolites which are significantly different between two pathway abundance matrices. The said pathways and metabolites are termed as 'probable metabolites' and 'probable pathways' respectively.

In another embodiment, metabolites within the gut sample can be identified. Any laboratory acceptable methods of identifying the metabolites are within scope of the invention. In one embodiment, the gut samples collected can be dried and suspended in water to be further processed by different chromatographic and mass spectrometry techniques including ultra-performance liquid chromatography or high performance liquid chromatography followed by high resolution/ tandem mass spectrometry MS/ MS. In another embodiment, biosensors including Nano sensors and those implemented in e-nose like devices can be used to assess metabolites in fecal matter. Any other method of analyzing and quantitating the metabolites in gut sample are within scope of the invention. Some of the methods of identification and quantitation of metabolites may also include colorimetric assay, chemical assays and enzymatic assays etc. for the metabolite. The metabolite content can be obtained for one sample obtained from an individual, samples obtained from an individual at two time points and samples obtained from two phenotypically different populations. The metabolite content from one sample taken from an individual can be compared to reference values for a healthy individual in order to identify metabolites whose concentrations have increased or reduced within an individual. In cases where the samples correspond to two points for same individual or to two kind of population data, the metabolites within the samples can be compared. The significance of metabolite differences between one sample and reference values, two time points or two populations using processor implemented software for calculations using statistic methods like parametric, non-parametric tests, Machine learning based algorithms including decision trees. Neural networks, random forest etc. The software programs for analyzing these changes might also involve assessing the fold change within each bacterium between two time points or populations.

The personalized therapeutic provides one or more treatments to a user (e.g., a human subject; a health-care professional providing the treatment; etc.) for treating/managing one or more dysbiotic conditions (e.g., risk reduction; improving states of the conditions; improving symptoms and/or other suitable aspects of the conditions; changing a user's microbiome profile towards a state more sensitive to therapies for the diseases; etc.). These might include orally administered medicines, such as probiotic supplements (includes the type, dosage, treatment time, the kind and quantity of microbes included, etc.), food supplements, antibiotics (includes the type, amount, treatment schedule, etc.), medical devices (e.g., medicine dispensers; devices administering antibiotics, etc.), user devices (e.g., biosensors), and/or other appropriate components. Personalized therapies include applications including those that alter/ modify the microbiome composition and/or function and thereby reduce the symptoms or improve the health condition. In a particular example, the treatments can be provided as probiotics-based therapies that are formulated from one or more microbes and/ or their metabolites (e.g., the microbial groups described herein, etc.).

Referring to FIG.2D, at step 212 of the method (200), identifying a perturbed pathway. The perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein the perturbed pathway is identified based on one of below cases:

(a) Case A: a decrease in the pathway abundance of the set of beneficial pathways , wherein the decrease is an indicative of a decrease in the set of beneficial microbes, or
(b) Case B: an increase in the pathway abundance of the set of harmful pathways wherein the increase is indicative of an increase in the set of harmful microbes, or
(c) Case C: a decrease in the pathway abundance of the set of beneficial pathways and increase in the pathway abundance of the set of harmful pathways indicative of an increase in set of harmful microbes and decrease in the set of beneficial microbes.

In an embodiment, the "perturbed pathway" refers to an increase in harmful pathways or biosynthesis of harmful metabolites or degradation of beneficial metabolites or a decrease in beneficial pathways or beneficial metabolites or degradation of harmful metabolites and the perturbed pathway are identified based on statistical techniques including one of a parametric technique, a non-parametric technique, a Machine learning based algorithm.

At step 214 of the method (200), an initial set of personalized therapeutics and diet recommendation is determined. The initial set of personalized therapeutics and diet comprises atleast one of: a personalized therapeutic and a personalized dietary regimen using the ProbMap, wherein the initial set of personalized therapeutics and diet recommendation is a row corresponding to the identified perturbed pathway.

The personalized therapeutic is determined as:

(a) the plurality of bacterial strains possessing the perturbed pathway or
(b) the plurality of bacterial strains possessing the pathway for degradation or/and biosynthesis of a probable metabolite, wherein the probable metabolite is a metabolite corresponding to the perturbed pathway in the ProbMap

In an embodiment, initial set of personalized therapeutics and diet recommendation is determined using the ProbMap. In an embodiment significant change in a metabolite or pathway may refer to statistically significant changes observed on comparison of pathway matrices for samples obtained at two time points from same individual. In another embodiment, the significant change may refer to statistically significant change in the levels of a certain metabolite within the gut samples obtained from an individual at two points. Statistical significance refers to rules of statistical hypothesis testing well known in the art, where a feature is very unlikely to have occurred given the null hypothesis. The significant change may also be referred to as features identified to be important for discrimination of two states using machine learning methods, supervised or unsupervised, neural networks, regression methods etc. or any other methods known in the art to ascertain features important to decipher functions different between samples obtained from set of individuals which belong to distinct classes, where classes may correspond to different phenotypic or physiological characteristics in individuals.

In an embodiment, classes may constitute healthy and individuals suffering from a disease. In another embodiment, classes may involve samples of individuals taken before and after a treatment regimen at different time points. Further, a fold change of predefined value can also indicate significant change in a particular function between two sample time points or samples obtained from phenotypically different populations.

Depending on the metabolites and pathways identified which show significant changes on comparison of pathway abundance matrices, the interventions or therapeutic regimes of probiotics can be accordingly designed. In cases where a reduction of beneficial metabolite or its biosynthesis pathway is observed, probiotics will be expected to comprise of bacteria which biosynthesize this metabolite and are capable of surviving in the gut environment. On the contrary, in cases where an increase in a deleterious metabolite, its biosynthetic pathway is observed or a decrease in its degradation pathway is seen, microbes capable of degrading the metabolite might form a more efficient probiotic. The information about bacterial strains capable of biosynthesizing and degrading specific metabolites has already been catalogued in matrix GMP and matrix FGmap and stored in the memory as a backend database. Further information about nutrient supplements, prebiotics, dietary components etc. which can increase the production or degradation of specific metabolites have been included in these matrices to create ProbMap using information obtained from mining literature. For e.g. obtaining more resistant starch in the diet can increase commensal butyrate producers within the microbiome. Similarly, reduction in animal protein diet helps in reduction of harmful ammonia as well as carnitine in gut. The information pertaining to dietary intake or nutrient supplements required for replenishing beneficial metabolites diminished in an individual as well as for reducing harmful metabolites present in significantly higher amount in an individual has been obtained using literature mining and manual curation. This information for each metabolite in FGmap has been added as columns in ProbMap. Therefore, Probmap comprises of information included in Genome-Metabolite-Pathway GMP matrix in a form of transpose of this matrix where Metabolite biosynthesis or degradation pathway for metabolites $M_i$ in Mmap forms the rows while genomes are taken as columns. In addition to this, a column providing details of the nutrient supplements, dietary information or prebiotics reported in the literature which augment or reduce a metabolite $M_i$ as reported in literature has been added to each row corresponding to that metabolite biosynthesis or degradation respectively. Thus on the basis of the metabolites or their biosynthetic/degradation pathways seen to significantly increase/ decrease within an individual with data collected over one or more time points or within two populations, the ProbMap database can be queried to design function based personalized probiotics and dietary regimens. An example scenario of a prototype for ProbMap database is provided below in Table 7 :

**Table 7 : Prototype for ProbMap database**

| Pathways | Genome1/ Org1/ Strain1 | Genome2/ Org2/ Strain2 | Genome3/ Org3/ Strain3 | Literature support for nutrient supplements, prebiotics, dietary components etc. to increase the production or degradation of specific metabolite |
|---|---|---|---|---|
| P1 (e.g., Butyrate biosynthesis) | 0 | 1 | 0 | Obtaining more resistant starch in the diet can increase commensal butyrate producers [Ref] |
| P2 | 1 | 0 | 1 | xxxx [Ref] |
| P3 | 1 | 1 | 0 | xxxx [Ref] |
| P4 | 0 | 1 | 1 | xxxx [Ref] |
| .... | ... | ... | ... | ...... |

The processor has software instructions to identify strains of bacteria which can be concocted to create a probiotic cocktail capable of augmenting beneficial metabolites and degrading deleterious metabolites depending on the metabolites/pathways analyzed and identified within the said samples. The probable pathways or perturbed pathways or probable metabolites obtained in the disclosure can be queried with the ProbMap knowledgebase. In an embodiment, if the probable pathways or perturbed pathways or metabolites include harmful metabolites or harmful pathways, the ProbMap is queried to find the row for the pathway for degradation of the said probable metabolites to identify strains where the value for this pathway is 1. In another embodiment, if the probable pathways or metabolites include reduction in benefical metabolites or beneficial pathways, the ProbMap is queried to find the row for the pathway for biosynthesis of the said probable metabolites to identify strains where the value for this pathway is 1. These strains can be considered as candidates for design of the personalized therapeutic. Similarly, the information in the said additional column corresponding to the said probable pathways and conditions can be utilized to design personalized dietary regimens also.

In another embodiment, if an individual lacks the microbes capable of production of a beneficial metabolite, the fecal matter obtained from another individual whose microbiome possesses the capabilities to make this metabolite can be administered using Fecal Microbial Transplant. Similarly fecal microbial transplant can be administered to an individual such that it can be used to replace organisms which cause biosynthesis of a deleterious metabolite. The donor individual here refers to the individual whose gut microbiome indicates the required composition to fulfil the desirable function in this scenario. The recipient is the individual whose gut sample and microbiome is being tested and indicates dysbiosis. In such cases, the microbiome of donor individual whose fecal matter is transplanted into the recipient should have either the bacteria that can degrade the deleterious metabolite or those which do not biosynthesize deleterious metabolite and can replace the harmful bacteria already present in the recipient individual. In one embodiment, the fecal sample obtained from the donor individual can be treated with a diluent and filtered using a filter medium, wherein the blended sample can be filtered through sieves of progressively smaller sizes. The filtrate so obtained can be centrifuged and frozen or freeze dried. This formulation created with the gut microbiome of the said healthy individual can be used for administration orally or by nasogastric tube or colonoscopy to the recipient. In one embodiment, the gut microbiome of the recipient individual can be flushed off/removed using methods like but not limited to administration of antibiotics. This helps the donor microbiome to colonize into recipient gut. In one embodiment, a combination of probiotics and nutrient supplements that favor beneficial bacteria's growth (prebiotics) can be administered in form of synbiotics or metabiotics or paraprobiotics to restore the favorable gut bacterial strains.

Thus, analysis of microbiome of an individual and identification of metabolites that may primarily affect the health condition can be used to personalize the probiotic formulations and regimens using the processor implemented steps on the pre-created knowledgebases stored in the memory. Further the processor is configured to analyze genetic data, blood reports and medical history of the patient in addition to factors like geography, lifestyle etc. to further pick strains of bacteria capable of biosynthesizing or degrading the said metabolites as well as be compatible with various factors and already present microbiome within a patient. The various health parameters and physiological markers of an individual might also be an indicator of the metabolites which can be removed or replenished within an individual using therapeutic and dietary regimens.

Probiotic supplements can be created and administered in several forms in the form of food products, dairy products, tablets depending on the requirements of the patient thereby, increasing patient compliance towards the therapy. Further the mode of administration and dosage can also be personalized depending on needs of a patient.

A follow up visit may be required in order to assess the efficacy of the probiotic. Additional tests to examine the gut microbiome and the corresponding metabolites may be performed and the formulation can be adjusted accordingly. An entirely new concoction or dietary pattern might be prescribed by the health care provider using the methodology described and the knowledgebase created after analyzing the results of the follow up tests. The knowledgebase contains information about a large number of bacterial strains and associations of their metabolic capabilities to degrade or biosynthesis various metabolites (beneficial or harmful). This large variety of strains helps to customize the probiotic formulation according to each individual and even designing multiple consortia for same individual. The factors that can be taken into account may include but are not limited to physiological and genotypic factors, geography, lifestyle, dietary patterns, lab reports and findings herewith etc.

As used herein, "reference values or range of values for healthy" mean a reference value or a range of values for abundance of a taxa established by analyses of composition of microbiota within populations determined to be in good health as obtained from publicly available microbiome profiles of healthy individuals or group of individuals or a population. The health condition can be diagnosed by methods known in the art for which may include but is not limited to clinical parameters, laboratory investigations, investigation by a certified clinical or health practitioner etc. The reference range values may also be established by governmental, academic, private or non-profit organizations that maintain these libraries of such microbiome profile or catalog the information about these reference values for taxa or metabolites as obtained from analyses of microbiome profiles.

As used herein, "reference values or range of values for dysbiosis" mean a reference value or a range of values for abundance of a taxa established by analyses of composition of microbiota within populations determined to have dysbiosis in gut or suffering from a microbiome related diseased condition. The disease condition can be diagnosed by methods known in the art for diagnosis of a disease condition which may include but is not limited to clinical parameters, laboratory investigations, investigation by a certified clinical or health practitioner etc. This information can also be obtained from publicly available microbiome profiles of individuals or group of individuals or a population with known dysbiosis in gut or manifestation of microbiome associated disease/ disorder. The reference range values for dysbiosis state or diseased/ disorder state may also be established by governmental, academic, private or non-profit organizations that maintain these libraries of such microbiome profile or catalog the information about these reference values for taxa or metabolites as obtained from analyses of microbiome profiles.

Thus, a reference value for healthy individuals would indicate atleast one of the following: relative abundance of taxonomic groups in the microbiome profile recognized as representative of subjects in good health, with absence of physiological symptoms of a diseased condition or absence of clinical/diagnostic parameters known in the art to be indicative of a disease. Similarly, reference value for diseased individuals would refer to relative abundance of taxonomic groups in the microbiome profile recognized as a representative of disease/ disease risk. It is further understood that higher than reference value would mean that relative abundance of a taxonomic group within a collected sample is higher than the reference value to an extent which can be associated with a diseased condition. The term lower than a reference value would mean that relative abundance of a taxonomic group within a collected sample is lower than the reference value to an extent which can be associated with a diseased condition.

At step 216 of the method (200), a taxa set is identified in the taxa abundance matrix obtained at the time stamp 2. The taxa set comprises of a TAXA_SET_P and a TAXA_SET_A.

The TAXA_SET_P comprises of taxa having a predefined first value for the one or more of the beneficial pathway in one of the FGmap and the GMP. In an embodiment, the predefined first value is 1 for one or more of beneficial pathways.

The TAXA_SET_A comprises of taxa having a predefined second value for the one or more of harmful pathways in one of the FGmap and the GMP. In an embodiment, the predefined second value is 1 for one or more of harmful pathways.

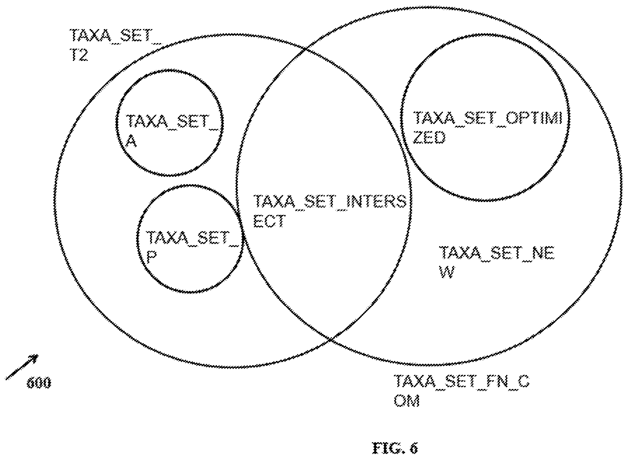

In an embodiment, the FIG.6 illustrates the taxa set comprises of a TAXA_SET_P and a TAXA_SET_A.

At step 218 of the method (200), a set of first neighbors is identified for each of the taxa in the TAXA_SET_P at the time stamp 2 from the TAXA_NET graph. The set of first neighbors are immediate neighbors of the TAXA_SET_P. The immediate neighbors comprising of a TAXA_SET_FN, a TAXA_SET_FN_COM, a TAXA_SET_INTERSECT, aa TAXA_SET_NEW.

In an embodiment, the FIG.6 illustrates the set of first neighbors in taxa set comprising of the TAXA_SET_P and the TAXA_SET_A. The set of first neighbors are immediate neighbors of the TAXA_SET_P. The immediate neighbors comprising of a TAXA_SET_FN, a TAXA_SET_FN_COM, a TAXA_SET_INTERSECT, aa TAXA_SET_NEW.

First neighbor refers to the set of taxa nodes in TAXA_NET which are directly connected by an edge to the set of nodes belonging to the TAXA_SET_P. The first neighbor of a query microbial node in a microbial association network essentially indicates the list of other microbial nodes which are in closest association in the microbial community and have a direct effect on the query node.

At step 220 of the method (200), recommending a personalized therapeutic intervention for improving gut health of the individual. The therapeutic intervention comprises recommending one of :

(a) a prebiotic cocktail,
(b) a probiotic cocktail,
(c) optimized diet .

The therapeutic intervention is recommend based on the identified first neighbors using the set of knowledge bases and the initial set of personalized therapeutics and diet recommendation, wherein the personalized therapeutic intervention comprises of at least one or more of:

(a) For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes,
(b) For Case B ( an increase in the pathway abundance of the set of harmful microbes) recommending an optimized diet, wherein the optimized diet promotes growth of the plurality of beneficial microbes and inhibits growth of harmful microbes, and
(c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

The recommending personalized therapeutic intervention comprises recommending one of the prebiotic cocktail, the probiotic cocktail and the optimized diet based on the critical taxa as described below:

Recommending personalized therapeutic intervention 1 : for a decrease in the pathway abundance of the plurality of beneficial microbes: The personalized therapeutic recommendation comprises:
(a) the prebiotic cocktail, (b) the probiotic cocktail and (c) the optimized diet

From among the microbial taxa available in sample belonging to T2, identify the ones primarily responsible for pyruvate utilization and save them in TAXA_SET_P. In one embodiment the taxa responsible for Pyruvate utilization can be obtained from GMP or FGmap whereby each taxa in microbial taxa abundance input of T2 is mapped to GMP and FGMap to see if the value corresponding to this taxa for Pyruvate utilization pathway is 1. Identify the first neighbors of TAXA_SET_P in TAXA_NET and save them in a new list TAXA_SET_FN. First neighbor refers to the set of nodes in TAXA_NET which are directly connected to the set of nodes belonging to the TAXA_SET_P. The first neighbor of a query microbial node in a microbial association network essentially indicates the list of other microbial nodes which are in closest association in the microbial community and have a direct effect on the query node. Predict the functional potential of the taxa in TAXA_SET_FN and identify the ones that are commensal which in one embodiment is based on their butyrate producing capability using Pyruvate utilizing pathway. In other embodiment pathways for biosynthesis of beneficial metabolites described in GMP, FGmap or literature can also be used. The said functional information can be obtained from the GMP and FGmap described in this invention whereby each taxa in TAXA_NET can be associated with a function based on genus-function or genome function mapping described in these matrices. Further function information can be added to each taxa on the basis of automated literature mining or by manual curation. Save the subset of these taxa from TAXA_SET_FN as TAXA_SET_FN_COM. Identify the set of taxa common between TAXA_SET_FN_COM and the set of all taxa present in T2. Save this list as TAXA_SET_INTERSECT. Save the remaining taxa in TAXA_SET_FN_COM which is not present in T2 as TAXA_SET_NEW.

The possible personalized therapeutic recommendation are suggested from below options

(a) Recommendation-1: Suggest administration of one or more prebiotic that can enhance the growth of TAXA_SET_INTERSECT and TAXA_SET_P

(b) Recommendation-2: Suggest one or more microbial strains belonging to the TAXA_SET_NEW as a probable candidate for a probiotic cocktail. Based on the fact that the collective synthesizing capability of microbes are responsible to shape their interaction profile, strains that are more probable to complete a metabolic pathway required for the community as evident from the predicted functional potential can be used as a measure to optimize a cocktail microbe constituent. Given the set of microbial strains belonging to the TAXA_SET_NEW as a suggested candidate, a subset of same can be obtained that help increase the collective synthesizing capability. For this purpose, methods like Netcooperate (https://doi.org/10.1186/s12859-015-0588-y) can be used to calculate the metabolic complementarity of a pair of microbes which can then be done for all possible unique pairwise combinations between the taxa identified in TAXA_SET_NEW (as first part of the pair) and TAXA_SET_INTERSECT (as second part of the pair). In the next step include the taxa identified in the pairs matching TAXA_SET_NEW as a final probiotic cocktail TAXA_SET_NEW_OPTIMIZED. The identification of TAXA_SET_NEW_OPTIMIZED is optional. In case the TAXA_SET_NEW_OPTIMIZED is identified, the TAXA_SET_NEW is replaced as the TAXA_SET_NEW_OPTIMIZED and all references to TAXA_SET_NEW is same as TAXA_SET_NEW_OPTIMIZED.

(c) Recommendation-3: In addition to recommendation 2, suggest administration of one or more prebiotic that can enhance the growth of TAXA_SET_INTERSECT, TAXA_SET_P and TAXA_SET_NEW.

(d) Recommendation-4: Obtain the list of food constituents used by the set of taxa in TAXA_SET_INTERSECT and TAXA_SET_P using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Use the OPTIMIZED_FOOD_SET for recommending a personalized diet for the subject identified to have a deteriorated gut health at the time point T2.

(e) Recommendation-5: Combine Recommendation-2 and Recommendation-4 to suggest administration of probiotic as well as the food required to maintain stability of the gut microbial community structure.

(f) Recommendation-6: Use Recommendation-2 followed by the suggesting a diet to maintain the existing community as well as the newly added probiotics. Obtain the list of food constituents used by the set of taxa in TAXA_SET_INTERSECT, TAXA_SET_NEW and TAXA_SET_P using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Use the OPTIMIZED_FOOD_SET for recommending a personalized diet for the subject identified to have a deteriorated gut health at the time point T2.

Recommending personalized therapeutic intervention 2 - for an increase in the pathway abundance of plurality of harmful microbes: The personalized therapeutic recommendation comprises optimized diet

From among the microbial taxa available in sample belonging to T2, identify the ones primarily responsible for ammonia production and save them in TAXA_SET_A. Identify the set of commensal bacteria in sample belonging to T2 and save them in a list T2_COM which in one embodiment can be done by evaluating the pyruvate utilizing potential of the constituent microbes.

The possible personalized therapeutic recommendation are suggested from below options :

(a) Recommendation-1: Obtain the list of food constituents used by the set of taxa in TAXA_SET_A using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Suggest the subject identified to have a deteriorated gut health at the time point T2 to reduce or stop intake of food as obtained in FOOD_SET.

(b) Recommendation-2: In addition to Recommendation-1, obtain the list of food constituents used by the set of taxa in T2_COM using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Use the OPTIMIZED_FOOD_SET for recommending a personalized diet for the subject identified to have a deteriorated gut health at the time point T2.

Recommending personalized therapeutic intervention 3 - For a decrease in the pathway abundance of the plurality of beneficial microbes and increase in the pathway abundance of the plurality of harmful microbes.

The personalized therapeutic recommendation comprises optimized diet:

(a) the prebiotic cocktail, (b) the probiotic cocktail and (c) the optimized diet

From among the microbial taxa available in sample belonging to T2, identify the ones primarily responsible for pyruvate utilization and save them in TAXA_SET_P. In the next step, from among the microbial taxa available in sample belonging to T2, identify the ones primarily responsible for ammonia production and save them in TAXA_SET_A. Identify the first neighbors of TAXA_SET_P in TAXA_NET and save them in a new list TAXA_SET_FN. Predict the functional potential of the taxa in TAXA_SET_FN and identify the ones that are commensal which in one embodiment is based on their butyrate producing capability. Save the subset of these taxa from TAXA_SET_FN as TAXA_SET_FN_COM. Identify the set of taxa common between TAXA_SET_FN_COM and the set of all taxa present in T2. Save this list as TAXA_SET_INTERSECT. Save the remaining taxa in TAXA_SET_FN_COM which is not present in T2 as TAXA_SET_NEW. Identify the set of commensal bacteria in sample belonging to T2 and save them in a list T2_COM which in one embodiment can be done by evaluating the pyruvate utilizing potential of the constituent microbes.

The possible personalized therapeutic recommendation are suggested from below options:

(a) Recommendation-1: Suggest administration of one or more prebiotic that can enhance the growth of TAXA_SET_INTERSECT and TAXA_SET_P.

(b) Recommendation-2: Suggest one or more microbial strains belonging to the TAXA_SET_NEW as a probable candidate for a probiotic cocktail. The final probiotic cocktail can then be decided by identifying the strains that are more probable to complete a metabolic pathway required for the individual microbes as well as the community. Multiple combinations of such cocktail designs can be obtained. A co-culture of the microbial cocktail in a laboratory mimicking the conditions of human gut with required nutrition sources can be used to finalize a combination.

(c) Recommendation-3: In addition to recommendation 2, suggest administration of one or more prebiotic that can enhance the growth of TAXA_SET_INTERSECT, TAXA_SET_P and TAXA_SET_NEW.

(d) Recommendation-4: Obtain the list of food constituents used by the set of taxa in TAXA_SET_INTERSECT and TAXA_SET_P using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Use the OPTIMIZED_FOOD_SET for recommending a personalized diet for the subject identified to have a deteriorated gut health at the time point T2.

(e) Recommendation-5: Combine Recommendation-2 and Recommendation-4 to suggest administration of probiotic as well as the food required to maintain stability of the gut microbial community structure.

(f) Recommendation-6: Use Recommendation-2 followed by the suggesting a diet to maintain the existing community as well as the newly added probiotics. Obtain the list of food constituents used by the set of taxa in TAXA_SET_INTERSECT, TAXA_SET_NEW and TAXA_SET_P using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Use the OPTIMIZED_FOOD_SET for recommending a personalized diet for the subject identified to have a deteriorated gut health at the time point T2.

(g) Recommendation-7: Use Recommendation-2 followed by the suggesting a diet to maintain the existing community as well as the newly added probiotics. Obtain the list of food constituents used by the set of taxa in TAXA_SET_INTERSECT, TAXA_SET_NEW and TAXA_SET_P using the TAXA_CONSTITUENT_NET and save the list in CONSTITUENT_SET. Find the set of food (FOOD_SET) that harbor the identified constituents in CONSTITUENT_SET using the FOOD_CONSTITUENT_NET. Optimize the food FOOD_SET based on personalized preferences and dietary requirements and save it as OPTIMIZED_FOOD_SET. Similarly, obtain the list of food constituents used by the set of taxa in TAXA_SET_A using the TAXA_CONSTITUENT_NET and save the list in CONSTITUENT_SET. Find the set of food (FOOD_SET_A) that harbor the identified constituents in CONSTITUENT_SET using the FOOD_CONSTITUENT_NET. Suggest the subject identified to have a deteriorated gut health at the time point T2 to reduce or stop intake of food as obtained in FOOD _SET _A.

(h) Recommendation-8: Use Recommendation-4 followed by obtaining a list of food constituents used by the set of taxa in TAXA_SET_A using the TAXA_CONSTITUENT_NET and save the list in CONSTITUENT_SET. Find the set of food (FOOD_SET_A) that harbor the identified constituents in CONSTITUENT_SET using the FOOD_CONSTITUENT_NET. Suggest the subject identified to have a deteriorated gut health at the time point T2 to reduce or stop intake of food as obtained in FOOD_SET_A.

The optimized diet is recommended based on the optimization technique, wherein the optimization technique includes optimizing a gut food score to obtain the optimized diet., The optimization is subject to a plurality of constraint parameters comprising a calories parameter, a carbohydrate parameter, a protein parameter and a fat parameter. In an embodiment, the optimization of different food items is done such that the food items with a high Gut Food Score is recommended and the items with low Gut Food Score is avoided. The Gut Food Score (GFS) is a quantified personalized score. wherein a food item specifically increases the abundance of commensal or useful bacterial taxa and does not cause a significant change in the abundance of harmful or pathogenic or potentially pathogenic bacterial taxa in an individual, given the change in microbiome composition among two samples. The Gut Food Score of a particular food item is in turn calculated using the properties of its constituent compounds.

Compounds present in the food can be utilized by bacterial taxa as energy source, leading to the growth of the utilizing taxa, consequently increasing its abundance. If the increased taxa are commensal in nature, the gut health improves, and if the increased taxa are pathogenic in nature, the gut health deteriorates. Therefore, having certain foods which contain compounds utilized specifically by commensal bacterial taxa is beneficial for gut health. Conversely, refraining from foods which can be utilized by pathogenic bacteria is also beneficial for health. Therefore, if a person / patient has undergone an increase in abundance of a set of pathogenic taxa, then foods containing compounds which can be utilized by the same set of pathogenic taxa should be especially avoided, as consuming those food items can lead to further increase in the abundance of the pathogenic taxa and consequent health issues. Conversely, if certain types of commensal bacteria have shown a decrease in abundance, then foods containing compounds which can be utilized by the commensal bacteria should be specifically eaten, in order to restore gut health. To quantify or score a food based on the utilization profile of its constituent compounds by commensal and pathogenic bacteria, as well as the increase in pathogenic taxa and decrease in commensal taxa, we calculate the Gut Food Score (GFS). The GFS score is personalized, i.e. every person / patient will have a different score based on the microbiome profile change between the healthy and diseased state. Foods having high GFS score must be consumed and foods having low (-ve) GFS score must be avoided.

A first requirement of the disclosed steps would be a list of compounds that occur in different food items. Let the list of such compounds be denoted by the following:

**compunds** = [$compound_1$, $compound_2$, $compound_3$, ... , $compound_p$ ], where p is the total number of such compounds.

In an embodiment, the said list of compounds might be obtained from different published food-constituent databases or tables.

A second requirement is a list of foods:

$$\mathbf{food} = [food_1, food_2, food_3, ..., food_m],$$

where m is the total number of such food items.

Such a list of food items might be obtained from different published food-constituent databases or tables. The constituents' compounds of these food items and their contents must be known. In an embodiment, these constituent compounds in a food item can be obtained by databases available in public repositories. In another embodiment, the constituent compounds of a food item can be obtained by literature mining techniques and manual curation.

A third requirement is the Food Composition matrix (C) contains the contents of compounds present in different foods items,

$$\mathbf{C} \in \mathbb{R}^{p \times m},$$

where

p = total number of compounds obtained in foods from list foods and

m = total number of foods.

In an embodiment, the Food Composition matrix (which is similar to the matrix form of the FOOD_CONSTITUENT_NET) is created from literature or from several published food-constituent databases or tables.
The matrix C can be obtained from different published food-constituent databases or literature and is defined in table 8 below:

**Table 8 : matrix c**

|  | food$_1$ | food$_2$ | ..... | food$_m$ |
|---|---|---|---|---|
| compoundi | $C_{1,1}$ | $C_{1,2}$ | .... | $C_{1,m}$ |
| compound$_2$ | $C_{2,1}$ | $C_{2,2}$ | ... | $C_{2,m}$ |
| .... | ... | ... | ... | ... |
| compound$_p$ | $C_{p,1}$ | $C_{p,2}$ | ... | $C_{p,m}$ |

where $\mathbf{C}_{i,j}$ is the content of compound $i$ per unit of Food item $j$.

A fourth requirement is a plurality of vectors using data available in publicly available databases and scientific literature:

$\mathbf{carb} \in \mathbb{R}^m$ ; **carb** = [$carb_1$, $carb_2$, $carb_3$, ..., $carb_m$] is the vector containing the carbohydrate content of foods such that $carb_i$ = carbohydrate content per unit of $food_i$ - which is obtained from existing literature.

**protein** = [$protein_1$, $protein_2$, $protein_3$, ..., $protein_m$] is the vector containing the protein content of foods such that $protein_i$ = protein content per unit of $food_i$ . This can be obtained from existing literature.

$\mathbf{fat} \in \mathbb{R}^m$ ; **fat** = [$fat_1$, $fat_2$, $fat_3$, ... , $fat_m$] is the vector containing the fat content of foods such that $fat_i$ = fat content per unit of $food_i$. This can be obtained from published literature.

$\mathbf{calorie} \in \mathbb{R}^m$ ; **calorie** = [$calorie_1$, $calorie_2$, $calorie_3$, ... , $calorie_m$] is the vector containing the $calorie$ content of foods such that $calorie_i$= $calorie$ content per unit of $food_i$. This can be obtained from published literature.

A fifth requirement is having a list of taxa. Let that list of taxa be the following:

$$\mathbf{taxa} = \left[ taxa_1, taxa_2, taxa_3, ..., taxa_q \right]$$

where q is the total number of taxa.

In an embodiment, the list of taxa can be only those taxa which have either a beneficial or harmful metabolite pathway, as defined previously in the disclosure.

A fifth requirement are two functions which can examine the genetic functional potential of different bacterial taxa and determine whether they are commensal (also referred as beneficial) or pathogenic (also referred as harmful).

$$commensal(taxa_i) = \begin{cases} True\ if\ taxa_i\ is\ \text{commensal} \\ False\ otherwise \end{cases}$$

$$pathogen(taxa_i) = \begin{cases} True\ if\ taxa_i\ is\ pathogenic \\ False\ otherwise \end{cases}$$

In an embodiment, the function can identify the commensal taxa as those taxa which have the functional ability or genes/enzymes/proteins corresponding to beneficial pathways described in this disclosure, and the pathogenic bacteria as those taxa with the functional ability or genes/enzymes/proteins corresponding to harmful pathways described in this disclosure. In such an embodiment, the commensal(x) would be a function which essentially examines the genes present in the taxa x and return 'True' if the genes encode proteins corresponding to beneficial pathways described in this disclosure. On the other hand, the pathogen(x) would be a function which essentially examine the genes present in the taxa x and return 'True' if the genes encode proteins corresponding to harmful pathways described in this disclosure. PU is defined as the set containing the taxon belonging to the list **taxa** which are commensal.

$$PU = \{taxa_i \; \forall \; taxa_i \; \epsilon \; \textbf{taxa}: commensal(taxa_i) = True\}$$

AP is defined as the set containing the taxa belonging to the list taxa which are pathogenic.

$$AP = \{taxa_i \; \forall \; taxa_i \; \epsilon \; \textbf{taxa}: pathogenic(taxa_i) = True\}$$

In one embodiment, all the bacterial taxa that are positively associated first neighbours (with a direct edge) with the elements / bacterial taxa of the set PU in TAXA_NET can also be added to the set PU. Additionally, all the bacterial taxa that are positively associated with the elements of the set AP in TAXA_NET can also be added to the set AP. In an embodiment PU can be the TAXA_SET_P and AP can be the TAXA_SET_A as disclosed earlier.

A sixth requirement is the Taxa-Compound Utilization matrix (which is essentially identical to the matrix form of TAXA_CONSTITUENT_NET), which is a matrix containing the compounds that can be used as the energy source by different bacterial taxa, i.e.

$$T \; \epsilon \; \{0, 1\}^{p \times q}; \; T_{i,j} :$$
$$= \left\{ \begin{array}{l} 1 \; if \; taxa_i \; can \; utilize \; compound_j \; as \; an \; energy \; source \\ \qquad\qquad\qquad 0 \; otherwise \end{array} \right\}$$

where

> p = total number of compounds and
> q = total number of **taxa.**

In one embodiment, such a matrix can be obtained using the functional potential estimation based on the different genes present in the bacterial taxa, wherein functional potential refers to the set of metabolic pathways encoded within a taxa which can help utilize a set of food constituents. In another embodiment, this table can also be obtained from publicly available repositories like Virtual Metabolic Human.

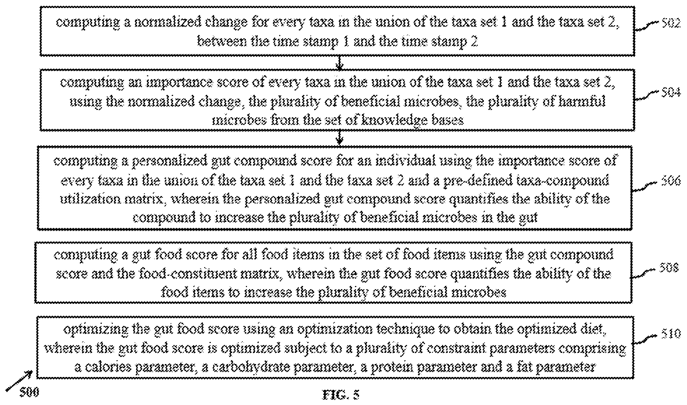

The process of recommending the optimized diet is explained in conjunction with flow diagram of FIG.5 below:

At step 502 of the method (500), a normalised change is computed for every taxa in the union of the taxa set 1 and the taxa set 2, between the time stamp 1 (or time point 1) and the time stamp 2 (or time point 2).

In an embodiment, the gut health deterioration refers to either a reduction in the combination of beneficial metabolic pathways and their constituent metabolites or an increase in harmful metabolic pathways and their constituent metabolites in gut microbiome. This may refer to the dysbiotic state of an individual between samples at timestamp T1 and T2. The first time point (T1) can be a state before a perturbation and second time point (T2) can be a state post perturbation wherein the perturbation has resulted in dysbiosis of the gut microbial community structure. Identify the subject whose gut health is seen to be deteriorated at the time point T2 with respect to T1. From the input microbial taxa abundance file of the subject's microbiome corresponding to T2 with respect to T1, identify the reason of dysbiosis with respect to the functional potential.

In an embodiment, the normalised change represented as a vector **d** is computed for every taxa denoted by taxa. $\textbf{d} \; \epsilon \; \mathbb{R}^q$ (q being the total number of taxa which we are interested in) is computed such that, for every taxa denoted by $taxa_i$, $d_i$ captures the change in the abundance of $taxa_i$ between T2 and T1 $taxa_i$[OBJ] in gut microbiome samples as shown below :

$$\textbf{d} \; \epsilon \; \mathbb{R}^q; \; \forall taxa_i \; \epsilon \; \textbf{taxa}, \; \textbf{d}_i := \frac{\left[A_i^2 - A_i^1\right]}{\sigma_i}$$

where,

$A_i^2$ is the abundance of $taxa_i$ at in the sample T2 ,

$A_i^1$ is the abundance of $taxa_i$ in T1,

$\sigma_i$ is the standard deviation of the abundance of $taxa_i$ in different gut microbiome samples of healthy humans.

In one embodiment, the gut microbiome samples of humans can be obtained from different publicly available datasets containing microbial abundance of several healthy human beings.

At step 504 of the method (500), an taxa importance score is computed for every taxa in the union of the taxa set 1 and the taxa set 2. The taxa importance score is computed using the normalised change, the plurality of beneficial microbes, the plurality of harmful microbes from the set of knowledge bases.

The taxa importance vector $\mathbf{b} \in \mathbb{R}^q$ such that for every taxa denoted by $taxa_i$:

$$\mathbf{b} \in \mathbb{R}^q; \ \forall taxa_i \in \mathbf{taxa}, \mathbf{b}_i :$$
$$= \max\big((1 + d_i \times \mathrm{I}[taxa_i \in AP] - d_i \times \mathrm{I}[taxa_i \in PU]), 1\big)$$

where,

$b_i$ is the taxa importance score of $taxa_i$

$d_i$ is the taxa normalised change score of $taxa_i$

I is the indicator function, i.e.

$$\mathrm{I}[y \in Z] := \begin{Bmatrix} 1 \ if \ y \in Z \\ 0 \ otherwise \end{Bmatrix}$$

i.e for any set Z and any variable y which may or may not lie in the set Z,

PU : set of commensal bacterial taxa

AP: set of pathogenic bacterial taxa

By the definition of the vector **b**, if a pathogenic taxon increases by a large amount (compared to its healthy state abundance and standard deviation) in T2, it gets a high score, but if the increase is low (compared to its healthy state abundance and standard deviation), it gets a lower score. If a pathogenic taxon does not increase at all or even decreases, it gets a score of 1. On the other hand, if a commensal bacteria taxon decreases by a large amount (compared to its healthy state abundance and standard deviation) in T2, it gets a higher importance score, otherwise 1. If the taxa belong to both the commensal and the pathogenic sets or none of them, it gets a score of 1.

At step 506 of the method (500), a personalized gut compound score is computed for an individual using the importance score of every taxa in the union of the taxa set 1 and the taxa set 2 and a pre-defined taxa-compound utilization matrix (T). The personalized gut compound score quantifies the ability of the compound to increase the plurality of beneficial microbes in the gut.

In an embodiment, the GCS score is computed for all p compounds present in the list compounds, as shown below:

$$\mathbf{gcs} \in \mathbb{R}^p; \ \mathbf{gcs} = [gcs_1, gcs_2, gcs_3, \ldots, gcs_p]$$

is the vector containing the Gut Compound Scores of different compounds, where
$\forall$ compound$_i \in$ **compounds**, the Gut Compound Score is defined as

$$gcs_i = \sum_{j=1}^{q} \mathrm{T}_{i,j} \times \mathrm{b}_j \times \mathrm{I}[taxa_j \in PU] - \sum_{j=1}^{q} \mathrm{T}_{i,j} \times \mathrm{b}_j \times \mathrm{I}[taxa_j \in AP]$$

where,

I is the indicator function.

The Gut Compound Score of a specific compound gives the importance of the different compounds in the optimization process. The first component of the equation quantifies the ability of the compound to increase commensal gut microbial taxa. If the compound is utilized by a commensal taxon which decreased significantly in T2, it gets a higher score in the first component of the equation. If the compound is utilized by any other commensal which does not decrease much in T2, it gets a score of lower score, lower bounded by 1. If the compound cannot be utilized by commensals, it gets a score of 0 in the first component.

Conversely, the second component of the equation quantifies the ability of the compound to decrease pathogenic bacterial taxa. If the compound is utilized by a pathogenic taxon which increases by a large amount in T2, it gets a high score in the second component of the equation. If it is utilized by any other pathogenic bacteria, it gets a lower score, bounded below by 1. If the compound is not utilized by pathogens, it gets a score of 0 in the second component.

Summing up the above two points, the Gut Compound Score of a specific compound quantifies that the compound will be utilized as an energy source by commensal bacterial taxa (with a higher score if it can be utilized by commensal taxa which are decreased in T2) but not the pathogenic taxa (with a lower score if it can be utilized by a pathogenic bacterial taxon which increased in T2). In other words, compounds with a high (positive) GCS can selectively increase the abundance of commensal bacteria, but not the pathogenic bacteria. Conversely, compounds with low (negative) GCS score can selectively increase pathogenic bacteria and not commensal bacteria. GCS is the vector containing the Gut Compound Score for every compound in the list compounds.

At step 508 of the method (500), a Gut Food Score (GFS) is computed for all food items in the set of food items using the Gut Compound Score and the food-constituent matrix. GFS quantifies the ability of the food items to increase the plurality of beneficial microbes.

In an embodiment, the GFS for a specific food item is the weighted sum of the Gut Compound Score of the constituent compounds of the food item, weighted by their concentration, that is, the amount of the compound per unit of the food item. The gfs is the vector containing the Gut Food Score for every food in the list **food**.

$$\mathbf{gfs} \in \mathbb{R}^m; \; \mathbf{gfs} = [gfs_1, gfs_2, gfs_3, \ldots, gfs_m]$$

is the vector containing the Gut Food Scores of the 'm' food items, where

$\forall \text{food}_i \in$ **food**, the Gut Food Score is defined as:

$$gfs_i = \sum_{j=1}^{p} C_{j,i} \times gcs_j$$

In order to calculate the GFS for a particular food item, the individual constituent compounds' GCS scores is multiplied by their content in the food, and then the resultant scores are summed up. Interpretation for GFS: Food with higher GFS be utilized by commensal bacterial taxa, which consequently leads to a proliferation in the commensal taxa abundances, with a higher score specifying that it can potentially be utilized by commensals which have decreased in the diseased state and can potentially improve gut health. This personalized Gut Food Score can help in finding the optimal diet.

At step 510 of the method (500), the diet is optimized such that Gut Food Score of all the optimized food items is maximized. The optimization is subject to a plurality of constraint parameters comprising a calories parameter, a carbohydrate parameter, a protein parameter and a fat parameter

In an embodiment, the optimized diet to be utilized specifically by the commensal genera and not by the pathogenic genera, as well as fulfilling the daily caloric and macronutrient requirements, is computed based below optimization problem based on the gut food score:

$$\mathbf{x}^* = \arg\max_x \left( \sum_{i=1}^{m} gfs_i \times x_i \times s_i \right)$$

subject to the following constraints

$$Calories_{min} \leq \sum_{i=1}^{m} calories_i \times x_i \times s_i \leq Calories_{max}$$

$$Carbs_{min} \leq \sum_{i=1}^{m} carbs_i \times x_i \times s_i \leq Carbs_{max}$$

$$Protein_{min} \leq \sum_{i=1}^{m} protein_i \times x_i \times s_i \leq Protein_{max}$$

$$Fat_{min} \leq \sum_{i=1}^{m} fat_i \times x_i \times s_i \leq Fat_{max}$$

$$\forall x_i \in \mathbf{x}, x_i \geq 0$$

where

the user has an option to choose from a list of m food items.

$$\mathbf{food} \in \mathbb{R}^m; \mathbf{food} = [food_1, food_2, food_3, \dots, food_m]$$

is the a list of all the 'm' food items,

$\mathbf{carb} \in \mathbb{R}^m$ ; **carb** = [$carb_1$, $carb_2$, $carb_3$, ..., $carb_m$] is the vector containing the carbohydrate content of foods such that $carb_i$ = carbohydrate content per unit of $food_i$ - which is obtained from existing literature.
protein = [$protein_1$, $protein_2$, $protein_3$, ..., $protein_m$] is the vector containing the protein content of foods such that $protein_i$ = protein content per unit of $food_i$ . This can be obtained from existing literature.

$\mathbf{fat} \in \mathbb{R}^m$ ; **fat** = [$fat_1$, $fat_2$, $fat_3$, ... , $fat_m$] is the vector containing the fat content of foods such that $fat_i$ = fat content per unit of $food_i$. This can be obtained from published literature.

$\mathbf{calorie} \in \mathbb{R}^m$ ; **calorie** = [$calorie_1$, $calorie_2$, $calorie_3$, ..., $calorie_m$] is the vector containing the *calorie* content of foods such that $calorie_i$ = *calorie* content per unit of *f ood$_i$*. This can be obtained from published literature.

$\mathbf{s} \in \mathbb{R}^m$ ; **s** = [$s_1$, $s_2$, $s_3$, ... , $s_m$] is a vector such that $s_i \in \{0, 1\} \forall i \in [1, 2, 3, ..., m]$ and

$$s_i := \begin{Bmatrix} 1 \; if \; user \; selects \; food_i \\ 0 \; otherwise \end{Bmatrix}$$

The users can select several of the food items from the list food based on their preferences. In one embodiment these preferences may be based on dietary preferences (including taste, allergy to certain food, religious preference, etc.), food availability in geographical location etc. This does not necessarily exclude other factors which may influence the preference for specific foods. This user food selection can be aided by a computational system for example website, mobile application etc.

$$\mathbf{x} \in \mathbb{R}^m; \mathbf{x}^* = [x_1, x_2, x_3, \dots, x_m]$$

is a vector containing the quantity of foods such that $x_i$ is the quantity of $food_i$.

$$\mathbf{x}^* \in \mathbb{R}^m; \mathbf{x}^* = [x_1^*, x_2^*, x_3^*, \dots, x_{m'}^*]$$

is a vector containing the optimized quantity of foods produced by solving the constrained optimization problem.

$\mathbf{gfs} \in \mathbb{R}^m$ ; **gfs** = [$gfs_1$, $gfs_2$, $gfs_3$, ... , $gfs_m$] is a vector containing the personalized gut food score given to the m food items, such that $gfs_i$ quantifies the Gut Food Score of $food_i$ for a specific person.
$Carbs_{min}$, $Carbs_{max}$, $Protein_{min}$, $Protein_{max}$, $Fat_{min}$, $Fat_{max}$, $Calories_{min}$, $Calories_{max}$ are the minimum and maximum bounds for the carbs, proteins, fats and total calorie intake of the user, and can be either provided by the users (assuming they have a fair idea about what constitutes a good diet) or can be selected from several preset standard values based on the end-users characteristics including but not limited to age, height and weight. The selection of these values can also be done by professionals for the end-users based on their experience. This selection can also be aided partially or in whole by a computational system for example : website, mobile application etc.
In an embodiment, the constrained optimization problem can be solved using any optimization technique including but not limited to linear programming, using solving using manual or computational tools.

Recommended food amounts = **r** = **x**\* ⊗ **s** ; *i.e.* $r_i = x_i^* \times s_i \ \forall \ i \ \epsilon \ [1, 2, 3, \dots, m]$ where ⊗ denotes the element wise product among the vectors.

The vector **r** is the final recommended quantity of foods, such that $r_i$ is the recommended quantity of food$_i$. It will have quantity 0 for all foods not selected by the user, and for the ones selected by the user, it will have 0 or +ve values depending on the results of the optimization step.

In another embodiment, the pathways for production of one or more beneficial metabolites, or/and the pathways for degradation/elimination of one or more harmful metabolites can be genetically engineered or bioengineered into the genetic makeup of gut microorganisms. These genetically engineered microorganisms can be administered as probiotics either alone or in combination with prebiotics/ metabiotics/ paraprobiotics to individuals having intestinal disorders.

In one embodiment the present disclosure provides a modified or an genetically engineered microorganism, wherein the organism expresses heterologously one or more of pathways required to produce metabolites. A "modified" microorganism refers to a microorganism capable of surviving in gut as a commensal or a symbiont whose naturally occurring form has been changed, such as having been genetically engineered to express a metabolic pathway heterologously. A "heterologous" protein or a pathway refers to a gene or a set of genes forming a pathway which are not normally encoded by the genome of the microorganism. Accordingly, heterologous production of proteins forming a pathway involves introducing a DNA sequence encoding genes/set of genes corresponding to a particular pathway into the microorganism along with a promoter and terminator sequence capable of operating in the microorganism. The proteins forming a pathway can be expressed using a suitable expression vector or other construct introduced into the microorganism. In embodiments the heterologous pathway is introduced into the modified organism encoded by a plasmid or on DNA segment which is introduced into the bacterial chromosome.

In embodiments, the disclosure includes modified bacteria comprising one or more of commensals or symbionts known in the art. In one embodiment the modified bacteria might include species of genera like Lactobacillus and Bifidobacterium capable of residing in the gut. Many members of these genera are active in food products intended for human and animal consumption including dairy products, such as yogurt, milk, milk-based creams, ice cream products, and cheese, or combinations of the foregoing. In certain embodiments the modified bacteria can be one or more of Lactobacillus *plantarum, Lactobacillus casei, Lactobacillus acidophilus, Lactobacillus salivarius,* or *L. Reuteri* as well as probiotic strains of *Bifidobacterium* such as *B. longum.* In another embodiment, the modified organism may include facultative anaerobes capable of residing the gut. In an embodiment, commercial probiotic strains including *Clostridium butyricum* and *Streptococcus thermophilus* can also be used as modified organisms. Any other commensals/symbionts in gut are within the scope for being engineered to express desired pathways.

In another aspect the disclosure comprises a food product comprising a modified bacteria that expresses the heterologous pathways can be synthesized. In another aspect the food product may comprise of personalized probiotic strains identified in this invention which possess the pathways required for improvement in gut health of an individual. In embodiments the food product is a dairy product, including but not necessarily limited to yogurt, milk, milk-based creams, and cheese or chocolates. The manufacture of food products containing live cultures of microorganisms, such as yogurts, are well known in the art. These can be adapted to include presently provided modified microorganisms or microbial consortia identified in this invention. The packaging of the food can provide information to the consumer that identifies the presence of modified or other bacterial strains in the food product.

In another aspect the disclosure includes a supplement product, such as a nutraceutical product, a dietary supplement, a food ingredient, etc., including but not limited to a probiotic formulation or functional food that contains one or more of the live bacteria, modified or naturally occuring as described in this disclosure. The supplement product can be available as a liquid, capsules, tablets, softgels, powders, and the like.

In another aspect the disclosure provides a pharmaceutical composition comprising the said modified microorganisms or the composition of probiotic formulations identified in this disclosure. The pharmaceutical composition can include any suitable diluent, carrier, excipient, buffer, etc., intended for use with the microorganisms present in the formulation. The administration of pharmaceutical formulation can be performed using one or more of suitable routes, including but not limited to parenteral, intraperitoneal, intrapulmonary, oral, intra-abdominal etc. Parenteral infusions may include one or more of intramuscular, intravenous, intraarterial, intraperitoneal, and subcutaneous administration.

The personalized therapeutic intervention further comprises administering a genetically engineered bacteria , wherein the genetically engineered bacteria comprise of genetically engineering (a) a plurality of microbial pathways for production of one or more beneficial metabolites, or/and (b) the plurality of microbial pathways for degradation/elimination of one or more harmful metabolites into the genetic makeup of gut microorganisms, wherein the genetically engineered bacteria is administered in one of the ways shared below:

(a) genetically engineered as probiotics either alone or in combination with one of a prebiotics, a metabiotics, and a paraprobiotics,

(b) genetically engineered organism expressing heterologous one or more of pathways required to produce metabolites, wherein the genetically engineered organism can be one or more of a gut commensals wherein the gut commensals may include but not limited to beneficial microbes, commercial probiotic strains or facultative anaerobes capable of residing the gut; wherein the genetically engineered organism is also referred to as modified bacteria, wherein the modified bacteria can be administered as a food product, food supplement or a pharmaceutical composition or a probiotic formulation.

The written description describes the subject matter herein to enable any person skilled in the art to make and use the embodiments. The scope of the subject matter embodiments is defined by the claims.

The embodiments of present disclosure herein provide a solution to address a problem of designing a personalized therapeutic intervention for an individual based on functions of microbiome. Each individual has a unique composition of microbiome in the gut, one probiotic or dietary regimen may not have same efficacy in different individuals. The disclosure recommends a personalized therapeutic intervention for improving gut health of an individual by designing personalized therapeutics and diet based on functions of microbiome. The personalized therapeutic intervention is recommended based on several steps including generating a set of knowledge bases, identifying a change in the gut health of an individual by monitoring the gut samples and recommending a personalized therapeutic intervention. The personalized therapeutic intervention comprises atleast one of a prebiotic, a probiotic and an optimized diet, wherein the optimized diet is estimated based on optimizing a gut food score, where the gut food score is computed based on the change in the gut health of an individual.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200) for designing personalized therapeutics and diet based on functions of microbiome comprising:

   receiving a set of input data (202), via one or more hardware processors, wherein the set of input data comprises data associated with
   a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts and a plurality of diet optimization pre-requisite parameters,

   wherein the plurality of metabolites comprises a set of beneficial metabolites and a set of harmful metabolites, wherein the set of beneficial metabolites is beneficial for gut health of an individual and the set of harmful metabolites is harmful for the gut health of the individual,
   wherein the plurality of metabolic pathways comprises one of (a) a beneficial pathway (b) harmful pathways,

   wherein the beneficial pathways comprise one or more of a set of metabolic pathways for biosynthesis of plurality of beneficial metabolites and a set of metabolic pathways for degradation of the plurality of harmful metabolites, and
   wherein the harmful pathways comprise one or more of a set of metabolic pathways for degradation of plurality of beneficial metabolites and a set of metabolic pathways for biosynthesis of the plurality harmful metabolites;
   wherein the plurality of taxonomic groups comprise a plurality of microbes, where the plurality of microbes comprises a plurality of beneficial microbes and a plurality of harmful microbes, where the plurality of microbes are classified according to a taxonomic hierarchy, wherein the taxonomic hierarchy comprises one or more of a plurality of bacterial phyla, a plurality of bacterial classes, a plurality of bacterial orders, a plurality of bacterial families, a plurality of bacterial genera, a plurality of bacterial species and a plurality of bacterial strains, where the plurality of microbes comprises of a plurality of genes having distinct genomic location, a set of nucleotide and protein sequences of the bacterial strains,
   wherein the plurality of beneficial microbes harbours the beneficial pathways and the plurality of harmful microbes harbours the harmful pathways; and
   wherein the plurality of diet optimization pre-requisite parameters comprises a set of food items, a complete set of compounds that are found in the set of food items, a food-constituent matrix, wherein the food-constituent matrix tabulates the content of every compound belonging to the set compounds in every food item belonging to the set foods, the total carbohydrate, protein, fats and caloric content of the each of the food items from the set of food items, a list of bacterial taxa in the human gut, a set of functions to identify a set of beneficial bacteria taxa and a set of harmful bacterial taxa, a taxa-compound utilization matrix, a user defined set of upper bound parameters-lower bound parameters associated with carbohydrate, protein, fat and calorie, and a user selected food items;

   generating a set of knowledge bases using the set of input data (204), via the one or more hardware processors, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap wherein the generation of the set of knowledge bases comprises:

   generating the FGmap and the GMP map, wherein the FGmap is a matrix associated with a function of the plurality of bacterial genera belonging to one of the plurality of taxonomic group and the plurality of metabolic pathways as columns, wherein a first pre-defined indicator indicates one of (a) a presence of the metabolic pathway and (b) an absence of the metabolic pathway and the GMP is a matrix of the plurality of bacterial strains as rows and the plurality of metabolic pathways as columns, wherein a second pre-defined indicator indicates one of (a) a presence of the metabolic pathway and (b) an absence of the metabolic pathway (204A);
   generating the TAXA_NET graph based on the plurality of taxonomic groups and the FGmap, wherein the TAXA_NET graph is a undirected graph, wherein each node in the TAXA_NET graph corresponds to a taxonomic group from the plurality of taxonomic groups and edges indicate a positive relationship between a pair of the taxonomic group wherein each taxa from a plurality of nodes is associated with the function of the plurality of taxonomic groups from the FGmap, wherein the positive relationship comprises one of a mutualism, a syntrophic, a commensalism and a cooperation (204B);

generating the TAXA-food constituent network based on a plurality of food constituents for each taxa of the TAXA_NET, wherein the TAXA-food constituent network is an undirected network of each taxa in association with a food constituent used by the taxa as a source of metabolism or nutrition (204C);

generating the food-constituent network based on a plurality of food items for each food constituent of the TAXA-food constituent network, wherein the food-constituent network is an undirected network of each food-constituent present in a food item (204D); and

generating a transpose of the GMP to obtain the ProbMap, wherein the ProbMap is a matrix with rows comprising each metabolite from the plurality of metabolites and the plurality of metabolic pathways and columns comprising the plurality of bacterial strains, probiotics, prebiotics, diet components capable of biosynthesis and/or degradation of the corresponding plurality of metabolites (204E);

receiving a plurality of gut samples of an individual at two-time stamps, via the one or more hardware processors, wherein the two-time stamps comprise a time stamp 1 and a time stamp 2 at which the two samples have been collected (206);

generating a taxa abundance matrix for the plurality of gut samples, via the one or more hardware processors, wherein the taxa abundance matrix comprises of a taxa set 1 and a taxa set 2, where the taxa set 1 and the taxa set 2 is generated using the time stamp 1 and the time stamp 2 (208);

generating a pathway abundance for each of the plurality of gut-samples obtained at the time stamp 1 and the time stamp 2 using the taxa abundance matrix and the FGmap, via the one or more hardware processors, wherein the pathway abundance is generated using a sample processing technique, wherein the pathway abundance for each time stamp is indicative of presence of the plurality of metabolic pathways of the GMP in the gut microbiome of the individual at the corresponding time stamp (210);

identifying a perturbed pathway (212), where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein the perturbed pathway is identified based on one of below cases:

(a) Case A : a decrease in the pathway abundance of the set of beneficial pathways , wherein the decrease is an indicative of a decrease in the set of beneficial microbes, or

(b) Case B : an increase in the pathway abundance of the set of harmful pathways wherein the increase is indicative of an increase in the set of harmful microbes , or

(c) Case C : a decrease in the pathway abundance of the set of beneficial pathways and increase in the pathway abundance of the set of harmful pathways indicative of an increase in set of harmful microbes and decrease in the set of beneficial microbes;

determining, using the ProbMap, an initial set of personalized therapeutics and diet recommendation, wherein the initial set of personalized therapeutics and diet comprises atleast one of : a personalized therapeutic and a personalized dietary regimen, wherein the initial set of personalized therapeutics and diet recommendation is a row of the ProbMap corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap (214);

identifying a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP (216);

identifying a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW (218), wherein the identified set of first neighbors of the TAXA_SET_P in the TAXA_NET graph is saved in the new list TAXA_SET_FN, followed by predicting the functional potential of the taxa in the TAXA_SET_FN and identifying the ones that are commensal based on their butyrate producing capability and save the subset of these taxa from the TAXA_SET_FN as the TAXA_SET_FN_COM, and

identifying a set of taxa common between the TAXA_SET_FN_COM and a set of all taxa present in the time stamp 2 and save the identified set of taxa as the TAXA_SET_INTERSECT and save the remaining taxa in the TAXA_SET_FN_COM which is not present in the time stamp 2 as the TAXA_SET_NEW; and

recommending a personalized therapeutic intervention for improving gut health of the individual (220), via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of:

(a) For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes,
(b) For Case B ( an increase in the pathway abundance of the set of harmful microbes) recommending an optimized diet, wherein the optimized diet promotes growth of the plurality of beneficial microbes and inhibits growth of harmful microbes, and
(c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

2. The processor implemented method of claim 1 wherein the plurality of beneficial metabolic pathways might include one or more of but not limited to Pyruvate to Butyrate production through pyruvate pathway, ammonia oxidation pathway as well as pathway for Trimethylamine oxide (TMA) degradation and the plurality of harmful metabolic pathways might include one or more of but not limited to ammonia releasing pathways, biogenic amine production and trimethylamine production.

3. The processor implemented method of claim 1, wherein the generation of the GMP and FGmap (300) comprises:

generating a matrix Metabolite Map (Mmap) based on the plurality of metabolites, plurality of metabolic pathways and a list of organisms associated with each of the plurality of metabolic pathways, wherein, the Mmap is generated for each the plurality of metabolites (302);
generating a bacterial genome map (BGM) using a bacterial genome database (BGD) wherein the BGD comprises of an exhaustive list the plurality of bacterial strains and the BGM comprises information associated with plurality of bacterial strains including a plurality of names of the plurality of bacterial strains, a plurality of identification number of plurality of bacterial strains, a plurality of gene location of the plurality of bacterial strains and a plurality of protein domain of the plurality of bacterial strains (304);
generation of a Metabolite pathway-domain map (MPDM) for each metabolite from the plurality of metabolites based on the Mmap and the BGM, wherein the MPDM comprises of the exhaustive list of protein domains associated with each of the plurality of metabolic s pathways (306);
identifying the presence of plurality of metabolic pathways in the list of bacterial strains and genomes from the using the MPDM and the BGM based on a first pre-defined threshold criterion (308);
generating the GMP using the BGM, the MPDM, the plurality of metabolic pathways and the plurality of metabolites, wherein the GMP is a matrix with set of bacterial genomes (bacterial strains) as a plurality of rows and the list of plurality of metabolic pathways corresponding to formation of each metabolite as a plurality of columns (310); and
generating the FGmap using the GMP, wherein FGmap is a matrix as the plurality of bacterial genera as a plurality of rows and the plurality of metabolic pathways as columns (312).

4. The processor implemented method of claim 1, wherein the sample processing technique (400) for generating the pathway abundance matrices comprises:

extracting a plurality of nucleic acids from the plurality of gut sample based on a nucleic acid extraction technique; (402)
generating a plurality of nucleotide sequences from the extracted plurality of nucleic acid using a sequencer; (404)
obtaining a set of bacterial taxonomic abundance matrix at each taxonomic hierarchy level using the plurality of nucleotide sequences, wherein the bacterial taxonomic abundance matrix is obtained by classifying the plurality of nucleotide sequences based on a pre-defined taxonomic level using a classification algorithm; (406) and
generating a pathway abundance using the set of bacterial taxonomic abundance matrix, the GMP and the FGmap. (408)

5. The processor implemented method of claim 1, wherein the process of recommending the optimized diet (500) comprises:

computing a normalised change ($d_k$) for every taxa in the union of the taxa set 1 and the taxa set 2, between the time stamp 1 and the time stamp 2 (502);
computing an importance score ($b_k$) of every taxa in the union of the taxa set 1 and the taxa set 2, using the normalised change, the plurality of beneficial microbes, the plurality of harmful microbes from the set of knowledge bases (504);
computing a personalized gut compound score ($gcs_j$) for an individual using the importance score ($b_k$) of every taxa in the union of the taxa set 1 and the taxa set 2 and a pre-defined taxa-compound utilization matrix, wherein the personalized gut compound score quantifies the ability of the compound to increase the plurality of beneficial microbes in the gut (506);
computing a gut food score ($gfs_j$) for all food items in the set of food items using the gut compound score and the food-constituent matrix, wherein the gut food score quantifies the ability of the food items to increase the plurality of beneficial microbes (508); and
optimizing the gut food score using an optimization technique to obtain the optimized diet, wherein the gut food score is optimized subject to a plurality of constraint parameters comprising a calories parameter, a carbohydrate parameter, a protein parameter and a fat parameter (510).

6. The processor implemented method of claim 5, wherein the gut food score of a specific food item ($food_i$) is expressed as below:

$$gfs_i = \sum_{j=1}^{p} \mathbf{C_{j,i}} \times gcs_j$$

*where,*

$\mathbf{C} \in \mathbb{R}^{p \times m}$ is the food-composition matrix, wherein $\mathbf{C_{j,i}}$ is the content of $compound_j$ per unit of $food_i$.
p = total number of compounds obtained in foods from list foods and
m = total number of foods.

$$gcs_j = \sum_{k=1}^{q} T_{j,k} \times b_k \times I[taxa_k \in PU] - \sum_{k=1}^{q} T_{j,k} \times b_k \times I[taxa_k \in AP]$$

Wherein,
T is the taxa-compound utilization matrix,
Wherein,

$$T \in \{0,1\}^{p \times q}; \ T_{j,k} :=$$

$$\left\{ \begin{matrix} 1 \text{ if } taxa_j \text{ can utilize } compound_k \text{ as an energy source} \\ 0 \text{ otherwise} \end{matrix} \right\},$$

q = total number of bacterial taxa;

I is the indicator function, i.e. $I[y \in Z] := \left\{ \begin{matrix} 1 \text{ if } y \in Z \\ 0 \text{ otherwise} \end{matrix} \right\}$,

PU: set of commensal bacterial taxa,
AP: set of pathogenic bacterial taxa,

$$b_k = \max\big((1 + d_k \times I[taxa_k \in AP] - d_i \times I[taxa_k \in PU]), 1\big)$$

Wherein,

$$d_k = \frac{[A_k^2 - A_k^1]}{\sigma_k},$$

Wherein,

$A_k^2$ is the abundance of $taxa_k$ at in the sample T2 ,

$A_k^1$ is the abundance of $taxa_k$ in T1,

$\sigma_k$ is the standard deviation of the $taxa_k$ in a plurality of healthy gut microbiome samples.

7. The processor implemented method of claim 1, wherein the term "perturbed pathway" refers to an increase in harmful pathways or biosynthesis of harmful metabolites or degradation of beneficial metabolites or a decrease in beneficial pathways or beneficial metabolites or degradation of harmful metabolites and the perturbed pathway are identified based on statistical techniques including one of a parametric technique, a non-parametric technique, a Machine learning based algorithm.

8. The processor implemented method of claim 1, wherein the personalized therapeutic intervention further comprises administering a genetically engineered bacteria , wherein the genetically engineered bacteria comprise of genetically engineering (a) a plurality of microbial pathways for production of one or more beneficial metabolites, or/and (b) the plurality of microbial pathways for degradation/elimination of one or more harmful metabolites into the genetic makeup of gut microorganisms, wherein the genetically engineered bacteria is administered in one of the ways shared below:

   (a) genetically engineered as probiotics either alone or in combination with one of a prebiotics, a metabiotics, and a paraprobiotics,
   (b) genetically engineered organism expressing heterologous one or more of pathways required to produce metabolites, wherein the genetically engineered organism can be one or more of a gut commensals wherein the gut commensals may include but not limited to beneficial microbes, commercial probiotic strains or facultative anaerobes capable of residing the gut; wherein the genetically engineered organism is also referred to as modified bacteria, wherein the modified bacteria can be administered as a food product, food supplement or a pharmaceutical composition or a probiotic formulation.

9. A system (100), comprising:

   an input/output interface (106);
   one or more memories (102); and
   one or more hardware processors (104), the one or more memories (104) coupled to the one or more hardware processors (104), wherein the one or more hardware processors (104) are configured to execute programmed instructions stored in the one or more memories (102), to:

   receive a set of input data, via one or more hardware processors, wherein the set of input data comprises data associated with

   a plurality of metabolites, a plurality of metabolic pathways, a plurality of taxonomic groups, information on nutrients, probiotics, prebiotics, supplements for replenishment or degradation of the plurality of metabolites,
   a plurality of food constituents, a plurality of food items, a microbial abundance matrix for healthy cohorts and a plurality of diet optimization pre-requisite parameters,

   wherein the plurality of metabolites comprises a set of beneficial metabolites and a set of harmful metabolites, wherein the set of beneficial metabolites is beneficial for gut health of an individual and the set of harmful metabolites is harmful for the gut health of the individual,
   wherein the plurality of metabolic pathways comprises one of (a) a beneficial pathway (b) harmful pathways,

   wherein the beneficial pathways comprise one or more of a set of metabolic pathways for biosynthesis of plurality of beneficial metabolites and a set of metabolic pathways for degradation of the plurality of harmful metabolites, and
   wherein the harmful pathways comprise one or more of a set of metabolic pathways for degradation of plurality of beneficial metabolites and a set of metabolic pathways for biosynthesis of the plurality harmful metabolites;

wherein the plurality of taxonomic groups comprise a plurality of microbes, where the plurality of microbes comprises a plurality of beneficial microbes and a plurality of harmful microbes, where the plurality of microbes are classified according to a taxonomic hierarchy, wherein the taxonomic hierarchy comprises one or more of a plurality of bacterial phyla, a plurality of bacterial classes, a plurality of bacterial orders, a plurality of bacterial families, a plurality of bacterial genera, a plurality of bacterial species and a plurality of bacterial strains, where the plurality of microbes comprises of a plurality of genes having distinct genomic location, a set of nucleotide and protein sequences of the bacterial strains,
wherein the plurality of beneficial microbes harbours the beneficial pathways and the plurality of harmful microbes harbours the harmful pathways; and
wherein the plurality of diet optimization pre-requisite parameters comprises a set of food items, a complete set of compounds that are found in the set of food items, a food-constituent matrix, wherein the food-constituent matrix tabulates the content of every compound belonging to the set compounds in every food item belonging to the set foods, the total carbohydrate, protein, fats and caloric content of the each of the food items from the set of food items, a list of bacterial taxa in the human gut, a set of functions to identify a set of beneficial bacteria taxa and a set of harmful bacterial taxa, a taxa-compound utilization matrix, a user defined set of upper bound parameters-lower bound parameters associated with carbohydrate, protein, fat and calorie, and a user selected food items;

generate a set of knowledge bases using the set of input data, via the one or more hardware processors, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap wherein the generation of the set of knowledge bases comprises:

generating the FGmap and the GMP map, wherein the FGmap is a matrix associated with a function of the plurality of bacterial genera belonging to one of the plurality of taxonomic group and the plurality of metabolic pathways as columns, wherein a first pre-defined indicator indicates one of (a) a presence of the metabolic pathway and (b) an absence of the metabolic pathway and the GMP is a matrix of the plurality of bacterial strains as rows and the plurality of metabolic pathways as columns, wherein a second pre-defined indicator indicates one of (a) a presence of the metabolic pathway and (b) an absence of the metabolic pathway;
generating the TAXA_NET graph based on the plurality of taxonomic groups and the FGmap, wherein the TAXA_NET graph is a undirected graph, wherein each node in the TAXA_NET graph corresponds to a taxonomic group from the plurality of taxonomic groups and edges indicate a positive relationship between a pair of the taxonomic group wherein each taxa from a plurality of nodes is associated with the function of the plurality of taxonomic groups from the FGmap, wherein the positive relationship comprises one of a mutualism, a syntrophic, a commensalism and a cooperation;
generating the TAXA-food constituent network based on a plurality of food constituents for each taxa of the TAXA_NET, wherein the TAXA-food constituent network is an undirected network of each taxa in association with a food constituent used by the taxa as a source of metabolism or nutrition;
generating the food-constituent network based on a plurality of food items for each food constituent of the TAXA-food constituent network, wherein the food-constituent network is an undirected network of each food-constituent present in a food item; and
generating a transpose of the GMP to obtain the ProbMap, wherein the ProbMap is a matrix with rows comprising each metabolite from the plurality of metabolites and the plurality of metabolic pathways and columns comprising the plurality of bacterial strains, probiotics, prebiotics, diet components capable of biosynthesis and/or degradation of the corresponding plurality of metabolites;

receive a plurality of gut samples of an individual at two-time stamps, via the one or more hardware processors, wherein the two-time stamps comprise a time stamp 1 and a time stamp 2 at which the two samples have been collected;
generate a taxa abundance matrix for the plurality of gut samples, via the one or more hardware processors, wherein the taxa abundance matrix comprises of a taxa set 1 and a taxa set 2, where the taxa set 1 and the taxa set 2 is generated using the time stamp 1 and the time stamp 2;

generate a pathway abundance for each of the plurality of gut-samples obtained at the time stamp 1 and the time stamp 2 using the taxa abundance matrix and the FGmap, via the one or more hardware processors, wherein the pathway abundance is generated using a sample processing technique, wherein the pathway abundance for each time stamp is indicative of presence of the plurality of metabolic pathways of the GMP in the gut microbiome of the individual at the corresponding time stamp;

identify a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein the perturbed pathway is identified based on one of below cases:

(a) Case A : a decrease in the pathway abundance of the set of beneficial pathways , wherein the decrease is an indicative of a decrease in the set of beneficial microbes, or

(b) Case B : an increase in the pathway abundance of the set of harmful pathways wherein the increase is indicative of an increase in the set of harmful microbes , or

(c) Case C : a decrease in the pathway abundance of the set of beneficial pathways and increase in the pathway abundance of the set of harmful pathways indicative of an increase in set of harmful microbes and decrease in the set of beneficial microbes;

determine, using the ProbMap, an initial set of personalized therapeutics and diet recommendation, wherein the initial set of personalized therapeutics and diet comprises atleast one of : a personalized therapeutic and a personalized dietary regimen, wherein the initial set of personalized therapeutics and diet recommendation is a row of the ProbMap corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap;

identify a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP;

identify a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW, wherein the identified set of first neighbors of the TAXA_SET_P in the TAXA_NET graph is saved in the new list TAXA_SET_FN, followed by predicting the functional potential of the taxa in the TAXA_SET_FN and identifying the ones that are commensal based on their butyrate producing capability and save the subset of these taxa from the TAXA_SET_FN as the TAXA_SET_FN_COM, and

identifying a set of taxa common between the TAXA_SET_FN_COM and a set of all taxa present in the time stamp 2 and save the identified set of taxa as the TAXA_SET_INTERSECT and save the remaining taxa in the TAXA_SET_FN_COM which is not present in the time stamp 2 as the TAXA_SET_NEW; and

recommend a personalized therapeutic intervention for improving gut health of the individual, via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of:

(a)For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes,

(b)For Case B ( an increase in the pathway abundance of the set of harmful microbes) recommending an optimized diet, wherein the optimized diet promotes growth of the plurality of beneficial microbes and inhibits growth of harmful microbes, and

(c)For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

10. The system of claim 9, wherein the one or more hardware processors are configured by the instructions to implement the generation of the GMP and FGmap comprising:

   generating a matrix Metabolite Map (Mmap) based on the plurality of metabolites, plurality of metabolic pathways and a list of organisms associated with each of the plurality of metabolic pathways, wherein, the Mmap is generated for each the plurality of metabolites;
   generating a bacterial genome map (BGM) using a bacterial genome database (BGD) wherein the BGD comprises of an exhaustive list the plurality of bacterial strains and the BGM comprises information associated with plurality of bacterial strains including a plurality of names of the plurality of bacterial strains, a plurality of identification number of plurality of bacterial strains, a plurality of gene location of the plurality of bacterial strains and a plurality of protein domain of the plurality of bacterial strains;
   generation of a Metabolite pathway-domain map (MPDM) for each metabolite from the plurality of metabolites based on the Mmap and the BGM, wherein the MPDM comprises of the exhaustive list of protein domains associated with each of the plurality of metabolic s pathways;
   identifying the presence of plurality of metabolic pathways in the list of bacterial strains and genomes from the using the MPDM and the BGM based on a first pre-defined threshold criterion;
   generating the GMP using the BGM, the MPDM, the plurality of metabolic pathways and the plurality of metabolites, wherein the GMP is a matrix with set of bacterial genomes (bacterial strains) as a plurality of rows and the list of plurality of metabolic pathways corresponding to formation of each metabolite as a plurality of columns; and
   generating the FGmap using the GMP, wherein FGmap is a matrix as the plurality of bacterial genera as a plurality of rows and the plurality of metabolic pathways as columns.

11. The system of claim 9, wherein the one or more hardware processors are configured by the instructions to implement the sample processing technique (400) for generating the pathway abundance matrices comprises:

   extracting a plurality of nucleic acids from the plurality of gut sample based on a nucleic acid extraction technique;
   generating a plurality of nucleotide sequences from the extracted plurality of nucleic acid using a sequencer;
   obtaining a set of bacterial taxonomic abundance matrix at each taxonomic hierarchy level using the plurality of nucleotide sequences, wherein the bacterial taxonomic abundance matrix is obtained by classifying the plurality of nucleotide sequences based on a pre-defined taxonomic level using a classification algorithm; and
   generating a pathway abundance using the set of bacterial taxonomic abundance matrix, the GMP and the FGmap.

12. The system of claim 9, wherein the one or more hardware processors are configured by the instructions to implement the process of recommending the optimized diet comprises:

   computing a normalised change ($d_k$) for every taxa in the union of the taxa set 1 and the taxa set 2, between the time stamp 1 and the time stamp 2 ;
   computing an importance score ($b_k$) of every taxa in the union of the taxa set 1 and the taxa set 2, using the normalised change, the plurality of beneficial microbes, the plurality of harmful microbes from the set of knowledge bases;
   computing a personalized gut compound score ($gcs_j$) for an individual using the importance score ($b_k$) of every taxa in the union of the taxa set 1 and the taxa set 2 and a pre-defined taxa-compound utilization matrix, wherein the personalized gut compound score quantifies the ability of the compound to increase the plurality of beneficial microbes in the gut;
   computing a gut food score ($gfs_j$) for all food items in the set of food items using the gut compound score and the food-constituent matrix, wherein the gut food score quantifies the ability of the food items to increase the plurality of beneficial microbes; and
   optimizing the gut food score using an optimization technique to obtain the optimized diet, wherein the gut food score is optimized subject to a plurality of constraint parameters comprising a calories parameter, a carbohydrate parameter, a protein parameter and a fat parameter.

13. The system of claim 12, wherein the one or more hardware processors are configured by the instructions to implement the computation of the gut food score of a specific food item ($food_i$), expressed as shown below :

EP 4 288 559 B1

$$gfs_i = \sum_{j=1}^{p} C_{j,i} \times gcs_j$$

where,

$C \in \mathbb{R}^{p \times m}$ is the food-composition matrix, wherein $C_{j,i}$ is the content of compound$_j$ per unit of food$_i$. p = total number of compounds obtained in foods from list foods and m = total number of foods.

$$gcs_j = \sum_{k=1}^{q} T_{j,k} \times b_k \times I[taxa_k \in PU] - \sum_{k=1}^{q} T_{j,k} \times b_k \times I[taxa_k \in AP]$$

Wherein,

T is the taxa-compound utilization matrix,
Wherein,

$$T \in \{0, 1\}^{p \times q}; \ T_{j,k} :=$$

$$\left\{ \begin{matrix} 1 \text{ if taxa}_j \text{ can utilize compound}_k \text{ as an energy source} \\ 0 \text{ otherwise} \end{matrix} \right\},$$

q = total number of bacterial taxa;

I is the indicator function, i.e. $I[y \in Z] := \left\{ \begin{matrix} 1 \text{ if } y \in Z \\ 0 \text{ otherwise} \end{matrix} \right\}$,

PU : set of commensal bacterial taxa,
AP: set of pathogenic bacterial taxa,

$$b_k = \max\big((1 + d_k \times I[taxa_k \in AP] - d_i \times I[taxa_k \in PU]), 1\big)$$

Wherein,

$$d_k = \frac{[A_k^2 - A_k^1]}{\sigma_k},$$

Wherein,

$A_k^2$ is the abundance of taxa$_k$ at in the sample T2 ,

$A_k^1$ is the abundance of taxa$_k$ in T1,
$\sigma_k$ is the standard deviation of the taxa$_k$ in a plurality of healthy gut microbiome samples.

14. The system of claim 9, wherein the one or more hardware processors are configured by the instructions to implement the personalized therapeutic intervention further comprises administering a genetically engineered bacteria , wherein the genetically engineered bacteria comprise of genetically engineering (a) a plurality of microbial pathways for production of one or more beneficial metabolites, or/and (b) the plurality of microbial pathways for degradation/elimination of one or more harmful metabolites into the genetic makeup of gut microorganisms, wherein the genetically engineered bacteria is administered in one of the ways shared below:

(a) genetically engineered as probiotics either alone or in combination with one of a prebiotics, a metabiotics, and a paraprobiotics,
(b) genetically engineered organism expressing heterologous one or more of pathways required to produce metabolites, wherein the genetically engineered organism can be one or more of a gut commensals wherein the gut commensals may include but not limited to beneficial microbes, commercial probiotic strains or facultative anaerobes capable of residing the gut; wherein the genetically engineered organism is also referred to as modified bacteria, wherein the modified bacteria can be administered as a food product, food supplement or a pharmaceutical composition or a probiotic formulation.

15. One or more non-transitory machine-readable information storage mediums comprising one or more instructions which when executed by one or more hardware processors cause:

receive a set of input data, via one or more hardware processors, wherein the set of input data comprises data associated with

a plurality of metabolites, a plurality of metabolic pathways, a plurality of taxonomic groups,
information on nutrients, probiotics, prebiotics, supplements for replenishment or degradation of the plurality of metabolites,
a plurality of food constituents, a plurality of food items, a microbial abundance matrix for healthy cohorts and a plurality of diet optimization pre-requisite parameters,

wherein the plurality of metabolites comprises a set of beneficial metabolites and a set of harmful metabolites, wherein the set of beneficial metabolites is beneficial for gut health of an individual and the set of harmful metabolites is harmful for the gut health of the individual,
wherein the plurality of metabolic pathways comprises one of (a) a beneficial pathway (b) harmful pathways,

wherein the beneficial pathways comprise one or more of a set of metabolic pathways for biosynthesis of plurality of beneficial metabolites and a set of metabolic pathways for degradation of the plurality of harmful metabolites, and
wherein the harmful pathways comprise one or more of a set of metabolic pathways for degradation of plurality of beneficial metabolites and a set of metabolic pathways for biosynthesis of the plurality harmful metabolites;

wherein the plurality of taxonomic groups comprise a plurality of microbes, where the plurality of microbes comprises a plurality of beneficial microbes and a plurality of harmful microbes, where the plurality of microbes are classified according to a taxonomic hierarchy, wherein the taxonomic hierarchy comprises one or more of a plurality of bacterial phyla, a plurality of bacterial classes, a plurality of bacterial orders, a plurality of bacterial families, a plurality of bacterial genera, a plurality of bacterial species and a plurality of bacterial strains, where the plurality of microbes comprises of a plurality of genes having distinct genomic location, a set of nucleotide and protein sequences of the bacterial strains,
wherein the plurality of beneficial microbes harbours the beneficial pathways and the plurality of harmful microbes harbours the harmful pathways; and
wherein the plurality of diet optimization pre-requisite parameters comprises a set of food items, a complete set of compounds that are found in the set of food items, a food-constituent matrix, wherein the food-constituent matrix tabulates the content of every compound belonging to the set compounds in every food item belonging to the set foods, the total carbohydrate, protein, fats and caloric content of the each of the food items from the set of food items, a list of bacterial taxa in the human gut, a set of functions to identify a set of beneficial bacteria taxa and a set of harmful bacterial taxa, a taxa-compound utilization matrix, a user defined set of upper bound parameters-lower bound parameters associated with carbohydrate, protein, fat and calorie, and a user selected food items;

generate a set of knowledge bases using the set of input data, via the one or more hardware processors, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap wherein the generation of the set of knowledge bases comprises:

generating the FGmap and the GMP map, wherein the FGmap is a matrix associated with a function of the plurality of bacterial genera belonging to one of the plurality of taxonomic group and the plurality of metabolic pathways as columns, wherein a first pre-defined indicator indicates one of (a) a presence of the metabolic pathway and (b) an absence of the metabolic pathway and the GMP is a matrix of the plurality of bacterial strains as rows and the plurality of metabolic pathways as columns, wherein a second pre-defined indicator indicates one of (a) a presence of the metabolic pathway and (b) an absence of the metabolic pathway;

generating the TAXA_NET graph based on the plurality of taxonomic groups and the FGmap, wherein the TAXA_NET graph is a undirected graph, wherein each node in the TAXA_NET graph corresponds to a taxonomic group from the plurality of taxonomic groups and edges indicate a positive relationship between a pair of the taxonomic group wherein each taxa from a plurality of nodes is associated with the function of the plurality of taxonomic groups from the FGmap, wherein the positive relationship comprises one of a mutualism, a syntrophic, a commensalism and a cooperation;

generating the TAXA-food constituent network based on a plurality of food constituents for each taxa of the TAXA_NET, wherein the TAXA-food constituent network is an undirected network of each taxa in association with a food constituent used by the taxa as a source of metabolism or nutrition;

generating the food-constituent network based on a plurality of food items for each food constituent of the TAXA-food constituent network, wherein the food-constituent network is an undirected network of each food-constituent present in a food item; and

generating a transpose of the GMP to obtain the ProbMap, wherein the ProbMap is a matrix with rows comprising each metabolite from the plurality of metabolites and the plurality of metabolic pathways and columns comprising the plurality of bacterial strains, probiotics, prebiotics, diet components capable of biosynthesis and/or degradation of the corresponding plurality of metabolites;

receive a plurality of gut samples of an individual at two-time stamps, via the one or more hardware processors, wherein the two-time stamps comprise a time stamp 1 and a time stamp 2 at which the two samples have been collected;

generate a taxa abundance matrix for the plurality of gut samples, via the one or more hardware processors, wherein the taxa abundance matrix comprises of a taxa set 1 and a taxa set 2, where the taxa set 1 and the taxa set 2 is generated using the time stamp 1 and the time stamp 2;

generate a pathway abundance for each of the plurality of gut-samples obtained at the time stamp 1 and the time stamp 2 using the taxa abundance matrix and the FGmap, via the one or more hardware processors, wherein the pathway abundance is generated using a sample processing technique, wherein the pathway abundance for each time stamp is indicative of presence of the plurality of metabolic pathways of the GMP in the gut microbiome of the individual at the corresponding time stamp;

identify a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein the perturbed pathway is identified based on one of below cases:

(a) Case A : a decrease in the pathway abundance of the set of beneficial pathways , wherein the decrease is an indicative of a decrease in the set of beneficial microbes, or

(b) Case B : an increase in the pathway abundance of the set of harmful pathways wherein the increase is indicative of an increase in the set of harmful microbes , or

(c) Case C : a decrease in the pathway abundance of the set of beneficial pathways and increase in the pathway abundance of the set of harmful pathways indicative of an increase in set of harmful microbes and decrease in the set of beneficial microbes;

determine, using the ProbMap, an initial set of personalized therapeutics and diet recommendation, wherein the initial set of personalized therapeutics and diet comprises atleast one of : a personalized therapeutic and a personalized dietary regimen, wherein the initial set of personalized therapeutics and diet recommendation is a row of the ProbMap corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap;

identify a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP;

identify a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, a TAXA_SET_NEW, wherein the identified set of first neighbors of the TAXA_SET_P in the TAXA_NET graph is saved in the new list TAXA_SET_FN, followed by predicting the functional potential of the taxa in the TAXA_SET_FN and identifying the ones that are commensal based on their butyrate producing capability and save the subset of these taxa from the TAXA_SET_FN as the TAXA_SET_FN_COM, and

identifying a set of taxa common between the TAXA_SET_FN_COM and a set of all taxa present in the time stamp 2 and save the identified set of taxa as the TAXA_SET_INTERSECT and save the remaining taxa in the TAXA_SET_FN_COM which is not present in the time stamp 2 as the TAXA_SET_NEW; and

recommend a personalized therapeutic intervention for improving gut health of the individual, via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of:

(a)For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes, (b)For Case B (an increase in the pathway abundance of the set of harmful microbes) recommending an optimized diet, wherein the optimized diet promotes growth of the plurality of beneficial microbes and inhibits growth of harmful microbes, and

(c)For Case C (a decrease in the pathway abundance of the set of beneficial microbes and increase in the pathway abundance of the set of harmful microbes) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes.

**Patentansprüche**

1. Prozessorimplementiertes Verfahren (200) zum Entwerfen personalisierter Therapeutika und Diäten basierend auf Funktionen von Mikrobiomen, umfassend:

Empfangen eines Satzes von Eingabedaten (202) über einen oder mehrere Hardwareprozessoren, wobei der Satz von Eingabedaten Daten umfasst, die mit Folgendem assoziiert sind:

einer Mehrzahl von Metaboliten, einer Mehrzahl von Stoffwechselpfaden, einer Mehrzahl von taxonomischen Gruppen, Informationen über Nährstoffe, Probiotika, Präbiotika, Ergänzungsmitteln zum Auffüllen oder Abbauen der Mehrzahl von Metaboliten, einer Mehrzahl von Lebensmittelbestandteilen, einer Mehrzahl von Lebensmitteln, einer mikrobiellen Häufigkeitsmatrix für gesunde Kohorten und einer Mehrzahl von Diätoptimierungsvoraussetzungsparametern,

wobei die Mehrzahl von Metaboliten einen Satz von nützlichen Metaboliten und einen Satz von schädlichen Metaboliten umfasst, wobei der Satz von nützlichen Metaboliten für die Darmgesundheit eines Individuums nützlich ist und der Satz von schädlichen Metaboliten für die Darmgesundheit des Individuums schädlich ist, wobei die Mehrzahl von Stoffwechselpfaden einen von (a) einem nützlichen Pfad und (b) schädlichen Pfaden umfasst,

wobei die nützlichen Pfade einen oder mehrere von einem Satz von Stoffwechselpfaden für die Biosynthese einer Mehrzahl von nützlichen Metaboliten und einem Satz von Stoffwechselpfaden für den Abbau der Mehrzahl von schädlichen Metaboliten umfassen, und

wobei die schädlichen Pfade einen oder mehrere von einem Satz von Stoffwechselpfaden für den Abbau einer Mehrzahl von nützlichen Metaboliten und einem Satz von Stoffwechselpfaden für die Biosynthese der Mehrzahl von schädlichen Metaboliten umfassen;

wobei die Mehrzahl von taxonomischen Gruppen eine Mehrzahl von Mikroben umfasst, wobei die Mehrzahl von Mikroben eine Mehrzahl von nützlichen Mikroben und eine Mehrzahl von schädlichen Mikroben umfasst, wobei die Mehrzahl von Mikroben gemäß einer taxonomischen Hierarchie klassifiziert ist, wobei die taxonomische Hierarchie eine oder mehrere von einer Mehrzahl von Bakterienstämmen, einer Mehrzahl von Bakterienklassen, einer Mehrzahl von Bakterienordnungen, einer Mehrzahl von Bakterienfamilien, einer Mehrzahl von Bakteriengattungen, einer Mehrzahl von Bakterienarten und einer Mehrzahl von Bakterienstämmen umfasst, wobei die Mehrzahl von Mikroben eine Mehrzahl von Genen mit einer eindeutigen genomischen Position, einen Satz von Nukleotid- und Proteinsequenzen der Bakterienstämme umfasst, wobei die Mehrzahl von nützlichen Mikroben die nützlichen Pfade beherbergt und die Mehrzahl von schädlichen Mikroben die schädlichen Pfade beherbergt; und

wobei die Mehrzahl von Diätoptimierungsvoraussetzungsparametern einen Satz von Lebensmitteln, einen vollständigen Satz von Verbindungen, die in dem Satz von Lebensmitteln zu finden sind, eine Lebensmittelbestandteilmatrix umfasst, wobei die Lebensmittelbestandteilmatrix den Gehalt jeder Verbindung, die zu dem Satz von Verbindungen gehört, in jedem Lebensmittel, das zu dem Satz von Lebensmitteln gehört, den Gesamtkohlenhydrat-, Protein-, Fett- und Kaloriengehalt jedes der Lebensmittel aus dem Satz von Lebensmitteln, eine Liste von Bakterientaxa im menschlichen Darm, einen Satz von Funktionen zum Identifizieren eines Satzes von nützlichen Bakterientaxa und eines Satzes von schädlichen Bakterientaxa, eine Taxaverbindungsnutzungsmatrix, einen benutzerdefinierten Satz von obergebundenen Parameternuntergebundenen Parametern, die mit Kohlenhydrat, Protein, Fett und Kalorie assoziiert sind, und ein benutzerausgewähltes Lebensmittel tabelliert;

Erzeugen eines Satzes von Wissensdatenbanken unter Verwendung des Satzes von Eingabedaten (204) über den einen oder die mehreren Hardwareprozessoren, wobei der Satz von Wissensdatenbanken eine Gattung-Funktions-Karte (Genus Function Map, FGmap), eine Genom-Metabolit-Pfad-Karte (GMP), ein Taxaassoziationsreferenzprofil-Graph (TAXA_NET), ein TAXA-Lebensmittel-Bestandteilnetzwerk, ein Lebensmittel-Bestandteilnetzwerk und eine ProbMap umfasst, wobei die Erzeugung des Satzes von Wissensdatenbanken umfasst:

Erzeugen der FGmap und der GMP-Karte, wobei die FGmap eine Matrix ist, die mit einer Funktion von der Mehrzahl von Bakteriengattungen, die zu einer der Mehrzahl von taxonomischen Gruppen gehören, und der Mehrzahl von metabolischen Pfaden als Spalten assoziiert ist, wobei ein erster vordefinierter Indikator eines von (a) einem Vorhandensein des metabolischen Pfads und (b) einem Fehlen des metabolischen Pfads anzeigt und die GMP eine Matrix der Mehrzahl von Bakterienstämmen als Reihen und der Mehrzahl von metabolischen Pfaden als Spalten ist, wobei ein zweiter vordefinierter Indikator eines von (a) einem Vorhandensein des metabolischen Pfads und (b) einem Fehlen des metabolischen Pfads (204A) anzeigt;

Erzeugen des TAXA_NET-Graphen basierend auf der Mehrzahl von taxonomischen Gruppen und der FGmap, wobei der TAXA_NET-Graph ein ungerichteter Graph ist, wobei jeder Knoten in dem TAXA_NET-Graph einer taxonomischen Gruppe aus der Mehrzahl von taxonomischen Gruppen entspricht und Kanten eine positive Beziehung zwischen einem Paar der taxonomischen Gruppe anzeigen, wobei jedes Taxa aus einer Mehrzahl von Knoten mit der Funktion der Mehrzahl von taxonomischen Gruppen aus der FGmap assoziiert ist, wobei die positive Beziehung eines von einem Mutualismus, einem Syntrophikum, einem Kommensalismus und einer Kooperation (204B) umfasst;

Erzeugen des TAXA-Lebensmittel-Bestandteilnetzwerks basierend auf einer Mehrzahl von Lebensmittelbestandteilen für jedes Taxa des TAXA_NET, wobei das TAXA-Lebensmittel-Bestandteilnetzwerk ein ungerichtetes Netzwerk jedes Taxas in Assoziation mit einem Lebensmittelbestandteil ist, der von dem Taxa als eine Quelle von Metabolismus oder Ernährung verwendet wird (204C);

Erzeugen des Lebensmittel-Bestandteilnetzwerks basierend auf einer Mehrzahl von Lebensmitteln für jeden Lebensmittelbestandteil des TAXA-Lebensmittel-Bestandteilnetzwerks, wobei das Lebensmittel-Bestandteilnetzwerk ein ungerichtetes Netzwerk jedes Lebensmittelbestandteils ist, der in einem Lebensmittel vorhanden ist (204D); und

Erzeugen einer Transponierung des GMP, um die ProbMap zu erhalten, wobei die ProbMap eine Matrix mit Zeilen ist, die jeden Metaboliten aus der Mehrzahl von Metaboliten und der Mehrzahl von metabolischen Pfaden und Spalten umfassen, die die Mehrzahl von Bakterienstämmen, Probiotika, Präbiotika, Diätkomponenten umfassen, die zur Biosynthese und/oder zum Abbau der entsprechenden Mehrzahl von Metaboliten in der Lage sind (204E);

Empfangen einer Mehrzahl von Darmproben eines Individuums an Zwei-Zeit-Stempeln über den einen oder die mehreren Hardwareprozessoren, wobei die Zwei-Zeit-Stempel einen Zeitstempel 1 und einen Zeitstempel 2 umfassen, an denen die zwei Proben gesammelt wurden (206);

Erzeugen einer Taxa-Häufigkeitsmatrix für die Mehrzahl von Darmproben über den einen oder die mehreren Hardwareprozessoren, wobei die Taxa-Häufigkeitsmatrix einen Taxa-Satz 1 und einen Taxa-Satz 2 umfasst, wobei der Taxa-Satz 1 und der Taxa-Satz 2 unter Verwendung des Zeitstempels 1 und des Zeitstempels 2 erzeugt werden (208);

Erzeugen einer Pfadhäufigkeit für jede der Mehrzahl von Darmproben, die an dem Zeitstempel 1 und dem Zeitstempel 2 erhalten wurden, unter Verwendung der Taxa-Häufigkeitsmatrix und der FGmap über den einen oder die mehreren Hardwareprozessoren, wobei die Pfadhäufigkeit unter Verwendung einer Probenverarbeitungstechnik erzeugt wird, wobei die Pfadhäufigkeit für jeden Zeitstempel das Vorhandensein der Mehrzahl von metabolischen Pfaden des GMP in dem Darmmikrobiom des Individuums an dem entsprechenden Zeitstempel anzeigt (210);

Identifizieren eines gestörten Pfades (212), wobei der gestörte Pfad eine Verschlechterung der Darmgesundheit des Individuums anzeigt, wobei der gestörte Pfad basierend auf einem Vergleich der Pfadhäufigkeit der Mehrzahl von Darmproben, die an dem Zeitstempel 1 und dem Zeitstempel 2 erhalten wurden, unter Verwendung einer statistischen Technik identifiziert wird, wobei der gestörte Pfad basierend auf einem der folgenden Fälle identifiziert wird:

(a) Fall A: eine Abnahme der Pfadhäufigkeit des Satzes von nützlichen Pfaden , wobei die Abnahme eine Abnahme des Satzes von nützlichen Mikroben anzeigt, oder

(b) Fall B: eine Zunahme der Pfadhäufigkeit des Satzes von schädlichen Pfaden, wobei die Zunahme eine Zunahme des Satzes von schädlichen Mikroben anzeigt, oder

(c) Fall C: eine Abnahme der Pfadhäufigkeit des Satzes von nützlichen Pfaden und eine Zunahme der Pfadhäufigkeit des Satzes von schädlichen Pfaden, die eine Zunahme des Satzes von schädlichen Mikroben und eine Abnahme des Satzes von nützlichen Mikroben anzeigen;

Bestimmen, unter Verwendung der ProbMap, eines anfänglichen Satzes von personalisierten Therapeutika und einer Ernährungsempfehlung, wobei der anfängliche Satz von personalisierten Therapeutika und Ernährung mindestens eines umfasst von: einem personalisierten Therapeutikum und einem personalisierten Ernährungsplan, wobei der anfängliche Satz von personalisierten Therapeutika und Ernährungsempfehlung eine Reihe der ProbMap ist, die dem identifizierten gestörten Pfad entspricht, wobei das personalisierte Therapeutikum bestimmt wird als (a) die Mehrzahl von Bakterienstämmen, die den gestörten Pfad besitzen, oder (b) die Mehrzahl von Bakterienstämmen, die den Pfad für den Abbau oder/und die Biosynthese eines wahrscheinlichen Metaboliten besitzen, wobei der wahrscheinliche Metabolit ein Metabolit ist, der dem gestörten Pfad in der ProbMap entspricht (214);

Identifizieren eines Taxasatzes in der Taxahäufigkeitsmatrix, die an dem Zeitstempel 2 erhalten wurde, wobei der Taxasatz eine TAXA_SET_P und eine TAXA_SET_A umfasst, wobei die TAXA_SET_P einen vordefinierten ersten Wert für den einen oder die mehreren von dem nützlichen Pfad in einer von der FGmap und dem GMP umfasst, und die TAXA_SET_A einen vordefinierten zweiten Wert für den einen oder die mehreren von schädlichen Pfaden in einer von der FGmap und dem GMP umfasst (216);

Identifizieren eines Satzes von ersten Nachbarn für jedes der Taxa in der TAXA_SET_P an dem Zeitstempel 2 aus dem TAXA_NET-Graphen, wobei der Satz von ersten Nachbarn unmittelbare Nachbarn der TAXA_SET_P sind umfassend eine TAXA_SET_FN, eine TAXA_SET_FN_COM, eine TAXA_SET_INTERSECT, eine TAXA_SET_NEW (218), wobei der identifizierte Satz von ersten Nachbarn der TAXA_SET_P in dem TAXA_NET-Graphen in der neuen Liste TAXA_SET_FN gespeichert wird, gefolgt von dem Vorhersagen des Funktionspotentials der Taxa in der TAXA_SET_FN und dem Identifizieren derjenigen, die kommenal sind, basierend auf ihrer Butyratproduktionsfähigkeit und dem Speichern der Teilmenge dieser Taxa aus der TAXA_SET_FN als die TAXA_SET_FN_COM, und

Identifizieren eines Satzes von Taxa, die zwischen der TAXA_SET_FN_COM und einem Satz aller Taxa, die in dem Zeitstempel 2 vorhanden sind, gemeinsam sind, und Speichern des identifizierten Satzes von Taxa als die TAXA_SET_INTERSECT und Speichern der verbleibenden Taxa in der TAXA_SET_FN_COM, die nicht in dem Zeitstempel 2 vorhanden ist, als die TAXA_SET_NEW; und

Empfehlen einer personalisierten therapeutischen Intervention zum Verbessern der Darmgesundheit des Individuums (220) über den einen oder die mehreren Hardwareprozessoren, wobei die therapeutische Intervention das Empfehlen von einem von (a) einem präbiotischen Cocktail, (b) einem probiotischen Cocktail, (c) optimierter Ernährung basierend auf den identifizierten ersten Nachbarn unter Verwendung des Satzes von Wissensdatenbanken und des anfänglichen Satzes von personalisierten Therapeutika und Ernährungsempfehlung umfasst, wobei die personalisierte therapeutische Intervention mindestens eines oder mehrere umfasst von:

(a) für Fall A (eine Abnahme der Pfad-Häufigkeit des Satzes von nützlichen Mikroben) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt und das Wachstum der nützlichen Mikroben unterstützt,

(b) für Fall B (eine Zunahme der Pfad-Häufigkeit des Satzes von schädlichen Mikroben) Empfehlen einer optimierten Ernährung, wobei die optimierte Ernährung das Wachstum der Mehrzahl von nützlichen Mikroben fördert und das Wachstum von schädlichen Mikroben hemmt, und

(c) für Fall C (eine Abnahme der Pfad-Häufigkeit des Satzes von nützlichen Mikroben und eine Zunahme der Pfad-Häufigkeit des Satzes von schädlichen Mikroben ) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt, das Wachstum von nützlichen Mikroben unterstützt und das Wachstum von schädlichen Mikroben hemmt.

2. Prozessorimplementiertes Verfahren nach Anspruch 1, wobei die Mehrzahl von nützlichen metabolischen Pfaden einen oder mehrere von, aber nicht beschränkt auf Pyruvat-zu-Butyrat-Produktion durch Pyruvat-Pfad, Ammoniak-Oxidations-Pfad sowie Pfad für Trimethylaminoxid(TMA)-Abbau beinhalten könnte und die Mehrzahl von schädlichen metabolischen Pfaden einen oder mehrere von, aber nicht beschränkt auf Ammoniak-freisetzende Pfade, biogene Aminproduktion und Trimethylaminproduktion beinhalten könnte.

3. Prozessorimplementiertes Verfahren nach Anspruch 1, wobei die Erzeugung der GMP und FGmap (300) umfasst:

Erzeugen einer Matrix-Metaboliten-Map (Mmap) basierend auf der Mehrzahl von Metaboliten, der Mehrzahl von metabolischen Pfaden und einer Liste von Organismen, die mit jedem der Mehrzahl von metabolischen Pfaden assoziiert sind, wobei die Mmap für jeden der Mehrzahl von Metaboliten (302) erzeugt wird;

Erzeugen einer bakteriellen Genomkarte (Bacterial Genome Map, BGM) unter Verwendung einer bakteriellen Genomdatenbank (BGD), wobei die BGD aus einer erschöpfenden Liste die Mehrzahl von Bakterienstämmen umfasst und die BGM Informationen umfasst, die mit der Mehrzahl von Bakterienstämmen assoziiert sind, einschließlich einer Mehrzahl von Namen der Mehrzahl von Bakterienstämmen, einer Mehrzahl von Identifikationsnummern der Mehrzahl von Bakterienstämmen, einer Mehrzahl von Genorten der Mehrzahl von Bakterienstämmen und einer Mehrzahl von Proteindomänen der Mehrzahl von Bakterienstämmen (304);

Erzeugen einer Metaboliten-Pfad-Domänen-Karte (Metabolite Pathway-Doman Map, MPDM) für jeden Metaboliten aus der Mehrzahl von Metaboliten basierend auf der Mmap und der BGM, wobei die MPDM aus der erschöpfenden Liste von Proteindomänen besteht, die mit jedem der Mehrzahl von metabolischen Pfaden (306) assoziiert sind;

Identifizieren des Vorhandenseins einer Mehrzahl von metabolischen Pfaden in der Liste von Bakterienstämmen und Genomen aus der unter Verwendung der MPDM und der BGM basierend auf einem ersten vordefinierten Schwellenwertkriterium (308);

Erzeugen der GMP unter Verwendung der BGM, der MPDM, der Mehrzahl von metabolischen Pfaden und der Mehrzahl von Metaboliten, wobei die GMP eine Matrix mit einem Satz von bakteriellen Genomen (Bakterienstämmen) als eine Mehrzahl von Zeilen und die Liste der Mehrzahl von metabolischen Pfaden, die der Bildung jedes Metaboliten entsprechen, als eine Mehrzahl von Spalten (310) ist; und

Erzeugen der FGmap unter Verwendung der GMP, wobei FGmap eine Matrix als die Mehrzahl von Bakteriengattungen als eine Mehrzahl von Reihen und die Mehrzahl von metabolischen Pfaden als Spalten (312) ist.

4. Prozessorimplementiertes Verfahren nach Anspruch 1, wobei die Probenverarbeitungstechnik (400) zum Erzeugen der Pfad-Häufigkeit-Matrizen umfasst:

Extrahieren einer Mehrzahl von Nukleinsäuren aus der Mehrzahl von Darmproben basierend auf einer Nukleinsäureextraktionstechnik; (402)

Erzeugen einer Mehrzahl von Nukleotidsequenzen aus der extrahierten Mehrzahl von Nukleinsäuren unter Verwendung eines Sequenzierers; (404)

Erhalten eines Satzes von bakterieller taxonomischer Häufigkeit-Matrix auf jeder taxonomischen Hierarchieebene unter Verwendung der Mehrzahl von Nukleotidsequenzen, wobei die bakterielle taxonomische Häufigkeit-Matrix durch Klassifizieren der Mehrzahl von Nukleotidsequenzen basierend auf einer vordefinierten taxonomischen Ebene unter Verwendung eines Klassifizierungsalgorithmus erhalten wird; (406) und

Erzeugen einer Pfad-Häufigkeit unter Verwendung des Satzes von bakterieller taxonomischer Häufigkeit-Matrix, der GMP und der FGmap. (408)

5. Prozessorimplementiertes Verfahren nach Anspruch 1, wobei der Prozess des Empfehlens der optimierten Diät (500) umfasst:

Berechnen einer normalisierten Änderung ($d_k$) für jedes Taxa in der Vereinigung des Taxa-Satzes 1 und des Taxa-Satzes 2 zwischen dem Zeitstempel 1 und dem Zeitstempel 2 (502);

Berechnen einer Wichtigkeitsbewertung ($b_k$) für jedes Taxa in der Vereinigung des Taxa-Satzes 1 und des Taxa-Satzes 2 unter Verwendung der normalisierten Änderung, der Mehrzahl von nützlichen Mikroben, der Mehrzahl von schädlichen Mikroben aus dem Satz von Wissensdatenbanken (504);

Berechnen einer personalisierten Darmzusammensetzungsbewertung ($gcs_j$) für ein Individuum unter Verwendung der Wichtigkeitsbewertung ($b_k$) für jedes Taxa in der Vereinigung des Taxa-Satzes 1 und des Taxa-Satzes 2 und einer vordefinierten Taxa-Zusammensetzung-Nutzungsmatrix, wobei die personalisierte Darmzusammensetzungsbewertung die Fähigkeit der Zusammensetzung quantifiziert, die Mehrzahl von nützlichen Mikroben im Darm zu erhöhen (506);

Berechnen einer Darm-Lebensmittel-Bewertung ($gfs_j$) für alle Lebensmittel in dem Satz von Lebensmitteln unter Verwendung der Darmzusammensetzungsbewertung und der Lebensmittel-Bestandsmatrix, wobei die Darm-Lebensmittel-Bewertung die Fähigkeit der Lebensmittel quantifiziert, die Mehrzahl von nützlichen Mikroben zu erhöhen (508); und

Optimieren der Darm-Lebensmittel-Bewertung unter Verwendung einer Optimierungstechnik, um die optimierte Diät zu erhalten, wobei die Darm-Lebensmittel-Bewertung unter Berücksichtigung einer Mehrzahl von Einschränkungsparametern optimiert wird, die einen Kalorienparameter, einen Kohlenhydratparameter, einen Proteinparameter und einen Fettparameter umfassen (510).

6. Prozessorimplementiertes Verfahren nach Anspruch 5, wobei die Darm-Lebensmittel-Bewertung eines spezifischen Lebensmittels (Lebensmittel$_i$) wie folgt ausgedrückt wird:

$$gfs_i = \sum_{j=1}^{p} C_{j,i} \times gcs_j$$

*wobei,*

$C \in \mathbb{R}^{p \times m}$ die Lebensmittel-Zusammensetzungsmatrix ist, wobei $C_{j,i}$ der Gehalt an Zusammensetzung$_j$ pro Einheit von Lebensmittel$_i$ ist.

p = Gesamtzahl von Zusammensetzungen, die in Lebensmitteln aus der Liste von Lebensmitteln erhalten werden, und

m = Gesamtzahl von Lebensmitteln.

$$gcs_j = \sum_{k=1}^{q} T_{j,k} \times b_k \times I[taxa_k \in PU] - \sum_{k=1}^{q} T_{j,k} \times b_k \times I[taxa_k \in AP]$$

wobei
T die Taxa-Zusammensetzung-Nutzungsmatrix ist,
wobei

$$T \in \{0,1\}^{p \times q}; \quad T_{j,k} := \begin{cases} 1 \text{ wenn } taxa_j \text{ Verbindung}_k \text{ als Energiequelle nutzen kann} \\ 0 \text{ andernfalls} \end{cases}$$

q = Gesamtzahl von bakteriellen Taxa;

I die Indikatorfunktion ist, d.h. $I[y \in Z] := \begin{cases} 1 \text{ wenn } y \in Z \\ 0 \text{ ansonsten} \end{cases}$,

PU: Satz kommentionaler bakterieller Taxa,
AP: Satz pathogener bakterieller Taxa,

$$b_k = \max\big((1 + d_k \times I[taxa_k \in AP] - d_i \times I[taxa_k \in PU]), 1\big)$$

wobei,

$$d_k = \frac{[A_k^2 - A_k^1]}{\sigma_k},$$

wobei,

$A_k^2$ die Häufigkeit von taxa$_k$ at in der Probe T2 ist,

$A_k^1$ die Häufigkeit von taxa$_k$ in T1 ist,

$\sigma_k$ die Standardabweichung der taxa$_k$ in einer Mehrzahl von gesunden Darmmikrobiomproben ist.

7. Prozessorimplementiertes Verfahren nach Anspruch 1, wobei sich der Begriff "gestörter Pfad" auf eine Zunahme von schädlichen Pfaden oder Biosynthese von schädlichen Metaboliten oder Abbau von nützlichen Metaboliten oder eine Abnahme von nützlichen Pfaden oder nützlichen Metaboliten oder Abbau von schädlichen Metaboliten bezieht und der gestörte Pfad basierend auf statistischen Techniken identifiziert wird, die eines von einer parametrischen Technik, einer nicht parametrischen Technik, einem auf maschinellem Lernen basierenden Algorithmus beinhalten.

8. Prozessorimplementiertes Verfahren nach Anspruch 1, wobei die personalisierte therapeutische Intervention ferner das Verabreichen eines gentechnisch veränderten Bakteriums umfasst, wobei das gentechnisch veränderte Bakterium das gentechnische Verändern (a) einer Mehrzahl von mikrobiellen Pfaden zur Produktion von einem oder mehreren nützlichen Metaboliten oder/und (b) der Mehrzahl von mikrobiellen Pfaden zum Abbau/Eliminieren von einem oder mehreren schädlichen Metaboliten in die genetische Zusammensetzung von Darmmikroorganismen umfasst, wobei das gentechnisch veränderte Bakterium auf eine der unten aufgeführten Weisen verabreicht wird:

(a) gentechnisch verändert als Probiotika entweder allein oder in Kombination mit einem von einem Präbiotikum, einem Metabiotikum und einem Paraprobiotikum,
(b) gentechnisch veränderter Organismus, der einen oder mehrere heterologe Pfade ausdrückt, die erforderlich sind, um Metabolite zu produzieren, wobei der gentechnisch veränderte Organismus eines oder mehrere von Darmkommatika sein kann, wobei die Darmkommatika nützliche Mikroben, kommerzielle probiotische Stämme oder fakultative Anaerobe, die in der Lage sind, den Darm zu enthalten, beinhalten können, aber nicht darauf beschränkt sind; wobei der gentechnisch veränderte Organismus auch als modifiziertes Bakterium bezeichnet wird, wobei das modifizierte Bakterium als ein Lebensmittelprodukt, Lebensmittelergänzungsmittel oder eine pharmazeutische Zusammensetzung oder eine probiotische Formulierung verabreicht werden kann.

9. System (100), umfassend:

eine Eingabe-/Ausgabeschnittstelle (106);
einen oder mehrere Speicher (102); und
einen oder mehrere Hardwareprozessoren (104), wobei der eine oder die mehreren Speicher (104) mit dem einen oder den mehreren Hardwareprozessoren (104) gekoppelt sind, wobei der eine oder die mehreren Hardwareprozessoren (104) konfiguriert sind, um programmierte Anweisungen auszuführen, die in dem einen oder den mehreren Speichern (102) gespeichert sind, um:
einen Satz von Eingabedaten über einen oder mehrere Hardwareprozessoren zu empfangen, wobei der Satz von Eingabedaten Daten umfasst, die mit Folgendem assoziiert sind:

einer Mehrzahl von Metaboliten, einer Mehrzahl von Stoffwechselpfaden, einer Mehrzahl von taxonomischen Gruppen,
Informationen über Nährstoffe, Probiotika, Präbiotika, Ergänzungsmitteln zum Auffüllen oder Abbauen der Mehrzahl von Metaboliten,
einer Mehrzahl von Lebensmittelbestandteilen, einer Mehrzahl von Lebensmitteln, einer mikrobiellen Häufigkeitsmatrix für gesunde Kohorten und einer Mehrzahl von Diätoptimierungsvoraussetzungsparametern,

wobei die Mehrzahl von Metaboliten einen Satz von nützlichen Metaboliten und einen Satz von schädlichen Metaboliten umfasst, wobei der Satz von nützlichen Metaboliten für die Darmgesundheit eines Individuums nützlich ist und der Satz von schädlichen Metaboliten für die Darmgesundheit des Individuums schädlich ist,
wobei die Mehrzahl von Stoffwechselpfaden einen von (a) einem nützlichen Pfad und (b) schädlichen Pfaden umfasst,

wobei die nützlichen Pfade einen oder mehrere von einem Satz von Stoffwechselpfaden für die Biosynthese einer Mehrzahl von nützlichen Metaboliten und einem Satz von Stoffwechselpfaden für den Abbau der Mehrzahl von schädlichen Metaboliten umfassen, und

wobei die schädlichen Pfade einen oder mehrere von einem Satz von Stoffwechselpfaden für den Abbau einer Mehrzahl von nützlichen Metaboliten und einem Satz von Stoffwechselpfaden für die Biosynthese der Mehrzahl von schädlichen Metaboliten umfassen;

wobei die Mehrzahl von taxonomischen Gruppen eine Mehrzahl von Mikroben umfasst, wobei die Mehrzahl von Mikroben eine Mehrzahl von nützlichen Mikroben und eine Mehrzahl von schädlichen Mikroben umfasst, wobei die Mehrzahl von Mikroben gemäß einer taxonomischen Hierarchie klassifiziert ist, wobei die taxonomische Hierarchie eine oder mehrere von einer Mehrzahl von Bakterienstämmen, einer Mehrzahl von Bakterienklassen, einer Mehrzahl von Bakterienordnungen, einer Mehrzahl von Bakterienfamilien, einer Mehrzahl von Bakteriengattungen, einer Mehrzahl von Bakterienarten und einer Mehrzahl von Bakterienstämmen umfasst, wobei die Mehrzahl von Mikroben eine Mehrzahl von Genen mit einer eindeutigen genomischen Position, einen Satz von Nukleotid- und Proteinsequenzen der Bakterienstämme umfasst,

wobei die Mehrzahl von nützlichen Mikroben die nützlichen Pfade beherbergt und die Mehrzahl von schädlichen Mikroben die schädlichen Pfade beherbergt; und

wobei die Mehrzahl von Diätoptimierungsvoraussetzungsparametern einen Satz von Lebensmitteln, einen vollständigen Satz von Verbindungen, die in dem Satz von Lebensmitteln zu finden sind, eine Lebensmittelbestandteilmatrix umfasst, wobei die Lebensmittelbestandteilmatrix den Gehalt jeder Verbindung, die zu dem Satz von Verbindungen gehört, in jedem Lebensmittel, das zu dem Satz von Lebensmitteln gehört, den Gesamtkohlenhydrat-, Protein-, Fett- und Kaloriengehalt jedes der Lebensmittel aus dem Satz von Lebensmitteln, eine Liste von Bakterientaxa im menschlichen Darm, einen Satz von Funktionen zum Identifizieren eines Satzes von nützlichen Bakterientaxa und eines Satzes von schädlichen Bakterientaxa, eine Taxaverbindungsnutzungsmatrix, einen benutzerdefinierten Satz von obergebundenen Parameteruntergebundenen Parametern, die mit Kohlenhydrat, Protein, Fett und Kalorie assoziiert sind, und ein benutzerausgewähltes Lebensmittel tabelliert;

Erzeugen eines Satzes von Wissensdatenbanken unter Verwendung des Satzes von Eingabedaten über den einen oder die mehreren Hardwareprozessoren, wobei der Satz von Wissensdatenbanken eine Gattung-Funktions-Karte (FGmap), eine Genom-Metabolit-Pfad-Karte (GMP), ein Taxaassoziationsreferenzprofil-Graph (TAXA_NET), ein TAXA-Lebensmittel-Bestandteilnetzwerk, ein Lebensmittel-Bestandteilnetzwerk und eine ProbMap umfasst, wobei die Erzeugung des Satzes von Wissensdatenbanken umfasst:

Erzeugen der FGmap und der GMP-Karte, wobei die FGmap eine Matrix ist, die mit einer Funktion von der Mehrzahl von Bakteriengattungen, die zu einer der Mehrzahl von taxonomischen Gruppen gehören, und der Mehrzahl von metabolischen Pfaden als Spalten assoziiert ist, wobei ein erster vordefinierter Indikator eines von (a) einem Vorhandensein des metabolischen Pfads und (b) einem Fehlen des metabolischen Pfads anzeigt und die GMP eine Matrix der Mehrzahl von Bakterienstämmen als Reihen und der Mehrzahl von metabolischen Pfaden als Spalten ist, wobei ein zweiter vordefinierter Indikator eines von (a) einem Vorhandensein des metabolischen Pfads und (b) einem Fehlen des metabolischen Pfads anzeigt;

Erzeugen des TAXA_NET-Graphen basierend auf der Mehrzahl von taxonomischen Gruppen und der FGmap, wobei der TAXA_NET-Graph ein ungerichteter Graph ist, wobei jeder Knoten in dem TAXA_NET-Graph einer taxonomischen Gruppe aus der Mehrzahl von taxonomischen Gruppen entspricht und Kanten eine positive Beziehung zwischen einem Paar der taxonomischen Gruppe anzeigen, wobei jedes Taxa aus einer Mehrzahl von Knoten mit der Funktion der Mehrzahl von taxonomischen Gruppen aus der FGmap assoziiert ist, wobei die positive Beziehung eines von einem Mutualismus, einem Syntrophikum, einem Kommensalismus und einer Kooperation umfasst;

Erzeugen des TAXA-Lebensmittel-Bestandteilnetzwerks basierend auf einer Mehrzahl von Lebensmittelbestandteilen für jedes Taxa des TAXA_NET, wobei das TAXA-Lebensmittel-Bestandteilnetzwerk ein ungerichtetes Netzwerk jedes Taxas in Assoziation mit einem Lebensmittelbestandteil ist, der von dem Taxa als eine Quelle von Metabolismus oder Ernährung verwendet wird;

Erzeugen des Lebensmittel-Bestandteilnetzwerks basierend auf einer Mehrzahl von Lebensmitteln für jeden Lebensmittelbestandteil des TAXA-Lebensmittel-Bestandteilnetzwerks, wobei das Lebensmittel-Bestandteilnetzwerk ein ungerichtetes Netzwerk jedes Lebensmittelbestandteils ist, der in einem Lebensmittel vorhanden ist; und

Erzeugen einer Transponierung der GMP, um die ProbMap zu erhalten, wobei die ProbMap eine Matrix mit Zeilen ist, die jeden Metaboliten aus der Mehrzahl von Metaboliten und der Mehrzahl von metabolischen Pfaden und Spalten umfassen, die die Mehrzahl von Bakterienstämmen, Probiotika, Präbiotika, Diätkomponenten umfassen, die zur Biosynthese und/oder zum Abbau der entsprechenden Mehrzahl von Metaboliten in der Lage sind;

Empfangen einer Mehrzahl von Darmproben eines Individuums an Zwei-Zeit-Stempeln über den einen oder die mehreren Hardwareprozessoren, wobei die Zwei-Zeit-Stempel einen Zeitstempel 1 und einen Zeitstempel 2 umfassen, an denen die zwei Proben gesammelt wurden;

Erzeugen einer Taxa-Häufigkeitsmatrix für die Mehrzahl von Darmproben über den einen oder die mehreren Hardwareprozessoren, wobei die Taxa-Häufigkeitsmatrix einen Taxa-Satz 1 und einen Taxa-Satz 2 umfasst, wobei der Taxa-Satz 1 und der Taxa-Satz 2 unter Verwendung des Zeitstempels 1 und des Zeitstempels 2 erzeugt werden;

Erzeugen einer Pfadhäufigkeit für jede der Mehrzahl von Darmproben, die an dem Zeitstempel 1 und dem Zeitstempel 2 erhalten wurden, unter Verwendung der Taxa-Häufigkeitsmatrix und der FGmap über den einen oder die mehreren Hardwareprozessoren, wobei die Pfadhäufigkeit unter Verwendung einer Probenverarbeitungstechnik erzeugt wird, wobei die Pfadhäufigkeit für jeden Zeitstempel das Vorhandensein der Mehrzahl von metabolischen Pfaden des GMP in dem Darmmikrobiom des Individuums an dem entsprechenden Zeitstempel anzeigt;

Identifizieren eines gestörten Pfades, wobei der gestörte Pfad eine Verschlechterung der Darmgesundheit des Individuums anzeigt, wobei der gestörte Pfad basierend auf einem Vergleich der Pfadhäufigkeit der Mehrzahl von Darmproben, die an dem Zeitstempel 1 und dem Zeitstempel 2 erhalten wurden, unter Verwendung einer statistischen Technik identifiziert wird, wobei der gestörte Pfad basierend auf einem der folgenden Fälle identifiziert wird:

(a) Fall A: eine Abnahme der Pfadhäufigkeit des Satzes von nützlichen Pfaden , wobei die Abnahme eine Abnahme des Satzes von nützlichen Mikroben anzeigt, oder
(b) Fall B: eine Zunahme der Pfadhäufigkeit des Satzes von schädlichen Pfaden, wobei die Zunahme eine Zunahme des Satzes von schädlichen Mikroben anzeigt, oder
(c) Fall C: eine Abnahme der Pfadhäufigkeit des Satzes von nützlichen Pfaden und eine Zunahme der Pfadhäufigkeit des Satzes von schädlichen Pfaden, die eine Zunahme des Satzes von schädlichen Mikroben und eine Abnahme des Satzes von nützlichen Mikroben anzeigen;

Bestimmen, unter Verwendung der ProbMap, eines anfänglichen Satzes von personalisierten Therapeutika und einer Ernährungsempfehlung, wobei der anfängliche Satz von personalisierten Therapeutika und Ernährung mindestens eines umfasst von: einem personalisierten Therapeutikum und einem personalisierten Ernährungsplan, wobei der anfängliche Satz von personalisierten Therapeutika und Ernährungsempfehlung eine Reihe der ProbMap ist, die dem identifizierten gestörten Pfad entspricht, wobei das personalisierte Therapeutikum bestimmt wird als (a) die Mehrzahl von Bakterienstämmen, die den gestörten Pfad besitzen, oder (b) die Mehrzahl von Bakterienstämmen, die den Pfad für den Abbau oder/und die Biosynthese eines wahrscheinlichen Metaboliten besitzen, wobei der wahrscheinliche Metabolit ein Metabolit ist, der dem gestörten Pfad in der ProbMap entspricht;

Identifizieren eines Taxasatzes in der Taxahäufigkeitsmatrix, die an dem Zeitstempel 2 erhalten wurde, wobei der Taxasatz eine TAXA_SET_P und eine TAXA_SET_A umfasst, wobei die TAXA_SET_P einen vordefinierten ersten Wert für den einen oder die mehreren von dem nützlichen Pfad in einer von der FGmap und dem GMP umfasst, und die TAXA_SET_A einen vordefinierten zweiten Wert für den einen oder die mehreren von schädlichen Pfaden in einer von der FGmap und dem GMP umfasst;

Identifizieren eines Satzes von ersten Nachbarn für jedes der Taxa in der TAXA_SET_P an dem Zeitstempel 2 aus dem TAXA_NET-Graphen, wobei der Satz von ersten Nachbarn unmittelbare Nachbarn der TAXA_SET_P sind, umfassend eine TAXA_SET_FN, eine TAXA_SET_FN_COM, eine TAXA_SET_INTERSECT, eine TAXA_SET_NEW, wobei der identifizierte Satz von ersten Nachbarn der TAXA_SET_P in dem TAXA_NET-Graphen in der neuen Liste TAXA_SET_FN gespeichert wird, gefolgt von dem Vorhersagen des Funktionspotentials der Taxa in der TAXA_SET_FN und dem Identifizieren derjenigen, die kommenal sind, basierend auf ihrer Butyratproduktionsfähigkeit und dem Speichern der Teilmenge dieser Taxa aus der TAXA_SET_FN als die TAXA_SET_FN_COM, und

Identifizieren eines Satzes von Taxa, die zwischen der TAXA_SET_FN_COM und einem Satz aller Taxa, die in dem Zeitstempel 2 vorhanden sind, gemeinsam sind, und Speichern des identifizierten Satzes von Taxa als die TAXA_SET_INTERSECT und Speichern der verbleibenden Taxa in der TAXA_SET_FN_COM, die nicht in dem Zeitstempel 2 vorhanden ist, als die TAXA_SET_NEW; und

Empfehlen einer personalisierten therapeutischen Intervention zum Verbessern der Darmgesundheit des Individuums über den einen oder die mehreren Hardwareprozessoren, wobei die therapeutische Intervention das Empfehlen von einem von (a) einem präbiotischen Cocktail, (b) einem probiotischen Cocktail, (c) optimierter Ernährung basierend auf den identifizierten ersten Nachbarn unter Verwendung des Satzes von Wissensdatenbanken und des anfänglichen Satzes von personalisierten Therapeutika und Ernährungsempfehlung umfasst, wobei die personalisierte therapeutische Intervention mindestens eines oder mehrere umfasst von:

(a) für Fall A (eine Abnahme der Pfad-Häufigkeit des Satzes von nützlichen Mikroben) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt und das Wachstum der nützlichen Mikroben unterstützt,

(b) für Fall B (eine Zunahme der Pfad-Häufigkeit des Satzes von schädlichen Mikroben) Empfehlen einer optimierten Ernährung, wobei die optimierte Ernährung das Wachstum der Mehrzahl von nützlichen Mikroben fördert und das Wachstum von schädlichen Mikroben hemmt, und

(c) für Fall C (eine Abnahme der Pfad-Häufigkeit des Satzes von nützlichen Mikroben und eine Zunahme der Pfad-Häufigkeit des Satzes von schädlichen Mikroben ) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt, das Wachstum von nützlichen Mikroben unterstützt und das Wachstum von schädlichen Mikroben hemmt.

10. System nach Anspruch 9, wobei der eine oder die mehreren Hardwareprozessoren durch die Anweisungen konfiguriert sind, um die Erzeugung der GMP und FGmap zu implementieren, umfassend:

Erzeugen einer Matrix-Metaboliten-Map (Mmap) basierend auf der Mehrzahl von Metaboliten, der Mehrzahl von metabolischen Pfaden und einer Liste von Organismen, die mit jedem der Mehrzahl von metabolischen Pfaden assoziiert sind, wobei die Mmap für jeden der Mehrzahl von Metaboliten erzeugt wird;

Erzeugen einer bakteriellen Genomkarte (Bacterial Genome Map, BGM) unter Verwendung einer bakteriellen Genomdatenbank (BGD), wobei die BGD aus einer erschöpfenden Liste die Mehrzahl von Bakterienstämmen umfasst und die BGM Informationen umfasst, die mit der Mehrzahl von Bakterienstämmen assoziiert sind, einschließlich einer Mehrzahl von Namen der Mehrzahl von Bakterienstämmen, einer Mehrzahl von Identifikationsnummern der Mehrzahl von Bakterienstämmen, einer Mehrzahl von Geneorten der Mehrzahl von Bakterienstämmen und einer Mehrzahl von Proteindomänen der Mehrzahl von Bakterienstämmen,

Erzeugen einer Metaboliten-Pfad-Domänen-Karte (Metabolite Pathway-Domain Map, MPDM) für jeden Metaboliten aus der Mehrzahl von Metaboliten basierend auf der Mmap und der BGM, wobei die MPDM aus der erschöpfenden Liste von Proteindomänen besteht, die mit jedem der Mehrzahl von metabolischen Pfaden assoziiert sind;

Identifizieren des Vorhandenseins einer Mehrzahl von metabolischen Pfaden in der Liste von Bakterienstämmen und Genomen aus der unter Verwendung der MPDM und der BGM basierend auf einem ersten vordefinierten Schwellenwertkriterium;

Erzeugen der GMP unter Verwendung der BGM, der MPDM, der Mehrzahl von metabolischen Pfaden und der Mehrzahl von Metaboliten, wobei die GMP eine Matrix mit einem Satz von bakteriellen Genomen (Bakterienstämmen) als eine Mehrzahl von Reihen und die Liste der Mehrzahl von metabolischen Pfaden, die der Bildung jedes Metaboliten entsprechen, als eine Mehrzahl von Spalten ist; und

Erzeugen der FGmap unter Verwendung der GMP, wobei FGmap eine Matrix als die Mehrzahl von Bakteriengattungen als eine Mehrzahl von Reihen und die Mehrzahl von metabolischen Pfaden als Spalten ist.

11. System nach Anspruch 9, wobei der eine oder die mehreren Hardwareprozessoren durch die Anweisungen konfiguriert sind, um die Probenverarbeitungstechnik (400) zum Erzeugen der Pfad-Häufigkeit-Matrizen zu implementieren, umfassend:

Extrahieren einer Mehrzahl von Nukleinsäuren aus der Mehrzahl von Darmproben basierend auf einer Nukleinsäureextraktionstechnik;

Erzeugen einer Mehrzahl von Nukleotidsequenzen aus der extrahierten Mehrzahl von Nukleinsäuren unter Verwendung eines Sequenzierers;

Erhalten eines Satzes von bakterieller taxonomischer Häufigkeit-Matrix auf jeder taxonomischen Hierarchieebene unter Verwendung der Mehrzahl von Nukleotidsequenzen, wobei die bakterielle taxonomische Häufigkeit-Matrix durch Klassifizieren der Mehrzahl von Nukleotidsequenzen basierend auf einer vordefinierten taxonomischen Ebene unter Verwendung eines Klassifizierungsalgorithmus erhalten wird; und
Erzeugen einer Pfad-Häufigkeit unter Verwendung des Satzes von bakterieller taxonomischer Häufigkeit-Matrix, der GMP und der FGmap.

12. System nach Anspruch 9, wobei der eine oder die mehreren Hardwareprozessoren durch die Anweisungen konfiguriert sind, um den Prozess des Empfehlens der optimierten Diät zu implementieren, umfassend:

Berechnen einer normalisierten Änderung ($d_k$) für jedes Taxa in der Vereinigung des Taxa-Satzes 1 und des Taxa-Satzes 2 zwischen dem Zeitstempel 1 und dem Zeitstempel 2;
Berechnen einer Wichtigkeitsbewertung ($b_k$) für jedes Taxa in der Vereinigung des Taxa-Satzes 1 und des Taxa-Satzes 2 unter Verwendung der normalisierten Änderung, der Mehrzahl von nützlichen Mikroben, der Mehrzahl von schädlichen Mikroben aus dem Satz von Wissensdatenbanken;
Berechnen einer personalisierten Darmzusammensetzungsbewertung ($gcs_j$) für ein Individuum unter Verwendung der Wichtigkeitsbewertung ($b_k$) für jedes Taxa in der Vereinigung des Taxa-Satzes 1 und des Taxa-Satzes 2 und einer vordefinierten Taxa-Zusammensetzung-Nutzungsmatrix, wobei die personalisierte Darmzusammensetzungsbewertung die Fähigkeit der Zusammensetzung quantifiziert, die Mehrzahl von nützlichen Mikroben im Darm zu erhöhen;
Berechnen einer Darm-Lebensmittel-Bewertung ($gfs_j$) für alle Lebensmittel in dem Satz von Lebensmitteln unter Verwendung der Darmzusammensetzungsbewertung und der Lebensmittel-Bestandsmatrix, wobei die Darm-Lebensmittel-Bewertung die Fähigkeit der Lebensmittel quantifiziert, die Mehrzahl von nützlichen Mikroben zu erhöhen; und
Optimieren der Darm-Lebensmittel-Bewertung unter Verwendung einer Optimierungstechnik, um die optimierte Diät zu erhalten, wobei die Darm-Lebensmittel-Bewertung unter Berücksichtigung einer Mehrzahl von Einschränkungsparametern optimiert wird, die einen Kalorienparameter, einen Kohlenhydratparameter, einen Proteinparameter und einen Fettparameter umfassen.

13. System nach Anspruch 12, wobei der eine oder die mehreren Hardwareprozessoren durch die Anweisungen konfiguriert sind, um die Berechnung der Darm-Lebensmittel-Bewertung eines spezifischen Lebensmittels (Lebensmittel$_i$) zu implementieren, die wie folgt ausgedrückt wird:

$$gfs_i = \sum_{j=1}^{p} C_{j,i} \times gcs_j$$

*wobei,*

$\mathbf{C} \in \mathbb{R}^{p \times m}$ die Lebensmittel-Zusammensetzungsmatrix ist, wobei $\mathbf{C_{j,i}}$ der Gehalt an Zusammensetzung$_j$ pro Einheit von Lebensmittel$_i$ ist.
p = Gesamtzahl von Zusammensetzungen, die in Lebensmitteln aus der Liste von Lebensmitteln erhalten werden, und
m = Gesamtzahl von Lebensmitteln.

$$gcs_j = \sum_{k=1}^{q} T_{j,k} \times b_k \times I[taxa_k \in PU] - \sum_{k=1}^{q} T_{j,k} \times b_k \times I[taxa_k \in AP]$$

wobei
T die Taxa-Zusammensetzung-Nutzungsmatrix ist,
wobei

$$T \in \{0,1\}^{p \times q}; \ T_{j,k}: = \begin{cases} 1 \text{ wennf taxa}_j \text{ Verbindung}_k \text{ als Energiequelle nutzen kann} \\ 0 \text{ ansonsten} \end{cases}$$

q = Gesamtzahl von bakteriellen Taxa;

I die Indikatorfunktion ist, d.h. $I[y \in Z] := \left\{ \begin{array}{l} 1 \text{ if } y \in Z \\ 0 \text{ ansonsten} \end{array} \right\}$,

PU: Satz kommentionaler bakterieller Taxa,
AP: Satz pathogener bakterieller Taxa,

$$b_k = \max\big((1 + d_k \times I[taxa_k \in AP] - d_i \times I[taxa_k \in PU]), 1\big)$$

Wobei,

$$d_k = \frac{[A_k^2 - A_k^1]}{\sigma_k},$$

Wobei,

$A_k^2$ die Häufigkeit von Taxa$_k$ an in der Probe T2 ist,

$A_k^1$ die Häufigkeit von Taxa$_k$ in T1 ist,

$\sigma_k$ die Standardabweichung der Taxa$_k$ in einer Mehrzahl von gesunden Darmmikrobiomproben ist.

14. System nach Anspruch 9, wobei der eine oder die mehreren Hardwareprozessoren durch die Anweisungen konfiguriert sind, um die personalisierte therapeutische Intervention ferner das Verabreichen eines gentechnisch veränderten Bakteriums umfasst, wobei das gentechnisch veränderte Bakterium das gentechnische Verändern (a) einer Mehrzahl von mikrobiellen Pfaden zur Produktion von einem oder mehreren nützlichen Metaboliten oder/und (b) der Mehrzahl von mikrobiellen Pfaden zum Abbau/Eliminieren von einem oder mehreren schädlichen Metaboliten in die genetische Zusammensetzung von Darmmikroorganismen umfasst, wobei das gentechnisch veränderte Bakterium auf eine der unten genannten Weisen verabreicht wird:

(a) gentechnisch verändert als Probiotika entweder allein oder in Kombination mit einem von einem Präbiotikum, einem Metabiotikum und einem Paraprobiotikum,
(b) gentechnisch veränderter Organismus, der einen oder mehrere heterologe Pfade ausdrückt, die erforderlich sind, um Metabolite zu produzieren, wobei der gentechnisch veränderte Organismus eines oder mehrere von Darmkommatika sein kann, wobei die Darmkommatika nützliche Mikroben, kommerzielle probiotische Stämme oder fakultative Anaerobe, die in der Lage sind, den Darm zu enthalten, beinhalten können, aber nicht darauf beschränkt sind; wobei der gentechnisch veränderte Organismus auch als modifiziertes Bakterium bezeichnet wird, wobei das modifizierte Bakterium als ein Lebensmittelprodukt, Lebensmittelergänzungsmittel oder eine pharmazeutische Zusammensetzung oder eine probiotische Formulierung verabreicht werden kann.

15. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, bewirken:
Empfangen eines Satzes von Eingabedaten über einen oder mehrere Hardwareprozessoren, wobei der Satz von Eingabedaten Daten umfasst, die mit Folgendem assoziiert sind:

einer Mehrzahl von Metaboliten, einer Mehrzahl von Stoffwechselpfaden, einer Mehrzahl von taxonomischen Gruppen,
Informationen über Nährstoffe, Probiotika, Präbiotika, Ergänzungsmitteln zum Auffüllen oder Abbauen der Mehrzahl von Metaboliten,
einer Mehrzahl von Lebensmittelbestandteilen, einer Mehrzahl von Lebensmitteln, einer mikrobiellen Häufigkeitsmatrix für gesunde Kohorten und einer Mehrzahl von Diätoptimierungsvoraussetzungsparametern,

wobei die Mehrzahl von Metaboliten einen Satz von nützlichen Metaboliten und einen Satz von schädlichen Metaboliten umfasst, wobei der Satz von nützlichen Metaboliten für die Darmgesundheit eines Individuums nützlich ist und der Satz von schädlichen Metaboliten für die Darmgesundheit des Individuums schädlich ist, wobei die Mehrzahl von Stoffwechselpfaden einen von (a) einem nützlichen Pfad und (b) schädlichen Pfaden umfasst,

wobei die nützlichen Pfade einen oder mehrere von einem Satz von Stoffwechselpfaden für die Biosynthese einer Mehrzahl von nützlichen Metaboliten und einem Satz von Stoffwechselpfaden für den Abbau der Mehrzahl von schädlichen Metaboliten umfassen, und

wobei die schädlichen Pfade einen oder mehrere von einem Satz von Stoffwechselpfaden für den Abbau einer Mehrzahl von nützlichen Metaboliten und einem Satz von Stoffwechselpfaden für die Biosynthese der Mehrzahl von schädlichen Metaboliten umfassen;

wobei die Mehrzahl von taxonomischen Gruppen eine Mehrzahl von Mikroben umfasst, wobei die Mehrzahl von Mikroben eine Mehrzahl von nützlichen Mikroben und eine Mehrzahl von schädlichen Mikroben umfasst, wobei die Mehrzahl von Mikroben gemäß einer taxonomischen Hierarchie klassifiziert ist, wobei die taxonomische Hierarchie eine oder mehrere von einer Mehrzahl von Bakterienstämmen, einer Mehrzahl von Bakterienklassen, einer Mehrzahl von Bakterienordnungen, einer Mehrzahl von Bakterienfamilien, einer Mehrzahl von Bakteriengattungen, einer Mehrzahl von Bakterienarten und einer Mehrzahl von Bakterienstämmen umfasst, wobei die Mehrzahl von Mikroben eine Mehrzahl von Genen mit einer eindeutigen genomischen Position, einen Satz von Nukleotid- und Proteinsequenzen der Bakterienstämme umfasst, wobei die Mehrzahl von nützlichen Mikroben die nützlichen Pfade beherbergt und die Mehrzahl von schädlichen Mikroben die schädlichen Pfade beherbergt; und

wobei die Mehrzahl von Diätoptimierungsvoraussetzungsparametern einen Satz von Lebensmitteln, einen vollständigen Satz von Verbindungen, die in dem Satz von Lebensmitteln zu finden sind, eine Lebensmittelbestandteilmatrix umfasst, wobei die Lebensmittelbestandteilmatrix den Gehalt jeder Verbindung, die zu dem Satz von Verbindungen gehört, in jedem Lebensmittel, das zu dem Satz von Lebensmitteln gehört, den Gesamtkohlenhydrat-, Protein-, Fett- und Kaloriengehalt jedes der Lebensmittel aus dem Satz von Lebensmitteln, eine Liste von Bakterientaxa im menschlichen Darm, einen Satz von Funktionen zum Identifizieren eines Satzes von nützlichen Bakterientaxa und eines Satzes von schädlichen Bakterientaxa, eine Taxaverbindungsnutzungsmatrix, einen benutzerdefinierten Satz von obergebundenen Parameternuntergebundenen Parametern, die mit Kohlenhydrat, Protein, Fett und Kalorie assoziiert sind, und ein benutzerausgewähltes Lebensmittel tabelliert;

Erzeugen eines Satzes von Wissensdatenbanken unter Verwendung des Satzes von Eingabedaten über den einen oder die mehreren Hardwareprozessoren, wobei der Satz von Wissensdatenbanken eine Gattung-Funktions-Karte (FGmap), eine Genom-Metabolit-Pfad-Karte (GMP), ein Taxaassoziationsreferenzprofil-Graph (TAXA_NET), ein TAXA-Lebensmittel-Bestandteilnetzwerk, ein Lebensmittel-Bestandteilnetzwerk und eine ProbMap umfasst, wobei die Erzeugung des Satzes von Wissensdatenbanken umfasst:

Erzeugen der FGmap und der GMP-Karte, wobei die FGmap eine Matrix ist, die mit einer Funktion von der Mehrzahl von Bakteriengattungen, die zu einer der Mehrzahl von taxonomischen Gruppen gehören, und der Mehrzahl von metabolischen Pfaden als Spalten assoziiert ist, wobei ein erster vordefinierter Indikator eines von (a) einem Vorhandensein des metabolischen Pfads und (b) einem Fehlen des metabolischen Pfads anzeigt und die GMP eine Matrix der Mehrzahl von Bakterienstämmen als Reihen und der Mehrzahl von metabolischen Pfaden als Spalten ist, wobei ein zweiter vordefinierter Indikator eines von (a) einem Vorhandensein des metabolischen Pfads und (b) einem Fehlen des metabolischen Pfads anzeigt;

Erzeugen des TAXA_NET-Graphen basierend auf der Mehrzahl von taxonomischen Gruppen und der FGmap, wobei der TAXA_NET-Graph ein ungerichteter Graph ist, wobei jeder Knoten in dem TAXA_NET-Graph einer taxonomischen Gruppe aus der Mehrzahl von taxonomischen Gruppen entspricht und Kanten eine positive Beziehung zwischen einem Paar der taxonomischen Gruppe anzeigen, wobei jedes Taxa aus einer Mehrzahl von Knoten mit der Funktion der Mehrzahl von taxonomischen Gruppen aus der FGmap assoziiert ist, wobei die positive Beziehung eines von einem Mutualismus, einem Syntrophikum, einem Kommensalismus und einer Kooperation umfasst;

Erzeugen des TAXA-Lebensmittel-Bestandteilnetzwerks basierend auf einer Mehrzahl von Lebensmittelbestandteilen für jedes Taxa des TAXA_NET, wobei das TAXA-Lebensmittel-Bestandteilnetzwerk ein ungerichtetes Netzwerk jedes Taxas in Assoziation mit einem Lebensmittelbestandteil ist, der von dem Taxa als eine Quelle von Metabolismus oder Ernährung verwendet wird;

Erzeugen des Lebensmittel-Bestandteilnetzwerks basierend auf einer Mehrzahl von Lebensmitteln für jeden Lebensmittelbestandteil des TAXA-Lebensmittel-Bestandteilnetzwerks, wobei das Lebensmittel-Bestandteilnetzwerk ein ungerichtetes Netzwerk jedes Lebensmittelbestandteils ist, der in einem Lebensmittel vorhanden ist; und

Erzeugen einer Transponierung der GMP, um die ProbMap zu erhalten, wobei die ProbMap eine Matrix mit Zeilen ist, die jeden Metaboliten aus der Mehrzahl von Metaboliten und der Mehrzahl von metabolischen Pfaden und Spalten umfassen, die die Mehrzahl von Bakterienstämmen, Probiotika, Präbiotika, Diätkomponenten umfassen, die zur Biosynthese und/oder zum Abbau der entsprechenden Mehrzahl von Metaboliten in der Lage sind;

Empfangen einer Mehrzahl von Darmproben eines Individuums an Zwei-Zeit-Stempeln über den einen oder die mehreren Hardwareprozessoren, wobei die Zwei-Zeit-Stempel einen Zeitstempel 1 und einen Zeitstempel 2 umfassen, an denen die zwei Proben gesammelt wurden;

Erzeugen einer Taxa-Häufigkeitsmatrix für die Mehrzahl von Darmproben über den einen oder die mehreren Hardwareprozessoren, wobei die Taxa-Häufigkeitsmatrix einen Taxa-Satz 1 und einen Taxa-Satz 2 umfasst, wobei der Taxa-Satz 1 und der Taxa-Satz 2 unter Verwendung des Zeitstempels 1 und des Zeitstempels 2 erzeugt werden;

Erzeugen einer Pfadhäufigkeit für jede der Mehrzahl von Darmproben, die an dem Zeitstempel 1 und dem Zeitstempel 2 erhalten wurden, unter Verwendung der Taxa-Häufigkeitsmatrix und der FGmap über den einen oder die mehreren Hardwareprozessoren, wobei die Pfadhäufigkeit unter Verwendung einer Probenverarbeitungstechnik erzeugt wird, wobei die Pfadhäufigkeit für jeden Zeitstempel das Vorhandensein der Mehrzahl von metabolischen Pfaden des GMP in dem Darmmikrobiom des Individuums an dem entsprechenden Zeitstempel anzeigt;

Identifizieren eines gestörten Pfades, wobei der gestörte Pfad eine Verschlechterung der Darmgesundheit des Individuums anzeigt, wobei der gestörte Pfad basierend auf einem Vergleich der Pfadhäufigkeit der Mehrzahl von Darmproben, die an dem Zeitstempel 1 und dem Zeitstempel 2 erhalten wurden, unter Verwendung einer statistischen Technik identifiziert wird, wobei der gestörte Pfad basierend auf einem der folgenden Fälle identifiziert wird:

(a) Fall A: eine Abnahme der Pfadhäufigkeit des Satzes von nützlichen Pfaden , wobei die Abnahme eine Abnahme des Satzes von nützlichen Mikroben anzeigt, oder
(b) Fall B: eine Zunahme der Pfadhäufigkeit des Satzes von schädlichen Pfaden, wobei die Zunahme eine Zunahme des Satzes von schädlichen Mikroben anzeigt, oder
(c) Fall C: eine Abnahme der Pfadhäufigkeit des Satzes von nützlichen Pfaden und eine Zunahme der Pfadhäufigkeit des Satzes von schädlichen Pfaden, die eine Zunahme des Satzes von schädlichen Mikroben und eine Abnahme des Satzes von nützlichen Mikroben anzeigen;

Bestimmen, unter Verwendung der ProbMap, eines anfänglichen Satzes von personalisierten Therapeutika und einer Ernährungsempfehlung, wobei der anfängliche Satz von personalisierten Therapeutika und Ernährung mindestens eines umfasst von: einem personalisierten Therapeutikum und einem personalisierten Ernährungsplan, wobei der anfängliche Satz von personalisierten Therapeutika und Ernährungsempfehlung eine Reihe der ProbMap ist, die dem identifizierten gestörten Pfad entspricht, wobei das personalisierte Therapeutikum bestimmt wird als (a) die Mehrzahl von Bakterienstämmen, die den gestörten Pfad besitzen, oder (b) die Mehrzahl von Bakterienstämmen, die den Pfad für den Abbau oder/und die Biosynthese eines wahrscheinlichen Metaboliten besitzen, wobei der wahrscheinliche Metabolit ein Metabolit ist, der dem gestörten Pfad in der ProbMap entspricht;

Identifizieren eines Taxasatzes in der Taxahäufigkeitsmatrix, die an dem Zeitstempel 2 erhalten wurde, wobei der Taxasatz eine TAXA_SET_P und eine TAXA_SET_A umfasst, wobei die TAXA_SET_P einen vordefinierten ersten Wert für den einen oder die mehreren von dem nützlichen Pfad in einer von der FGmap und dem GMP umfasst, und die TAXA_SET_A einen vordefinierten zweiten Wert für den einen oder die mehreren von schädlichen Pfaden in einer von der FGmap und dem GMP umfasst;

Identifizieren eines Satzes von ersten Nachbarn für jedes der Taxa in der TAXA_SET_P an dem Zeitstempel 2 aus dem TAXA_NET-Graphen, wobei der Satz von ersten Nachbarn unmittelbare Nachbarn der TAXA_SET_P sind, umfassend eine TAXA_SET_FN, eine TAXA_SET_FN_COM, eine TAXA_SET_INTERSECT, eine TAXA_SET_NEW, wobei der identifizierte Satz von ersten Nachbarn der TAXA_SET_P in dem TAXA_NET-Graphen in der neuen Liste TAXA_SET_FN gespeichert wird, gefolgt von dem Vorhersagen des Funktionspotentials der Taxa in der TAXA_SET_FN und dem Identifizieren derjenigen, die kommenal sind, basierend auf ihrer Butyratproduktionsfähigkeit und dem Speichern der Teilmenge dieser Taxa aus der TAXA_SET_FN als die TAXA_SET_FN_COM, und

Identifizieren eines Satzes von Taxa, die zwischen der TAXA_SET_FN_COM und einem Satz aller Taxa, die in dem Zeitstempel 2 vorhanden sind, gemeinsam sind, und Speichern des identifizierten Satzes von Taxa als die TAXA_SET_INTERSECT und Speichern der verbleibenden Taxa in der TAXA_SET_FN_COM, die nicht in dem Zeitstempel 2 vorhanden ist, als die TAXA_SET_NEW; und

Empfehlen einer personalisierten therapeutischen Intervention zum Verbessern der Darmgesundheit des Individuums über den einen oder die mehreren Hardwareprozessoren, wobei die therapeutische Intervention das Empfehlen von einem von (a) einem präbiotischen Cocktail, (b) einem probiotischen Cocktail, (c) optimierter Ernährung basierend auf den identifizierten ersten Nachbarn unter Verwendung des Satzes von Wissensdatenbanken und des anfänglichen Satzes von personalisierten Therapeutika und Ernährungsempfehlung umfasst, wobei die personalisierte therapeutische Intervention mindestens eines oder mehrere umfasst von:

(a)für Fall A (eine Abnahme der Pfad-Häufigkeit des Satzes von nützlichen Mikroben) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt und das Wachstum der nützlichen Mikroben unterstützt,
(b)für Fall B (eine Zunahme der Pfad-Häufigkeit des Satzes von schädlichen Mikroben) Empfehlen einer optimierten Ernährung, wobei die optimierte Ernährung das Wachstum der Mehrzahl von nützlichen Mikroben fördert und das Wachstum von schädlichen Mikroben hemmt, und
(c)für Fall C (eine Abnahme der Pfad-Häufigkeit des Satzes von nützlichen Mikroben und eine Zunahme der Pfad-Häufigkeit des Satzes von schädlichen Mikroben) Empfehlen eines präbiotischen Cocktails, eines probiotischen Cocktails und einer optimierten Ernährung, wobei der präbiotische Cocktail, der probiotische Cocktail und die optimierte Ernährung die Mehrzahl von nützlichen Mikroben auffüllt, das Wachstum von nützlichen Mikroben unterstützt und das Wachstum von schädlichen Mikroben hemmt.

## Revendications

1. Procédé mis en œuvre par processeur (200) pour concevoir des produits thérapeutiques personnalisés et un régime alimentaire sur la base de fonctions de microbiome comprenant :
la réception d'un ensemble de données d'entrée (202), via un ou plusieurs processeurs matériels, dans lequel l'ensemble de données d'entrée comprend des données associées à

une pluralité de métabolites, une pluralité de voies métaboliques, une pluralité de groupes taxonomiques, des informations sur des nutriments, des probiotiques, des prébiotiques, des compléments pour la régénération ou la dégradation de la pluralité de métabolites, une pluralité de constituants alimentaires, une pluralité d'articles alimentaires, une matrice d'abondance microbienne pour des cohortes saines et une pluralité de paramètres préalables d'optimisation de régime alimentaire,

dans lequel la pluralité de métabolites comprend un ensemble de métabolites bénéfiques et un ensemble de métabolites nocifs, dans lequel l'ensemble de métabolites bénéfiques est bénéfique pour la santé intestinale d'un individu et l'ensemble de métabolites nocifs est nocif pour la santé intestinale de l'individu,
dans lequel la pluralité de voies métaboliques comprend l'une parmi (a) une voie bénéfique et (b) des voies nocives,

dans lequel les voies bénéfiques comprennent une ou plusieurs parmi un ensemble de voies métaboliques pour la biosynthèse de la pluralité de métabolites bénéfiques et un ensemble de voies métaboliques pour la dégradation de la pluralité de métabolites nocifs, et
dans lequel les voies nocives comprennent une ou plusieurs parmi un ensemble de voies métaboliques pour la dégradation de la pluralité de métabolites bénéfiques et un ensemble de voies métaboliques pour la biosynthèse de la pluralité de métabolites nocifs ;

dans lequel la pluralité de groupes taxonomiques comprend une pluralité de microbes, où la pluralité de microbes comprend une pluralité de microbes bénéfiques et une pluralité de microbes nocifs, où la pluralité de microbes sont classés selon une hiérarchie taxonomique, dans lequel la hiérarchie taxonomique comprend un ou plusieurs parmi une pluralité de phylères bactériennes, une pluralité de classes bactériennes, une pluralité d'ordres bactériens, une pluralité de familles bactériennes, une pluralité de genres bactériens, une pluralité d'espèces bactériennes et une pluralité de souches bactériennes, où la pluralité de microbes comprend parmi une pluralité de gènes ayant une localisation génomique distincte, un ensemble de séquences nucléotidiques et protéiques des souches bactériennes,
dans lequel la pluralité de microbes bénéfiques abrite les voies bénéfiques et la pluralité de microbes nocifs abrite les voies nocives ; et
dans lequel la pluralité de paramètres préalables d'optimisation de régime alimentaire comprend un ensemble d'articles alimentaires, un ensemble complet de composés qui sont trouvés dans l'ensemble d'articles alimentaires, une matrice de constituants alimentaires, dans lequel la matrice de constituants alimentaires tabule la teneur de chaque composé appartenant à l'ensemble de composés dans chaque article alimentaire appartenant à l'ensemble d'aliments, la teneur totale en glucides, protéines, graisses et calories de chacun des articles alimentaires de l'ensemble d'articles alimentaires, une liste de taxa bactériens dans l'intestin humain, un ensemble de fonctions pour identifier un ensemble de taxa bactériens bénéfiques et un ensemble de taxa bactériens nocifs, une matrice d'utilisation de composés de taxa, un ensemble défini par l'utilisateur de paramètres liés supérieurs - paramètres liés inférieurs associés à des glucides, protéines, graisses et calories, et des articles alimentaires sélectionnés par l'utilisateur ;

la génération d'un ensemble de bases de connaissances en utilisant l'ensemble de données d'entrée (204), via les un ou plusieurs processeurs matériels, dans lequel l'ensemble de bases de connaissances comprend une carte de fonction de genre (FGmap), une carte de voie métabolique génomique (GMP), un graphique de profil de référence d'association de taxa (TAXA_NET), un réseau de constituants alimentaires TAXA, un réseau de constituants alimentaires et une ProbMap, dans lequel la génération de l'ensemble de bases de connaissances comprend :

la génération de la FGmap et de la carte GMP, dans lequel la FGmap est une matrice associée à une fonction de la pluralité de genres bactériens appartenant à l'un de la pluralité de groupes taxonomiques et de la pluralité de voies métaboliques sous forme de colonnes, dans lequel un premier indicateur prédéfini indique l'une de (a) une présence de la voie métabolique et (b) une absence de la voie métabolique et la GMP est une matrice de la pluralité de souches bactériennes sous forme de rangées et de la pluralité de voies métaboliques sous forme de colonnes, dans lequel un second indicateur prédéfini indique l'une de (a) une présence de la voie métabolique et (b) une absence de la voie métabolique (204A) ;

la génération du graphique TAXA_NET sur la base de la pluralité de groupes taxonomiques et de la FGmap, dans lequel le graphique TAXA_NET est un graphique non dirigé, dans lequel chaque noeud dans le graphique TAXA_NET correspond à un groupe taxonomique de la pluralité de groupes taxonomiques et les bords indiquent une relation positive entre une paire du groupe taxonomique dans lequel chaque taxon d'une pluralité de noeuds est associé à la fonction de la pluralité de groupes taxonomiques de la FGmap, dans lequel la relation positive comprend l'un d'un mutualisme, d'un syntrophisme, d'un commensalisme et d'une coopération (204B) ;

la génération du réseau de constituants alimentaires TAXA sur la base d'une pluralité de constituants alimentaires pour chaque taxon du TAXA_NET, dans lequel le réseau de constituants alimentaires TAXA est un réseau non dirigé de chaque taxon en association avec un constituant alimentaire utilisé par le taxon comme source de métabolisme ou de nutrition (204C) ;

la génération du réseau de constituants alimentaires sur la base d'une pluralité d'articles alimentaires pour chaque constituant alimentaire du réseau de constituants alimentaires TAXA, dans lequel le réseau de constituants alimentaires est un réseau non dirigé de chaque constituant alimentaire présent dans un article alimentaire (204D) ; et

la génération d'une transposition de la GMP pour obtenir la ProbMap, dans lequel la ProbMap est une matrice avec des rangées comprenant chaque métabolite de la pluralité de métabolites et la pluralité de voies métaboliques et des colonnes comprenant la pluralité de souches bactériennes, de probiotiques, de prébiotiques, de composants de régime alimentaire capables de biosynthèse et/ou de dégradation de la pluralité correspondante de métabolites (204E) ;

la réception d'une pluralité d'échantillons intestinaux d'un individu à deux horodatages, via les un ou plusieurs processeurs matériels, dans lequel les deux horodatages comprennent un horodatage 1 et un horodatage 2 auxquels les deux échantillons ont été collectés (206) ;

la génération d'une matrice d'abondance de taxa pour la pluralité d'échantillons intestinaux, via les un ou plusieurs processeurs matériels, dans lequel la matrice d'abondance de taxa comprend un ensemble de taxa 1 et un ensemble de taxa 2, où l'ensemble de taxa 1 et l'ensemble de taxa 2 sont générés en utilisant l'horodatage 1 et l'horodatage 2 (208) ;

la génération d'une abondance de voie pour chacun de la pluralité d'échantillons intestinaux obtenus à l'horodatage 1 et à l'horodatage 2 en utilisant la matrice d'abondance de taxa et la FGmap, via les un ou plusieurs processeurs matériels, dans lequel l'abondance de voie est générée en utilisant une technique de traitement d'échantillon, dans lequel l'abondance de voie pour chaque horodatage est indicative de la présence de la pluralité de voies métaboliques de la GMP dans le microbiome intestinal de l'individu à l'horodatage correspondant (210) ;

l'identification d'une voie perturbée (212), où la voie perturbée indique une détérioration de la santé intestinale de l'individu, dans lequel la voie perturbée est identifiée sur la base d'une comparaison de l'abondance de voie de la pluralité d'échantillons intestinaux obtenus à l'horodatage 1 et à l'horodatage 2 en utilisant une technique statistique, dans lequel la voie perturbée est identifiée sur la base de l'un des cas ci-dessous :

(a) cas A : une diminution de l'abondance de voie de l'ensemble de voies bénéfiques, dans lequel la diminution est indicative d'une diminution de l'ensemble de microbes bénéfiques, ou
(b) cas B : une augmentation de l'abondance de voie de l'ensemble de voies nocives dans lequel l'augmentation est indicative d'une augmentation de l'ensemble de microbes nocifs, ou
(c) cas C : une diminution de l'abondance de voie de l'ensemble de voies bénéfiques et une augmentation de l'abondance de voie de l'ensemble de voies nocives indicative d'une augmentation de l'ensemble de microbes nocifs et d'une diminution de l'ensemble de microbes bénéfiques ;

la détermination, en utilisant la ProbMap, d'un ensemble initial de produits thérapeutiques personnalisés et d'une recommandation de régime alimentaire, dans lequel l'ensemble initial de produits thérapeutiques personnalisés et de régime alimentaire comprend au moins l'un parmi : un produit thérapeutique personnalisé et un régime alimentaire personnalisé, dans lequel l'ensemble initial de produits thérapeutiques personnalisés et de recommandation de régime alimentaire est une rangée de la ProbMap correspondant à la voie perturbée identifiée, dans lequel le produit thérapeutique personnalisé est déterminé comme étant (a) la pluralité de souches bactériennes possédant la voie perturbée ou (b) la pluralité de souches bactériennes possédant la voie pour la dégradation et/ou la biosynthèse d'un métabolite probable, dans lequel le métabolite probable est un métabolite correspondant à la voie perturbée dans la ProbMap (214) ;
l'identification d'un ensemble de taxa dans la matrice d'abondance de taxa obtenue à l'horodatage 2, dans lequel l'ensemble de taxa comprend un TAXA_SET_P et un TAXA_SET_A, où le TAXA_SET_P comprend une première valeur prédéfinie pour la ou les voies bénéfiques dans l'un parmi la FGmap et la GMP, et le TAXA_SET_A comprend une seconde valeur prédéfinie pour la ou les voies nocives dans l'un parmi la FGmap et la GMP (216) ;
l'identification d'un ensemble de premiers voisins pour chacun des taxa dans le TAXA_SET_P à l'horodatage 2 à partir du graphe TAXA_NET, dans lequel l'ensemble de premiers voisins sont des voisins immédiats du TAXA_SET_P comprenant un TAXA_SET_FN, un TAXA_SET_FN_COM, un TAXA_SET_INTERSECT, un TAXA_SET_NEW (218), dans lequel l'ensemble identifié de premiers voisins du TAXA_SET_P dans le graphe TAXA_NET est sauvegardé dans la nouvelle liste TAXA_SET_FN, suivie par la prédiction du potentiel fonctionnel des taxa dans le TAXA_SET_FN et l'identification de ceux qui sont commensales sur la base de leur capacité de production de butyrate et la sauvegarde du sous-ensemble de ces taxa à partir du TAXA_SET_FN comme TAXA_SET_FN_COM, et
l'identification d'un ensemble de taxa commun entre le TAXA_SET_FN_COM et un ensemble de tous les taxa présents dans l'horodatage 2 et la sauvegarde de l'ensemble identifié de taxa comme TAXA_SET_INTERSECT et la sauvegarde des taxa restants dans le TAXA_SET_FN_COM qui ne sont pas présents dans l'horodatage 2 comme TAXA_SET_NEW ; et
la recommandation d'une intervention thérapeutique personnalisée pour améliorer la santé intestinale de l'individu (220), via le ou les processeurs matériels, dans lequel l'intervention thérapeutique comprend la recommandation de l'un parmi (a) un cocktail prébiotique, (b) un cocktail probiotique, (c) un régime alimentaire optimisé sur la base des premiers voisins identifiés en utilisant l'ensemble de bases de connaissances et l'ensemble initial de produits thérapeutiques personnalisés et la recommandation de régime alimentaire, dans lequel l'intervention thérapeutique personnalisée comprend au moins l'un ou plusieurs parmi :

(a) pour le cas A (une diminution de l'abondance de voie de l'ensemble de microbes bénéfiques) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques et aident à la croissance des microbes bénéfiques,
(b) pour le cas B (une augmentation de l'abondance de voie de l'ensemble de microbes nocifs) la recommandation d'un régime alimentaire optimisé, dans lequel le régime alimentaire optimisé favorise la croissance de la pluralité de microbes bénéfiques et inhibe la croissance de microbes nocifs, et
(c) pour le cas C (une diminution de l'abondance de voie de l'ensemble de microbes bénéfiques et une augmentation de l'abondance de voie de l'ensemble de microbes nocifs) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques, aident à la croissance de microbes bénéfiques et inhibent la croissance de microbes nocifs.

2. Procédé mis en œuvre par processeur selon la revendication 1, dans lequel la pluralité de voies métaboliques bénéfiques pourraient inclure une ou plusieurs parmi, mais sans s'y limiter, la production de pyruvate en butyrate par la voie pyruvate, la voie d'oxydation d'ammoniac ainsi que la voie pour la dégradation d'oxyde de triméthylamine (TMA) et la pluralité de voies métaboliques nocives pourraient inclure une ou plusieurs parmi, mais sans s'y limiter, les voies de libération d'ammoniac, la production d'amine biogénique et la production de triméthylamine.

3. Procédé mis en œuvre par processeur selon la revendication 1, dans lequel la génération de la GMP et du FGmap (300) comprend :

la génération d'une carte métabolique matricielle (Mmap) sur la base de la pluralité de métabolites, de la pluralité de voies métaboliques et d'une liste d'organismes associés à chacune de la pluralité de voies métaboliques, dans lequel la Mmap est générée pour chacun de la pluralité de métabolites (302) ;
la génération d'une carte génomique bactérienne (BGM) à l'aide d'une base de données génomiques bactériennes (BGD), dans lequel la BGD comprend parmi une liste exhaustive la pluralité de souches bactériennes et la BGM comprend des informations associées à la pluralité de souches bactériennes incluant une pluralité de noms de la pluralité de souches bactériennes, une pluralité de numéros d'identification de la pluralité de souches bactériennes, une pluralité de localisations géniques de la pluralité de souches bactériennes et une pluralité de domaines protéiques de la pluralité de souches bactériennes (304) ;
la génération d'une carte de domaine de voie métabolique (MPDM) pour chaque métabolite de la pluralité de métabolites sur la base de la Mmap et de la BGM, dans lequel la MPDM comprend parmi la liste exhaustive des domaines protéiques associés à chacune de la pluralité de voies métaboliques (306) ;
l'identification de la présence de la pluralité de voies métaboliques dans la liste de souches bactériennes et de génomes à partir de l'utilisation de la MPDM et de la BGM sur la base d'un premier critère de seuil prédéfini (308) ;
la génération de la GMP à l'aide de la BGM, de la MPDM, de la pluralité de voies métaboliques et de la pluralité de métabolites, dans lequel la GMP est une matrice avec un ensemble de génomes bactériens (souches bactériennes) comme une pluralité de rangées et la liste de la pluralité de voies métaboliques correspondant à la formation de chaque métabolite comme une pluralité de colonnes (310) ; et
la génération de la FGmap à l'aide de la GMP, dans lequel la FGmap est une matrice avec la pluralité de genres bactériens comme une pluralité de rangées et la pluralité de voies métaboliques comme des colonnes (312).

4. Procédé mis en œuvre par processeur selon la revendication 1, dans lequel la technique de traitement d'échantillon (400) pour générer les matrices d'abondance de voie comprend :

l'extraction d'une pluralité d'acides nucléiques à partir de la pluralité d'échantillons intestinaux sur la base d'une technique d'extraction d'acide nucléique ; (402)
la génération d'une pluralité de séquences nucléotidiques à partir de la pluralité extraite d'acides nucléiques à l'aide d'un séquenceur ; (404)
l'obtention d'un ensemble de matrice d'abondance taxonomique bactérienne à chaque niveau de hiérarchie taxonomique à l'aide de la pluralité de séquences nucléotidiques, dans lequel la matrice d'abondance taxonomique bactérienne est obtenue en classant la pluralité de séquences nucléotidiques sur la base d'un niveau taxonomique prédéfini à l'aide d'un algorithme de classification ; (406) et
la génération d'une abondance de voie à l'aide de l'ensemble de matrice d'abondance taxonomique bactérienne, de la GMP et de la FGmap. (408)

5. Procédé mis en œuvre par processeur selon la revendication 1, dans lequel le processus de recommandation du régime alimentaire optimisé (500) comprend :

le calcul d'un changement normalisé ($d_k$) pour chaque taxa dans l'union de l'ensemble de taxa 1 et de l'ensemble de taxa 2, entre l'horodatage 1 et l'horodatage 2 (502) ;
le calcul d'un score d'importance ($b_k$) de chaque taxa dans l'union de l'ensemble de taxa 1 et de l'ensemble de taxa 2, à l'aide du changement normalisé, de la pluralité de microbes bénéfiques, de la pluralité de microbes nocifs à partir de l'ensemble de bases de connaissances (504) ;
le calcul d'un score de composé intestinal personnalisé ($gcs_j$) pour un individu à l'aide du score d'importance ($b_k$) de chaque taxa dans l'union de l'ensemble de taxa 1 et de l'ensemble de taxa 2 et d'une matrice d'utilisation de composé de taxa prédéfinie, dans lequel le score de composé intestinal personnalisé quantifie la capacité du composé à augmenter la pluralité de microbes bénéfiques dans l'intestin (506) ;

le calcul d'un score alimentaire intestinal (*gfs<sub>j</sub>*) pour tous les articles alimentaires dans l'ensemble d'articles alimentaires à l'aide du score de composé intestinal et de la matrice de constituants alimentaires, dans lequel le score alimentaire intestinal quantifie la capacité des articles alimentaires à augmenter la pluralité de microbes bénéfiques (508) ; et

l'optimisation du score alimentaire intestinal à l'aide d'une technique d'optimisation pour obtenir le régime alimentaire optimisé, dans lequel le score alimentaire intestinal est optimisé sous réserve d'une pluralité de paramètres de contrainte comprenant un paramètre de calories, un paramètre de glucides, un paramètre de protéines et un paramètre de graisses (510).

6. Procédé mis en œuvre par processeur selon la revendication 5, dans lequel le score alimentaire intestinal d'un article alimentaire spécifique (aliment$_i$) est exprimé comme ci-dessous :

$$\text{gfs}_i \; = \; \sum_{j=1}^{p} \mathbf{C}_{j,i} \times \text{gcs}_j$$
$$o\grave{u},$$

$\mathbf{C} \in \mathbb{R}^{p \times m}$ est la matrice de composition alimentaire, dans lequel $\mathbf{C}_{j,i}$ est la teneur en composé$_j$ par unité d'aliment.

p = nombre total de composés obtenus dans des aliments à partir d'aliments de liste et

m = nombre total d'aliments.

$$\text{gcs}_j = \sum_{k=1}^{q} T_{j,k} \times b_k \times I[\text{taxa}_k \in \text{PU}] \; - \; \sum_{k=1}^{q} T_{j,k} \times b_k \times I[\text{taxa}_k \in \text{AP}]$$

dans lequel,

T est la matrice d'utilisation de composé de taxa,

dans lequel,

$$T \in \{0, 1\}^{p \times q}; \; T_{j,k} :=$$

$$\begin{Bmatrix} 1 \text{ si taxa}_j \text{ peut utiliser } \text{composé}_k \text{ comme source d'énergie} \\ 0 \text{ sinon} \end{Bmatrix},$$

q = nombre total de taxa bactériens ;

I est la fonction d'indicateur, c'est-à-dire $I[y \in Z] := \begin{Bmatrix} 1 \text{ if } y \in Z \\ 0 \text{ sinon} \end{Bmatrix},$

PU : ensemble de taxa bactériens commensales,

AP : ensemble de taxa bactériens pathogènes,

$$b_k \; = \; \max\big((1 \, + \, d_k \times I[\text{taxa}_k \in \text{AP}] \, - \, d_i \times I[\text{taxa}_k \in \text{PU}]), 1\big)$$

dans lequel,

$$d_k \; = \; \frac{[A_k^2 - A_k^1]}{\sigma_k},$$

dans lequel,

$A_k^2$ est l'abondance de taxa$_k$ dans l'échantillon T2,

$A_k^1$ est l'abondance de taxa$_k$ dans T1,

$\sigma_k$ est l'écart-type de taxa$_k$ dans une pluralité d'échantillons de microbiome intestinal sains.

7.  Procédé mis en œuvre par processeur selon la revendication 1, dans lequel le terme « voie perturbée » fait référence à une augmentation de voies nocives ou de biosynthèse de métabolites nocifs ou de dégradation de métabolites bénéfiques ou une diminution de voies bénéfiques ou de métabolites bénéfiques ou de dégradation de métabolites nocifs et la voie perturbée sont identifiées sur la base de techniques statistiques incluant l'une parmi une technique paramétrique, une technique non paramétrique et un algorithme basé sur l'apprentissage machine.

8.  Procédé mis en œuvre par processeur selon la revendication 1, dans lequel l'intervention thérapeutique personnalisée comprend en outre l'administration d'une bactérie génétiquement modifiée, dans lequel la bactérie génétiquement modifiée comprend la modification génétique (a) d'une pluralité de voies microbiennes pour la production d'un ou plusieurs métabolites bénéfiques, ou/et (b) de la pluralité de voies microbiennes pour la dégradation/élimination d'un ou plusieurs métabolites nocifs dans la composition génétique de micro-organismes intestinaux, dans lequel la bactérie génétiquement modifiée est administrée de l'une des manières partagées ci-dessous :

> (a) génétiquement modifiée en tant que probiotiques soit seuls soit en combinaison avec l'un parmi un prébiotique, un métabiotique et un paraprobiotique,
> (b) organisme génétiquement modifié exprimant une ou plusieurs voies hétérologues requises pour produire des métabolites, dans lequel l'organisme génétiquement modifié peut être l'un ou plusieurs parmi des commensaux intestinaux, dans lequel les commensaux intestinaux peuvent inclure, mais sans s'y limiter, des microbes bénéfiques, des souches probiotiques commerciales ou des anaérobies facultatives capables de résider dans l'intestin ; dans lequel l'organisme génétiquement modifié est également appelé bactérie modifiée, dans lequel la bactérie modifiée peut être administrée en tant que produit alimentaire, complément alimentaire ou composition pharmaceutique ou formulation probiotique.

9.  Système (100), comprenant :

> une interface d'entrée/sortie (106) ;
> une ou plusieurs mémoires (102) ; et
> un ou plusieurs processeurs matériels (104), les une ou plusieurs mémoires (104) étant couplées aux un ou plusieurs processeurs matériels (104), dans lequel les un ou plusieurs processeurs matériels (104) sont configurés pour exécuter des instructions programmées stockées dans les une ou plusieurs mémoires (102), pour :
> recevoir un ensemble de données d'entrée, via un ou plusieurs processeurs matériels, dans lequel l'ensemble de données d'entrée comprend des données associées à

>> une pluralité de métabolites, une pluralité de voies métaboliques, une pluralité de groupes taxonomiques, des informations sur des nutriments, des probiotiques, des prébiotiques, des compléments pour la régénération ou la dégradation de la pluralité de métabolites,
>> une pluralité de constituants alimentaires, une pluralité d'articles alimentaires, une matrice d'abondance microbienne pour des cohortes saines et une pluralité de paramètres préalables d'optimisation de régime alimentaire,

>> dans lequel la pluralité de métabolites comprend un ensemble de métabolites bénéfiques et un ensemble de métabolites nocifs, dans lequel l'ensemble de métabolites bénéfiques est bénéfique pour la santé intestinale d'un individu et l'ensemble de métabolites nocifs est nocif pour la santé intestinale de l'individu,
>> dans lequel la pluralité de voies métaboliques comprend l'une parmi (a) une voie bénéfique et (b) des voies nocives,

>>> dans lequel les voies bénéfiques comprennent une ou plusieurs parmi un ensemble de voies métaboliques pour la biosynthèse de la pluralité de métabolites bénéfiques et un ensemble de voies métaboliques pour la dégradation de la pluralité de métabolites nocifs, et
>>> dans lequel les voies nocives comprennent une ou plusieurs parmi un ensemble de voies métaboliques pour la dégradation de la pluralité de métabolites bénéfiques et un ensemble de voies métaboliques pour la biosynthèse de la pluralité de métabolites nocifs ;

dans lequel la pluralité de groupes taxonomiques comprend une pluralité de microbes, où la pluralité de microbes comprend une pluralité de microbes bénéfiques et une pluralité de microbes nocifs, où la pluralité de microbes sont classés selon une hiérarchie taxonomique, dans lequel la hiérarchie taxonomique comprend un ou plusieurs parmi une pluralité de phylères bactériennes, une pluralité de classes bactériennes, une pluralité d'ordres bactériens, une pluralité de familles bactériennes, une pluralité de genres bactériens, une pluralité d'espèces bactériennes et une pluralité de souches bactériennes, où la pluralité de microbes comprend parmi une pluralité de gènes ayant une localisation génomique distincte, un ensemble de séquences nucléotidiques et protéiques des souches bactériennes,

dans lequel la pluralité de microbes bénéfiques abrite les voies bénéfiques et la pluralité de microbes nocifs abrite les voies nocives ; et

dans lequel la pluralité de paramètres préalables d'optimisation de régime alimentaire comprend un ensemble d'articles alimentaires, un ensemble complet de composés qui sont trouvés dans l'ensemble d'articles alimentaires, une matrice de constituants alimentaires, dans lequel la matrice de constituants alimentaires tabule la teneur de chaque composé appartenant à l'ensemble de composés dans chaque article alimentaire appartenant à l'ensemble d'aliments, la teneur totale en glucides, protéines, graisses et calories de chacun des articles alimentaires de l'ensemble d'articles alimentaires, une liste de taxa bactériens dans l'intestin humain, un ensemble de fonctions pour identifier un ensemble de taxa bactériens bénéfiques et un ensemble de taxa bactériens nocifs, une matrice d'utilisation de composés de taxa, un ensemble défini par l'utilisateur de paramètres liés supérieurs - paramètres liés inférieurs associés à des glucides, protéines, graisses et calories, et des articles alimentaires sélectionnés par l'utilisateur ;

générer un ensemble de bases de connaissances en utilisant l'ensemble de données d'entrée, via les un ou plusieurs processeurs matériels, dans lequel l'ensemble de bases de connaissances comprend une carte de fonction de genre (FGmap), une carte de voie métabolique génomique (GMP), un graphique de profil de référence d'association de taxa (TAXA_NET), un réseau de constituants alimentaires TAXA, un réseau de constituants alimentaires et une ProbMap, dans lequel la génération de l'ensemble de bases de connaissances comprend :

la génération de la FGmap et de la carte GMP, dans lequel la FGmap est une matrice associée à une fonction de la pluralité de genres bactériens appartenant à l'un de la pluralité de groupes taxonomiques et de la pluralité de voies métaboliques sous forme de colonnes, dans lequel un premier indicateur prédéfini indique l'une de (a) une présence de la voie métabolique et (b) une absence de la voie métabolique et la GMP est une matrice de la pluralité de souches bactériennes sous forme de rangées et de la pluralité de voies métaboliques sous forme de colonnes, dans lequel un second indicateur prédéfini indique l'une de (a) une présence de la voie métabolique et (b) une absence de la voie métabolique ;

la génération du graphique TAXA_NET sur la base de la pluralité de groupes taxonomiques et de la FGmap, dans lequel le graphique TAXA_NET est un graphique non dirigé, dans lequel chaque noeud dans le graphique TAXA_NET correspond à un groupe taxonomique de la pluralité de groupes taxonomiques et les bords indiquent une relation positive entre une paire du groupe taxonomique dans lequel chaque taxon d'une pluralité de noeuds est associé à la fonction de la pluralité de groupes taxonomiques de la FGmap, dans lequel la relation positive comprend l'un d'un mutualisme, d'un syntrophisme, d'un commensalisme et d'une coopération ;

la génération du réseau de constituants alimentaires TAXA sur la base d'une pluralité de constituants alimentaires pour chaque taxon du TAXA_NET, dans lequel le réseau de constituants alimentaires TAXA est un réseau non dirigé de chaque taxon en association avec un constituant alimentaire utilisé par le taxon comme source de métabolisme ou de nutrition ;

la génération du réseau de constituants alimentaires sur la base d'une pluralité d'articles alimentaires pour chaque constituant alimentaire du réseau de constituants alimentaires TAXA, dans lequel le réseau de constituants alimentaires est un réseau non dirigé de chaque constituant alimentaire présent dans un article alimentaire ; et

la génération d'une transposition de la GMP pour obtenir la ProbMap, dans lequel la ProbMap est une matrice avec des rangées comprenant chaque métabolite de la pluralité de métabolites et la pluralité de voies métaboliques et des colonnes comprenant la pluralité de souches bactériennes, de probiotiques, de prébiotiques, de composants de régime alimentaire capables de biosynthèse et/ou de dégradation de la pluralité correspondante de métabolites ;

la réception d'une pluralité d'échantillons intestinaux d'un individu à deux horodatages, via les un ou plusieurs processeurs matériels, dans lequel les deux horodatages comprennent un horodatage 1 et un horodatage 2 auxquels les deux échantillons ont été collectés ;

la génération d'une matrice d'abondance de taxa pour la pluralité d'échantillons intestinaux, via les un ou plusieurs processeurs matériels, dans lequel la matrice d'abondance de taxa comprend un ensemble de taxa 1 et un ensemble de taxa 2, où l'ensemble de taxa 1 et l'ensemble de taxa 2 sont générés en utilisant l'horodatage 1 et l'horodatage 2 ;

la génération d'une abondance de voie pour chacun de la pluralité d'échantillons intestinaux obtenus à l'horodatage 1 et à l'horodatage 2 en utilisant la matrice d'abondance de taxa et la FGmap, via les un ou plusieurs processeurs matériels, dans lequel l'abondance de voie est générée en utilisant une technique de traitement d'échantillon, dans lequel l'abondance de voie pour chaque horodatage est indicative de la présence de la pluralité de voies métaboliques de la GMP dans le microbiome intestinal de l'individu à l'horodatage correspondant ;

l'identification d'une voie perturbée, où la voie perturbée indique une détérioration de la santé intestinale de l'individu, dans lequel la voie perturbée est identifiée sur la base d'une comparaison de l'abondance de voie de la pluralité d'échantillons intestinaux obtenus à l'horodatage 1 et à l'horodatage 2 en utilisant une technique statistique, dans lequel la voie perturbée est identifiée sur la base de l'un des cas ci-dessous :

(a) cas A : une diminution de l'abondance de voie de l'ensemble de voies bénéfiques, dans lequel la diminution est indicative d'une diminution de l'ensemble de microbes bénéfiques, ou

(b) cas B : une augmentation de l'abondance de voie de l'ensemble de voies nocives dans lequel l'augmentation est indicative d'une augmentation de l'ensemble de microbes nocifs, ou

(c) cas C : une diminution de l'abondance de voie de l'ensemble de voies bénéfiques et une augmentation de l'abondance de voie de l'ensemble de voies nocives indicative d'une augmentation de l'ensemble de microbes nocifs et d'une diminution de l'ensemble de microbes bénéfiques ;

la détermination, en utilisant la ProbMap, d'un ensemble initial de produits thérapeutiques personnalisés et d'une recommandation de régime alimentaire, dans lequel l'ensemble initial de produits thérapeutiques personnalisés et de régime alimentaire comprend au moins l'un parmi : un produit thérapeutique personnalisé et un régime alimentaire personnalisé, dans lequel l'ensemble initial de produits thérapeutiques personnalisés et de recommandation de régime alimentaire est une rangée de la ProbMap correspondant à la voie perturbée identifiée, dans lequel le produit thérapeutique personnalisé est déterminé comme étant (a) la pluralité de souches bactériennes possédant la voie perturbée ou (b) la pluralité de souches bactériennes possédant la voie pour la dégradation et/ou la biosynthèse d'un métabolite probable, dans lequel le métabolite probable est un métabolite correspondant à la voie perturbée dans la ProbMap ;

l'identification d'un ensemble de taxa dans la matrice d'abondance de taxa obtenue à l'horodatage 2, dans lequel l'ensemble de taxa comprend un TAXA_SET_P et un TAXA_SET_A, où le TAXA_SET_P comprend une première valeur prédéfinie pour la ou les voies bénéfiques dans l'un parmi la FGmap et la GMP, et le TAXA_SET_A comprend une seconde valeur prédéfinie pour la ou les voies nocives dans l'un parmi la FGmap et la GMP ;

l'identification d'un ensemble de premiers voisins pour chacun des taxa dans le TAXA_SET_P à l'horodatage 2 à partir du graphe TAXA_NET, dans lequel l'ensemble de premiers voisins sont des voisins immédiats du TAXA_SET_P comprenant un TAXA_SET_FN, un TAXA_SET_FN_COM, un TAXA_SET_INTERSECT, un TAXA_SET_NEW, dans lequel l'ensemble identifié de premiers voisins du TAXA_SET_P dans le graphe TAXA_NET est sauvegardé dans la nouvelle liste TAXA_SET_FN, suivie par la prédiction du potentiel fonctionnel des taxa dans le TAXA_SET_FN et l'identification de ceux qui sont commensales sur la base de leur capacité de production de butyrate et la sauvegarde du sous-ensemble de ces taxa à partir du TAXA_SET_FN comme TAXA_SET_FN_COM, et

l'identification d'un ensemble de taxa commun entre le TAXA_SET_FN_COM et un ensemble de tous les taxa présents dans l'horodatage 2 et la sauvegarde de l'ensemble identifié de taxa comme TAXA_SET_INTERSECT et la sauvegarde des taxa restants dans le TAXA_SET_FN_COM qui ne sont pas présents dans l'horodatage 2 comme TAXA_SET_NEW ; et

la recommandation d'une intervention thérapeutique personnalisée pour améliorer la santé intestinale de l'individu, via le ou les processeurs matériels, dans lequel l'intervention thérapeutique comprend la recommandation de l'un parmi (a) un cocktail prébiotique, (b) un cocktail probiotique, (c) un régime alimentaire optimisé sur la base des premiers voisins identifiés en utilisant l'ensemble de bases de connaissances et l'ensemble initial de produits thérapeutiques personnalisés et la recommandation de régime alimentaire, dans lequel l'intervention thérapeutique personnalisée comprend au moins l'un ou plusieurs parmi :

(a) pour le cas A (une diminution de l'abondance de voie de l'ensemble de microbes bénéfiques) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques et aident à la croissance des microbes bénéfiques,

(b) pour le cas B (une augmentation de l'abondance de voie de l'ensemble de microbes nocifs) la recommandation d'un régime alimentaire optimisé, dans lequel le régime alimentaire optimisé favorise la croissance de la pluralité de microbes bénéfiques et inhibe la croissance de microbes nocifs, et

(c) pour le cas C (une diminution de l'abondance de voie de l'ensemble de microbes bénéfiques et une augmentation de l'abondance de voie de l'ensemble de microbes nocifs) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques, aident à la croissance de microbes bénéfiques et inhibent la croissance de microbes nocifs.

10. Système selon la revendication 9, dans lequel les un ou plusieurs processeurs matériels sont configurés par les instructions pour mettre en œuvre la génération de la GMP et du FGmap comprenant :

la génération d'une carte métabolique matricielle (Mmap) sur la base de la pluralité de métabolites, de la pluralité de voies métaboliques et d'une liste d'organismes associée à chacune de la pluralité de voies métaboliques, dans lequel la Mmap est générée pour chacun de la pluralité de métabolites ;

la génération d'une carte génomique bactérienne (BGM) à l'aide d'une base de données génomiques bactériennes (BGD), dans lequel la BGD comprend parmi une liste exhaustive la pluralité de souches bactériennes et la BGM comprend des informations associées à la pluralité de souches bactériennes incluant une pluralité de noms de la pluralité de souches bactériennes, une pluralité de numéros d'identification de la pluralité de souches bactériennes, une pluralité de localisations géniques de la pluralité de souches bactériennes et une pluralité de domaines protéiques de la pluralité de souches bactériennes ;

la génération d'une carte de domaine de voie métabolique (MPDM) pour chaque métabolite de la pluralité de métabolites sur la base de la Mmap et de la BGM, dans lequel la MPDM comprend parmi la liste exhaustive des domaines protéiques associés à chacune de la pluralité de voies métaboliques ;

l'identification de la présence de la pluralité de voies métaboliques dans la liste de souches bactériennes et de génomes à partir de l'utilisation de la MPDM et de la BGM sur la base d'un premier critère de seuil prédéfini ;

la génération de la GMP à l'aide de la BGM, de la MPDM, de la pluralité de voies métaboliques et de la pluralité de métabolites, dans lequel la GMP est une matrice avec un ensemble de génomes bactériens (souches bactériennes) comme une pluralité de rangées et la liste de la pluralité de voies métaboliques correspondant à la formation de chaque métabolite comme une pluralité de colonnes ; et

la génération de la FGmap à l'aide de la GMP, dans lequel la FGmap est une matrice avec la pluralité de genres bactériens comme une pluralité de rangées et la pluralité de voies métaboliques comme des colonnes.

11. Système selon la revendication 9, dans lequel les un ou plusieurs processeurs matériels sont configurés par les instructions pour mettre en œuvre la technique de traitement d'échantillon (400) pour générer les matrices d'abondance de voie comprenant :

l'extraction d'une pluralité d'acides nucléiques à partir de la pluralité d'échantillons intestinaux sur la base d'une technique d'extraction d'acide nucléique ;

la génération d'une pluralité de séquences nucléotidiques à partir de la pluralité extraite d'acides nucléiques à l'aide d'un séquenceur ;

l'obtention d'un ensemble de matrice d'abondance taxonomique bactérienne à chaque niveau de hiérarchie taxonomique à l'aide de la pluralité de séquences nucléotidiques, dans lequel la matrice d'abondance taxonomique bactérienne est obtenue en classant la pluralité de séquences nucléotidiques sur la base d'un niveau taxonomique prédéfini à l'aide d'un algorithme de classification ; et

la génération d'une abondance de voie à l'aide de l'ensemble de matrice d'abondance taxonomique bactérienne, de la GMP et de la FGmap.

12. Système selon la revendication 9, dans lequel les un ou plusieurs processeurs matériels sont configurés par les instructions pour mettre en œuvre le processus de recommandation du régime alimentaire optimisé comprend :

le calcul d'un changement normalisé ($d_k$) pour chaque taxa dans l'union de l'ensemble de taxa 1 et de l'ensemble de taxa 2, entre l'horodatage 1 et l'horodatage 2 ;

le calcul d'un score d'importance ($b_k$) de chaque taxa dans l'union de l'ensemble de taxa 1 et de l'ensemble de taxa 2, à l'aide du changement normalisé, de la pluralité de microbes bénéfiques, de la pluralité de microbes nocifs à partir de l'ensemble de bases de connaissances ;

le calcul d'un score de composé intestinal personnalisé (*gcs*$_j$) pour un individu à l'aide du score d'importance ($b_k$) de chaque taxa dans l'union de l'ensemble de taxa 1 et de l'ensemble de taxa 2 et d'une matrice d'utilisation de composé de taxa prédéfinie, dans lequel le score de composé intestinal personnalisé quantifie la capacité du composé à augmenter la pluralité de microbes bénéfiques dans l'intestin ;

le calcul d'un score alimentaire intestinal (*gfs*$_j$) pour tous les articles alimentaires dans l'ensemble d'articles alimentaires à l'aide du score de composé intestinal et de la matrice de constituants alimentaires, dans lequel le score alimentaire intestinal quantifie la capacité des articles alimentaires à augmenter la pluralité de microbes bénéfiques ; et

l'optimisation du score alimentaire intestinal à l'aide d'une technique d'optimisation pour obtenir le régime alimentaire optimisé, dans lequel le score alimentaire intestinal est optimisé sous réserve d'une pluralité de paramètres de contrainte comprenant un paramètre de calories, un paramètre de glucides, un paramètre de protéines et un paramètre de graisses.

**13.** Système selon la revendication 12, dans lequel les un ou plusieurs processeurs matériels sont configurés par les instructions pour mettre en œuvre le calcul du score alimentaire intestinal d'un article alimentaire spécifique (aliment$_i$), exprimé comme montré ci-dessous :

$$\text{gfs}_i = \sum_{j=1}^{p} \mathbf{C_{j,i}} \times \text{gcs}_j$$

où, $\mathbf{C} \in \mathbb{R}^{p \times m}$ est la matrice de composition alimentaire, dans lequel $\mathbf{C_{j,i}}$ est la teneur en composé$_j$ par unité d'aliment.

p = nombre total de composés obtenus dans des aliments à partir d'aliments de liste et

m = nombre total d'aliments.

$$\text{gcs}_j = \sum_{k=1}^{q} T_{j,k} \times b_k \times I[\text{taxa}_k \in PU] - \sum_{k=1}^{q} T_{j,k} \times b_k \times I[\text{taxa}_k \in AP]$$

dans lequel,

T est la matrice d'utilisation de composé de taxa,

dans lequel,

$$T \in \{0,1\}^{p \times q}; \; T_{j,k} :=$$

$$\left\{ \begin{array}{c} 1 \text{ si taxa}_j \text{ peut utiliser composé}_k \text{ comme source d'énergie} \\ 0 \text{ sinon} \end{array} \right\},$$

q = nombre total de taxa bactériens ;

I est la fonction d'indicateur, c'est-à-dire $I[y \in Z] := \left\{ \begin{array}{c} 1 \text{ if } y \in Z \\ 0 \text{ sinon} \end{array} \right\}$,

PU : ensemble de taxa bactériens commensales,

AP : ensemble de taxa bactériens pathogènes,

$$b_k = \max\big((1 + d_k \times I[\text{taxa}_k \in AP] - d_i \times I[\text{taxa}_k \in PU]), 1\big)$$

dans lequel,

$$d_k = \frac{[A_k^2 - A_k^1]}{\sigma_k},$$

dans lequel,

$A_k^2$ est l'abondance de taxa$_k$ dans l'échantillon T2,

$A_k^1$ est l'abondance de taxa$_k$ dans T1,

$\sigma_k$ est l'écart-type de taxa$_k$ dans une pluralité d'échantillons de microbiome intestinal sains.

14. Système selon la revendication 9, dans lequel les un ou plusieurs processeurs matériels sont configurés par les instructions pour mettre en œuvre l'intervention thérapeutique personnalisée et lequel comprend en outre l'administration d'une bactérie génétiquement modifiée, dans lequel la bactérie génétiquement modifiée comprend la modification génétique (a) d'une pluralité de voies microbiennes pour la production d'un ou plusieurs métabolites bénéfiques, ou/et (b) de la pluralité de voies microbiennes pour la dégradation/élimination d'un ou plusieurs métabolites nocifs dans la composition génétique de micro-organismes intestinaux, dans lequel la bactérie génétiquement modifiée est administrée de l'une des manières partagées ci-dessous :

(a) génétiquement modifiée en tant que probiotiques soit seuls soit en combinaison avec l'un parmi un prébiotique, un métabiotique et un paraprobiotique,
(b) organisme génétiquement modifié exprimant une ou plusieurs voies hétérologues requises pour produire des métabolites, dans lequel l'organisme génétiquement modifié peut être l'un ou plusieurs parmi des commensaux intestinaux, dans lequel les commensaux intestinaux peuvent inclure, mais sans s'y limiter, des microbes bénéfiques, des souches probiotiques commerciales ou des anaérobies facultatives capables de résider dans l'intestin ; dans lequel l'organisme génétiquement modifié est également appelé bactérie modifiée, dans lequel la bactérie modifiée peut être administrée en tant que produit alimentaire, complément alimentaire ou composition pharmaceutique ou formulation probiotique.

15. Un ou plusieurs supports de stockage d'informations lisibles par machine non transitoires comprenant une ou plusieurs instructions qui, lorsqu'elles sont exécutées par un ou plusieurs processeurs matériels, amènent :
la réception d'un ensemble de données d'entrée, via un ou plusieurs processeurs matériels, dans lequel l'ensemble de données d'entrée comprend des données associées à

une pluralité de métabolites, une pluralité de voies métaboliques, une pluralité de groupes taxonomiques, des informations sur des nutriments, des probiotiques, des prébiotiques, des compléments pour la régénération ou la dégradation de la pluralité de métabolites, une pluralité de constituants alimentaires, une pluralité d'articles alimentaires, une matrice d'abondance microbienne pour des cohortes saines et une pluralité de paramètres préalables d'optimisation de régime alimentaire,

dans lequel la pluralité de métabolites comprend un ensemble de métabolites bénéfiques et un ensemble de métabolites nocifs, dans lequel l'ensemble de métabolites bénéfiques est bénéfique pour la santé intestinale d'un individu et l'ensemble de métabolites nocifs est nocif pour la santé intestinale de l'individu, dans lequel la pluralité de voies métaboliques comprend l'une parmi (a) une voie bénéfique et (b) des voies nocives,

dans lequel les voies bénéfiques comprennent une ou plusieurs parmi un ensemble de voies métaboliques pour la biosynthèse de la pluralité de métabolites bénéfiques et un ensemble de voies métaboliques pour la dégradation de la pluralité de métabolites nocifs, et
dans lequel les voies nocives comprennent une ou plusieurs parmi un ensemble de voies métaboliques pour la dégradation de la pluralité de métabolites bénéfiques et un ensemble de voies métaboliques pour la biosynthèse de la pluralité de métabolites nocifs ;

dans lequel la pluralité de groupes taxonomiques comprend une pluralité de microbes, où la pluralité de microbes comprend une pluralité de microbes bénéfiques et une pluralité de microbes nocifs, où la pluralité de microbes sont classés selon une hiérarchie taxonomique, dans lequel la hiérarchie taxonomique comprend un ou plusieurs parmi une pluralité de phylères bactériennes, une pluralité de classes bactériennes, une pluralité d'ordres bactériens, une pluralité de familles bactériennes, une pluralité de genres bactériens, une pluralité d'espèces bactériennes et une pluralité de souches bactériennes, où la pluralité de microbes comprend parmi une pluralité de gènes ayant une localisation génomique distincte, un ensemble de séquences nucléotidiques et protéiques des souches bactériennes,

dans lequel la pluralité de microbes bénéfiques abrite les voies bénéfiques et la pluralité de microbes nocifs abrite les voies nocives ; et

dans lequel la pluralité de paramètres préalables d'optimisation de régime alimentaire comprend un ensemble d'articles alimentaires, un ensemble complet de composés qui sont trouvés dans l'ensemble d'articles alimentaires, une matrice de constituants alimentaires, dans lequel la matrice de constituants alimentaires tabule la teneur de chaque composé appartenant à l'ensemble de composés dans chaque article alimentaire appartenant à l'ensemble d'aliments, la teneur totale en glucides, protéines, graisses et calories de chacun des articles alimentaires de l'ensemble d'articles alimentaires, une liste de taxa bactériens dans l'intestin humain, un ensemble de fonctions pour identifier un ensemble de taxa bactériens bénéfiques et un ensemble de taxa bactériens nocifs, une matrice d'utilisation de composés de taxa, un ensemble défini par l'utilisateur de paramètres liés supérieurs - paramètres liés inférieurs associés à des glucides, protéines, graisses et calories, et des articles alimentaires sélectionnés par l'utilisateur ;

la génération d'un ensemble de bases de connaissances en utilisant l'ensemble de données d'entrée, via les un ou plusieurs processeurs matériels, dans lequel l'ensemble de bases de connaissances comprend une carte de fonction de genre (FGmap), une carte de voie métabolique génomique (GMP), un graphique de profil de référence d'association de taxa (TAXA_NET), un réseau de constituants alimentaires TAXA, un réseau de constituants alimentaires et une ProbMap, dans lequel la génération de l'ensemble de bases de connaissances comprend :

la génération de la FGmap et de la carte GMP, dans lequel la FGmap est une matrice associée à une fonction de la pluralité de genres bactériens appartenant à l'un de la pluralité de groupes taxonomiques et de la pluralité de voies métaboliques sous forme de colonnes, dans lequel un premier indicateur prédéfini indique l'une de (a) une présence de la voie métabolique et (b) une absence de la voie métabolique et la GMP est une matrice de la pluralité de souches bactériennes sous forme de rangées et de la pluralité de voies métaboliques sous forme de colonnes, dans lequel un second indicateur prédéfini indique l'une de (a) une présence de la voie métabolique et (b) une absence de la voie métabolique ;

la génération du graphique TAXA_NET sur la base de la pluralité de groupes taxonomiques et de la FGmap, dans lequel le graphique TAXA_NET est un graphique non dirigé, dans lequel chaque noeud dans le graphique TAXA_NET correspond à un groupe taxonomique de la pluralité de groupes taxonomiques et les bords indiquent une relation positive entre une paire du groupe taxonomique dans lequel chaque taxon d'une pluralité de noeuds est associé à la fonction de la pluralité de groupes taxonomiques de la FGmap, dans lequel la relation positive comprend l'un d'un mutualisme, d'un syntrophisme, d'un commensalisme et d'une coopération ;

la génération du réseau de constituants alimentaires TAXA sur la base d'une pluralité de constituants alimentaires pour chaque taxon du TAXA_NET, dans lequel le réseau de constituants alimentaires TAXA est un réseau non dirigé de chaque taxon en association avec un constituant alimentaire utilisé par le taxon comme source de métabolisme ou de nutrition ;

la génération du réseau de constituants alimentaires sur la base d'une pluralité d'articles alimentaires pour chaque constituant alimentaire du réseau de constituants alimentaires TAXA, dans lequel le réseau de constituants alimentaires est un réseau non dirigé de chaque constituant alimentaire présent dans un article alimentaire ; et

la génération d'une transposition de la GMP pour obtenir la ProbMap, dans lequel la ProbMap est une matrice avec des rangées comprenant chaque métabolite de la pluralité de métabolites et la pluralité de voies métaboliques et des colonnes comprenant la pluralité de souches bactériennes, de probiotiques, de prébiotiques, de composants de régime alimentaire capables de biosynthèse et/ou de dégradation de la pluralité correspondante de métabolites ;

la réception d'une pluralité d'échantillons intestinaux d'un individu à deux horodatages, via les un ou plusieurs processeurs matériels, dans lequel les deux horodatages comprennent un horodatage 1 et un horodatage 2 auxquels les deux échantillons ont été collectés ;

la génération d'une matrice d'abondance de taxa pour la pluralité d'échantillons intestinaux, via les un ou plusieurs processeurs matériels, dans lequel la matrice d'abondance de taxa comprend un ensemble de taxa 1 et un ensemble de taxa 2, où l'ensemble de taxa 1 et l'ensemble de taxa 2 sont générés en utilisant l'horodatage 1 et l'horodatage 2 ;

la génération d'une abondance de voie pour chacun de la pluralité d'échantillons intestinaux obtenus à l'horodatage 1 et à l'horodatage 2 en utilisant la matrice d'abondance de taxa et la FGmap, via les un ou plusieurs processeurs matériels, dans lequel l'abondance de voie est générée en utilisant une technique de traitement d'échantillon, dans lequel l'abondance de voie pour chaque horodatage est indicative de la présence de la pluralité de voies métaboliques de la GMP dans le microbiome intestinal de l'individu à l'horodatage correspondant ;

l'identification d'une voie perturbée, où la voie perturbée indique une détérioration de la santé intestinale de l'individu, dans lequel la voie perturbée est identifiée sur la base d'une comparaison de l'abondance de voie de la pluralité d'échantillons intestinaux obtenus à l'horodatage 1 et à l'horodatage 2 en utilisant une technique statistique, dans lequel la voie perturbée est identifiée sur la base de l'un des cas ci-dessous :

(a) cas A : une diminution de l'abondance de voie de l'ensemble de voies bénéfiques, dans lequel la diminution est indicative d'une diminution de l'ensemble de microbes bénéfiques, ou
(b) cas B : une augmentation de l'abondance de voie de l'ensemble de voies nocives dans lequel l'augmentation est indicative d'une augmentation de l'ensemble de microbes nocifs, ou
(c) cas C : une diminution de l'abondance de voie de l'ensemble de voies bénéfiques et une augmentation de l'abondance de voie de l'ensemble de voies nocives indicative d'une augmentation de l'ensemble de microbes nocifs et d'une diminution de l'ensemble de microbes bénéfiques ;

la détermination, en utilisant la ProbMap, d'un ensemble initial de produits thérapeutiques personnalisés et d'une recommandation de régime alimentaire, dans lequel l'ensemble initial de produits thérapeutiques personnalisés et de régime alimentaire comprend au moins l'un parmi : un produit thérapeutique personnalisé et un régime alimentaire personnalisé, dans lequel l'ensemble initial de produits thérapeutiques personnalisés et de recommandation de régime alimentaire est une rangée de la ProbMap correspondant à la voie perturbée identifiée, dans lequel le produit thérapeutique personnalisé est déterminé comme étant (a) la pluralité de souches bactériennes possédant la voie perturbée ou (b) la pluralité de souches bactériennes possédant la voie pour la dégradation et/ou la biosynthèse d'un métabolite probable, dans lequel le métabolite probable est un métabolite correspondant à la voie perturbée dans la ProbMap ;

l'identification d'un ensemble de taxa dans la matrice d'abondance de taxa obtenue à l'horodatage 2, dans lequel l'ensemble de taxa comprend un TAXA_SET_P et un TAXA_SET_A, où le TAXA_SET_P comprend une première valeur prédéfinie pour la ou les voies bénéfiques dans l'un parmi la FGmap et la GMP, et le TAXA_SET_A comprend une seconde valeur prédéfinie pour la ou les voies nocives dans l'un parmi la FGmap et la GMP ;

l'identification d'un ensemble de premiers voisins pour chacun des taxa dans le TAXA_SET_P à l'horodatage 2 à partir du graphe TAXA_NET, dans lequel l'ensemble de premiers voisins sont des voisins immédiats du TAXA_SET_P comprenant un TAXA_SET_FN, un TAXA_SET_FN_COM, un TAXA_SET_INTERSECT, un TAXA_SET_NEW, dans lequel l'ensemble identifié de premiers voisins du TAXA_SET_P dans le graphe TAXA_NET est sauvegardé dans la nouvelle liste TAXA_SET_FN, suivie par la prédiction du potentiel fonctionnel des taxa dans le TAXA_SET_FN et l'identification de ceux qui sont commensales sur la base de leur capacité de production de butyrate et la sauvegarde du sous-ensemble de ces taxa à partir du TAXA_SET_FN comme TAXA_SET_FN_COM, et

l'identification d'un ensemble de taxa commun entre le TAXA_SET_FN_COM et un ensemble de tous les taxa présents dans l'horodatage 2 et la sauvegarde de l'ensemble identifié de taxa comme TAXA_SET_INTERSECT et la sauvegarde des taxa restants dans le TAXA_SET_FN_COM qui ne sont pas présents dans l'horodatage 2 comme TAXA_SET_NEW ; et

la recommandation d'une intervention thérapeutique personnalisée pour améliorer la santé intestinale de l'individu, via le ou les processeurs matériels, dans lequel l'intervention thérapeutique comprend la recommandation de l'un parmi (a) un cocktail prébiotique, (b) un cocktail probiotique, (c) un régime alimentaire optimisé sur la base des premiers voisins identifiés en utilisant l'ensemble de bases de connaissances et l'ensemble initial de produits thérapeutiques personnalisés et la recommandation de régime alimentaire, dans lequel l'intervention thérapeutique personnalisée comprend au moins l'un ou plusieurs parmi :

(a) pour le cas A (une diminution de l'abondance de voie de l'ensemble de microbes bénéfiques) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques et aident à la croissance des microbes bénéfiques,
(b) pour le cas B (une augmentation de l'abondance de voie de l'ensemble de microbes nocifs) la recommandation d'un régime alimentaire optimisé, dans lequel le régime alimentaire optimisé favorise la croissance de la pluralité de microbes bénéfiques et inhibe la croissance de microbes nocifs, et

(c) pour le cas C (une diminution de l'abondance de voie de l'ensemble de microbes bénéfiques et une augmentation de l'abondance de voie de l'ensemble de microbes nocifs) la recommandation d'un cocktail prébiotique, d'un cocktail probiotique et d'un régime alimentaire optimisé, dans lequel le cocktail prébiotique, le cocktail probiotique et le régime alimentaire optimisé reconstituent la pluralité de microbes bénéfiques, aident à la croissance de microbes bénéfiques et inhibent la croissance de microbes nocifs.

SYSTEM
100

PROCESSOR(S)
104

I/O
INTERFACE(S)
106

MEMORY
102

DATABASE
108

FIG. 1

receiving a set of input data, via one or more hardware processors, wherein the set of input data comprises data associated with a plurality of metabolites, a plurality of metabolic pathways, a plurality of taxonomic groups, information on nutrients, probiotics, prebiotics, supplements for replenishment or degradation of the plurality of metabolites, a plurality of food constituents, a plurality of food items, a microbial abundance matrix for healthy cohorts, a plurality of diet optimization pre-requisite parameters

202

generating a set of knowledge bases using the set of input data, via the one or more hardware processors, wherein the set of knowledge bases comprises a genus function map (FGmap), a genome metabolite pathway (GMP) map, a taxa association reference profile (TAXA_NET) graph, a TAXA-food constituent network, a food-constituent network and a ProbMap wherein the generation of the set of knowledge bases comprises

204

A

200

FIG. 2A

A

generating the FGmap and the GMP map, wherein the FGmap is a matrix associated with a function of the plurality of bacterial genera belonging to one of the plurality of taxonomic group and the plurality of metabolic pathways as columns and the GMP is a matrix of the plurality of bacterial strains as rows and the plurality of metabolic pathways as columns — 204A

generating the TAXA_NET graph based on the plurality of taxonomic groups and the FGmap, wherein the TAXA_NET graph is an undirected graph, wherein each node in the TAXA_NET graph corresponds to a taxonomic group from the plurality of taxonomic groups and edges indicate a positive relationship between a pair of the taxonomic group — 204B

generating the TAXA-food constituent network based on a plurality of food constituents for each taxa of the TAXA_NET, wherein the TAXA-food constituent network is an undirected network of each taxa in association with a food constituent used by the taxa as a source of metabolism — 204C

200

B

FIG. 2B

generating the food-constituent network based on a plurality of food items for each food constituent of the TAXA-food constituent network, wherein the food-constituent network is an undirected network of each food-constituent present in a food item

*204D*

generating a transpose of the GMP to obtain the ProbMap, wherein the ProbMap is a matrix with rows comprising each metabolite from the plurality of metabolites and the plurality of metabolic pathways and columns comprising the plurality of bacterial strains, probiotics, prebiotics, diet components capable of biosynthesis and/or degradation of the corresponding plurality of metabolites

*204E*

receiving a plurality of gut samples of an individual at two-time stamps, via the one or more hardware processors, wherein the two-time stamps comprise a time stamp 1 and a time stamp 2 at which the two samples have been collected

*206*

B

C

*200*

FIG. 2C

```
        ( C )
          │
          ▼
┌─────────────────────────────────────────────────────────────────┐
│ generating a taxa abundance matrix for the plurality of gut samples, via the one or more hardware │
│ processors, wherein the taxa abundance matrix comprises of a taxa set 1 and a taxa set 2, where   │── 208
│ the taxa set 1 and the taxa set 2 is generated using the time stamp 1 and the time stamp 2        │
└─────────────────────────────────────────────────────────────────┘
          │
          ▼
┌─────────────────────────────────────────────────────────────────┐
│ generating a pathway abundance for each of the plurality of gut-samples obtained at the time      │
│ stamp 1 and the time stamp 2 using the taxa abundance matrix and the FGmap, via the one or        │
│ more hardware processors, wherein the pathway abundance is generated using a sample               │── 210
│ processing technique, wherein the pathway abundance for each time stamp is indicative of          │
│ presence of the plurality of metabolic pathways of the GMP in the gut microbiome of the           │
│ individual at the corresponding time stamp.                                                       │
└─────────────────────────────────────────────────────────────────┘
          │
          ▼
        ( D )
```

200

FIG. 2D

D

identifying a perturbed pathway, where the perturbed pathway indicates a deterioration in the gut health of the individual, wherein the perturbed pathway is identified based on a comparison of the pathway abundance of the plurality of gut-samples obtained at the time stamp 1 and the time stamp 2 using a statistical technique, wherein the perturbed pathway is identified based on one of below cases:

(a) Case A : a decrease in the pathway abundance of the set of beneficial pathways , wherein the decrease is an indicative of a decrease in the set of beneficial microbes, or

(b) Case B : an increase in the pathway abundance of the set of harmful pathways wherein the increase is indicative of an increase in the set of harmful microbes , or

(c) Case C : a decrease in the pathway abundance of the set of beneficial pathways and increase in the pathway abundance of the set of harmful pathways indicative of an increase in set of harmful microbes and decrease in the set of beneficial microbes

212

E

200

FIG. 2E

(E)

determining an initial set of personalized therapeutics and diet recommendation, wherein the initial set of personalized therapeutics and diet comprises atleast one of : a personalized therapeutic and a personalized dietary regimen using the ProbMap, wherein the initial set of personalized therapeutics and diet recommendation is a row corresponding to the identified perturbed pathway, wherein the personalized therapeutic is determined as (a) the plurality of bacterial strains possessing the perturbed pathway or (b) the plurality of bacterial strains possessing the pathway for degradation or/and biosynthesis of a probable metabolite, wherein the probable metabolite is a metabolite corresponding to the perturbed pathway in the ProbMap —214

identify a taxa set in the taxa abundance matrix obtained at the time stamp 2, wherein the taxa set comprises of a TAXA_SET_P and a TAXA_SET_A, where the TAXA_SET_P comprises a predefined first value for the one or more of the beneficial pathway in one of the FGmap and the GMP, and the TAXA_SET_A comprises a predefined second value for the one or more of harmful pathways in one of the FGmap and the GMP —216

200

(F)

FIG. 2F

EP 4 288 559 B1

F

identifying a set of first neighbors for each of the taxa in the TAXA_SET_P at the time stamp 2 from the TAXA_NET graph, wherein the set of first neighbors are immediate neighbors of the TAXA_SET_P comprising of a TAXA_SET_FN, a TAXA_SET_FN_COM, a TAXA_SET_INTERSECT, aa TAXA_SET_NEW ⌐218

G

200

FIG. 2G

EP 4 288 559 B1

(G)

recommending a personalized therapeutic intervention for improving gut health of the individual (X20), via the one or more hardware processors, wherein the therapeutic intervention comprises recommending one of (a) a prebiotic cocktail, (b) a probiotic cocktail (c) optimized diet based on the identified first neighbors using the set of knowledge bases and the initial set of personalized therapeutics and diet recommendation, wherein the personalized therapeutic intervention comprises of at least one or more of:

(a)     For Case A (a decrease in the pathway abundance of the set of beneficial microbes) recommending a prebiotic cocktail, a probiotic cocktail and an optimized diet, wherein the prebiotic cocktail, probiotic cocktail and the optimized diet replenishes the plurality of beneficial microbes and helps growth of the beneficial microbes,

(b)     For Case B ( an increase in the pathway abundance of the set of harmful microbes) recommending an optimized diet, wherein the optimized diet promotes growth of the plurality of beneficial microbes and inhibits growth of harmful microbes, and

(c) For Case C (a decrease in the pathway abundance of the set of beneficial microbes and increase in the pathway abundance of the set of harmful microbes ) recommending a prebiotic cocktail, probiotic cocktail and an optimized diet, wherein the prebiotic cocktail, probiotic cocktail and the optimized diet to replenishes the plurality of beneficial microbes, helps growth of beneficial microbes and inhibits growth of harmful microbes

220

200

FIG. 2H

EP 4 288 559 B1

generating a matrix Metabolite Map (Mmap) based on the plurality of metabolites, plurality of metabolic pathways and a list of organisms associated with each of the plurality of metabolic pathways, wherein, the Mmap is generated for each the plurality of metabolites ⎯302

generating a bacterial genome map (BGM) using a bacterial genome database (BGD) wherein the BGD comprises of an exhaustive list the plurality of bacterial strains and the BGM comprises information associated with plurality of bacterial strains including a plurality of names of the plurality of bacterial strains, a plurality of identification number of plurality of bacterial strains, a plurality of gene location of the plurality of bacterial strains and a plurality of protein domain of the plurality of bacterial strains ⎯304

generation of a Metabolite pathway-domain map (MPDM) for each metabolite from the plurality of metabolites based on the Mmap and the BGM, wherein the MPDM comprises of the exhaustive list of protein domains associated with each of the plurality of metabolic s pathways ⎯306

300

FIG. 3A

identifying the presence of plurality of metabolic pathways in the list of bacterial strains and genomes from the using the MPDM and the BGM based on a first pre-defined threshold criterion ⌐310

generating the GMP using the BGM, the MPDM, the plurality of metabolic pathways and the plurality of metabolites, wherein the GMP is a matrix with set of bacterial genomes (bacterial strains) as a plurality of rows and the list of plurality of metabolic pathways corresponding to formation of each metabolite as a plurality of columns ⌐312

generating the FGmap using the GMP, wherein FGmap is a matrix as the plurality of bacterial genera as a plurality of rows and the plurality of metabolic pathways as columns ⌐314

300

FIG. 3B

extracting a plurality of nucleic acids from the plurality of gut sample based on a nucleic acid extraction technique — 402

generating a plurality of nucleotide sequences from the extracted plurality of nucleic acid using a sequencer — 404

obtaining a set of bacterial taxonomic abundance matrix at each taxonomic level using the plurality of nucleotide sequences, wherein the bacterial taxonomic abundance matrix is obtained by classifying the plurality of nucleotide sequences based on a pre-defined taxonomic level using a classification algorithm — 406

generating a pathway abundance using the set of bacterial taxonomic abundance matrix, the GMP and the FGmap — 408

400

FIG. 4

computing a normalized change for every taxa in the union of the taxa set 1 and the taxa set 2, between the time stamp 1 and the time stamp 2 ⟶ 502

computing an importance score of every taxa in the union of the taxa set 1 and the taxa set 2, using the normalized change, the plurality of beneficial microbes, the plurality of harmful microbes from the set of knowledge bases ⟶ 504

computing a personalized gut compound score for an individual using the importance score of every taxa in the union of the taxa set 1 and the taxa set 2 and a pre-defined taxa-compound utilization matrix, wherein the personalized gut compound score quantifies the ability of the compound to increase the plurality of beneficial microbes in the gut ⟶ 506

computing a gut food score for all food items in the set of food items using the gut compound score and the food-constituent matrix, wherein the gut food score quantifies the ability of the food items to increase the plurality of beneficial microbes ⟶ 508

optimizing the gut food score using an optimization technique to obtain the optimized diet, wherein the gut food score is optimized subject to a plurality of constraint parameters comprising a calories parameter, a carbohydrate parameter, a protein parameter and a fat parameter ⟶ 510

500

FIG. 5

TAXA_SET_OPTIMIZED

TAXA_SET_NEW

TAXA_SET_FN_COM

TAXA_SET_INTERSECT

TAXA_SET_T2

TAXA_SET_A

TAXA_SET_P

600

FIG. 6

**EP 4 288 559 B1**

**Patent documents cited in the description**

- IN 2022050094 W
- IN 202121004712
- EP 18180341 A